# EUROPÄISCHE PATENTSCHRIFT

(11) **EP 4 330 240 B1**
(45) Veröffentlichungstag und Bekanntmachung des Hinweises auf die Patenterteilung: **15.10.2025**
(21) Anmeldenummer: 22726078.3
(22) Anmeldetag: 28.04.2022
(51) Int. Cl.: C07D 493/06, C07D 307/92, C07D 495/06, H10K 50/11

(54) **MATERIALIEN FÜR ORGANISCHE ELEKTROLUMINESZENZVORRICHTUNGEN**
MATERIALS FOR ORGANIC ELECTROLUMINESCENT DEVICES
MATÉRIAUX POUR DISPOSITIFS ÉLECTROLUMINESCENTS ORGANIQUES

(30) Priorität: 29.04.2021 EP 21171071
(43) Veröffentlichungstag der Anmeldung: 06.03.2024
(73) Patentinhaber: Merck Patent GmbH, 64293 Darmstadt (DE)
(72) Erfinder: PARHAM, Amir Hossain, 64293 DARMSTADT (DE); STOESSEL, Philipp, 64293 DARMSTADT (DE); EHRENREICH, Christian, 64293 DARMSTADT (DE)
(74) Vertreter: Merck Patent Association
(86) Internationale Anmeldenummer: PCT/EP2022/061288
(87) Internationale Veröffentlichungsnummer: WO 2022/229298

(56) Entgegenhaltungen:
- WO-A1-2012/048781
- CN-A- 112 062 772
- CN-A- 112 625 032

## Beschreibung

Die vorliegende Erfindung betrifft Materialien für die Verwendung in elektronischen Vorrichtungen, insbesondere in organischen Elektrolumineszenzvorrichtungen, sowie elektronische Vorrichtungen, insbesondere organische Elektrolumineszenzvorrichtungen enthaltend diese Materialien.

In organischen Elektrolumineszenzvorrichtungen (OLEDs) werden als emittierende Materialien häufig phosphoreszierende metallorganische Komplexe eingesetzt. Generell gibt es bei OLEDs, insbesondere auch bei OLEDs, die Triplettemission (Phosphoreszenz) zeigen, immer noch Verbesserungsbedarf, beispielsweise im Hinblick auf Effizienz, Betriebsspannung und Lebensdauer. Die Eigenschaften phosphoreszierender OLEDs werden nicht nur von den eingesetzten Triplettemittern bestimmt. Hier sind insbesondere auch die anderen verwendeten Materialien, wie Matrixmaterialien, von besonderer Bedeutung. Verbesserungen dieser Materialien können somit auch zu Verbesserungen der OLED-Eigenschaften führen.

Aufgabe der vorliegenden Erfindung ist die Bereitstellung von Verbindungen, welche sich für den Einsatz in einer OLED eignen, insbesondere als Matrixmaterial für phosphoreszierende Emitter, als Elektronentransportmaterial, als Lochblockiermaterial oder auch als Lochtransport- oder Elektronenblockiermaterial und dort zu guten Eigenschaften führen.

In CN112625032 A, CN112062772 A und WO2012048781 A1 werden verbrückte Triphenylenverbindungen beschrieben, die für die Anwendung in elektronischen Vorrichtungen geeignet sind.

Überraschend wurde gefunden, dass bestimmte, unten näher beschriebene Verbindungen diese Aufgabe lösen und sich gut für die Verwendung in OLEDs eignen. Dabei weisen die OLEDs insbesondere eine lange Lebensdauer, eine hohe Effizienz und eine geringere Betriebsspannung auf. Diese Verbindungen sowie elektronische Vorrichtungen, insbesondere organische Elektrolumineszenzvorrichtungen, welche diese Verbindungen enthalten, sind daher der Gegenstand der vorliegenden Erfindung. Gegenstand der vorliegenden Erfindung ist eine Verbindung gemäß Formel (1), 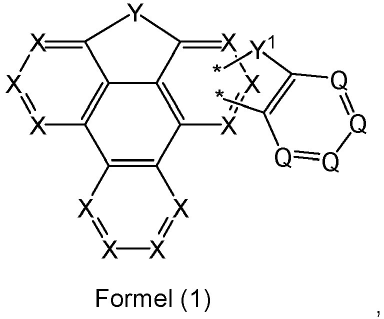 wobei für die verwendeten Symbole gilt:
- X: ist gleich oder verschieden bei jedem Auftreten CR oder N mit der Maßgabe, dass maximal zwei Gruppen X pro Zyklus für N stehen und dass zwei benachbarte Gruppen X, die Teil des Zyklus mit drei Gruppen X sind, für C stehen, welche über die mit * bezeichneten Bindungen ein an den Zyklus ankondensiertes aromatisches oder heteroaromatisches Ringsystem bilden;
- Y: ist C(R)₂, O oder S;
- Y¹: ist C(R)₂;
- Q: ist gleich oder verschieden bei jedem Auftreten CR oder N mit der Maßgabe, dass maximal zwei Gruppen Q pro Zyklus für N stehen.
- R: ist bei jedem Auftreten gleich oder verschieden H, D, F, Cl, Br, I, N(Ar')₂, N(R¹)₂, OAr', SAr', B(OR¹)₂, CHO, C(=O)R¹, CR¹=C(R¹)₂, CN, C(=O)OR¹, C(=O)NR¹, Si(R¹)₃, NO₂, P(=O)(R¹)₂, OSO₂R¹, OR¹, S(=O)R¹, S(=O)₂R¹, SR¹, eine geradkettige Alkylgruppe mit 1 bis 20 C-Atomen oder eine Alkenyl- oder Alkinylgruppe mit 2 bis 20 C-Atomen oder eine verzweigte oder zyklische Alkylgruppe mit 3 bis 20 C-Atomen, wobei die Alkyl-, Alkenyl- oder Alkinylgruppe jeweils mit einem oder mehreren Resten R¹ substituiert sein kann, wobei eine oder mehrere nicht benachbarte CH₂-Gruppen durch -R¹C=CR¹-, -C≡C-, Si(R¹)₂, NR¹, CONR¹, C=O, C=S, -C(=O)O-, P(=O)(R¹), -O-, -S-, SO oder SO₂ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann, wobei zwei oder mehr an den gleichen Zyklus gebundene Reste R miteinander ein aliphatisches, heteroaliphatisches, aromatisches oder heteroaromatisches Ringsystem bilden können, das mit einem oder mehreren Resten R¹ substituiert sein kann, und wobei zwei an dasselbe Kohlenstoff-, Silicium-, Germanium- oder Zinnatom gebundene Reste R ein monozyklisches oder polyzyklisches, aliphatisches, aromatisches oder heteroaromatisches Ringsystem miteinander bilden können, das mit einem oder mehreren Resten R¹ substituiert sein kann;
- Ar': ist bei jedem Auftreten gleich oder verschieden ein aromatisches oder heteroaromatisches Ringsystem mit 5 bis 40 aromatischen Ringatomen, das durch einen oder mehrere Reste R¹ substituiert sein kann;
- R¹: ist bei jedem Auftreten gleich oder verschieden H, D, F, I, B(OR²)₂, N(R²)2, CHO, C(=O)R², CR²=C(R²)2, CN, C(=O)OR², Si(R²)3, NO₂, P(=O)(R²)₂, OSO₂R², SR², OR², S(=O)R², S(=O)₂R², eine geradkettige Alkylgruppe mit 1 bis 20 C-Atomen oder eine Alkenyl- oder Alkinylgruppe mit 2 bis 20 C-Atomen oder eine verzweigte oder zyklische Alkylgruppe mit 3 bis 20 C-Atomen, wobei die Alkyl-, Alkenyl- oder Alkinylgruppe jeweils mit einem oder mehreren Resten R² substituiert sein kann und wobei eine oder mehrere CH₂-Gruppen in den oben genannten Gruppen durch -R²C=CR²-, -C=C-, Si(R²)₂, C=O, C=S, -C(=O)O-, NR², CONR², P(=O)(R²), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R² substituiert sein kann, wobei zwei oder mehr Reste R¹ miteinander ein aliphatisches, heteroaliphatisches, aromatisches oder heteroaromatisches Ringsystem bilden können;
- R²: ist bei jedem Auftreten gleich oder verschieden H, D, F, CN oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R² miteinander verknüpft sein und einen Ring bilden.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Zyklus, also Benzol, bzw. ein einfacher heteroaromatischer Zyklus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 60 C-Atome, bevorzugt 6 bis 40 C-Atome im Ringsystem. Ein heteroaromatisches Ringsystem im Sinne dieser Erfindung enthält 2 bis 60 C-Atome, bevorzugt 2 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. Ebenso sollen hierunter Systeme verstanden werden, in denen zwei oder mehr Aryl- bzw. Heteroarylgruppen direkt miteinander verknüpft sind, wie z. B. Biphenyl, Terphenyl, Bipyridin oder Phenylpyridin. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Bevorzugte aromatische bzw. heteroaromatische Ringsysteme sind einfache Aryl- bzw. Heteroarylgruppen sowie Gruppen, in denen zwei oder mehr Aryl- bzw. Heteroarylgruppen direkt miteinander verknüpft sind, beispielsweise Biphenyl oder Bipyridin, sowie Fluoren oder Spirobifluoren.

Ein elektronenreiches heteroaromatisches Ringsystem ist dadurch gekennzeichnet, dass es sich dabei um ein heteroaromatisches Ringsystem handelt, das keine elektronenarmen Heteroarylgruppen enthält. Eine elektronenarme Heteroarylgruppe ist eine Sechsring-Heteroarylgruppe mit mindestens einem Stickstoffatom oder eine Fünfring-Heteroarylgruppe mit mindestens zwei Heteroatomen, von denen eines ein Stickstoffatom und das andere Sauerstoff, Schwefel oder ein substituiertes Stickstoffatom ist, wobei an diese Gruppen jeweils noch weitere Aryl- oder Heteroarylgruppen ankondensiert sein können. Dagegen sind elektronenreiche Heteroarylgruppen Fünfring-Heteroarylgruppen mit genau einem Heteroatom, ausgewählt aus Sauerstoff, Schwefel oder substituiertem Stickstoff, an welche noch weitere Arylgruppen und/oder weitere elektronenreiche Fünfring-Heteroarylgruppen ankondensiert sein können. So sind Beispiele für elektronenreiche Heteroarylgruppen Pyrrol, Furan, Thiophen, Indol, Benzofuran, Benzothiophen, Carbazol, Dibenzofuran, Dibenzothiophen oder Indenocarbazol. Eine elektronenreiche Heteroarylgruppe wird auch als elektronenreicher heteroaromatischer Rest bezeichnet.

Ein elektronenarmes heteroaromatisches Ringsystem ist dadurch gekennzeichnet, dass es mindestens eine elektronenarme Heteroarylgruppe enthält, und insbesondere bevorzugt keine elektronenreiche Heteroarylgruppen.

Im Rahmen der vorliegenden Erfindung wird der Begriff Alkylgruppe als Oberbegriff sowohl für lineare oder verzweigte Alkylgruppen wie auch für zyklische Alkylgruppen verwendet. Analog werden die Begriffe Alkenylgruppe bzw. Alkinylgruppe als Oberbegriffe sowohl für lineare oder verzweigte Alkenyl- bzw. Alkinylgruppen wie auch für zyklische Alkenyl- bzw. Alkinylgruppen verwendet.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe OR¹ mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe SR¹ mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder zyklisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, besonders bevorzugt F oder CN ersetzt sein.

Unter einem aromatischen oder heteroaromatischen Ringsystem mit 5 - 60 aromatischen Ringatomen, welches noch jeweils mit den oben genannten Resten R² oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

Unter der Formulierung, dass zwei oder mehr Reste miteinander ein Ringsystem bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht:

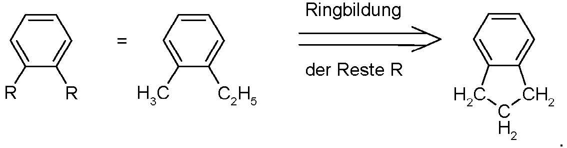

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

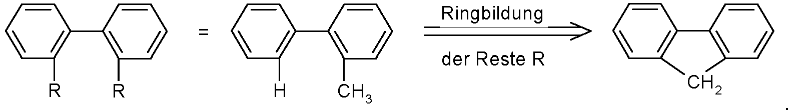

Weitere bevorzugte Ausführungsformen zeigen die folgenden Formeln (2-1) bis (2-4): 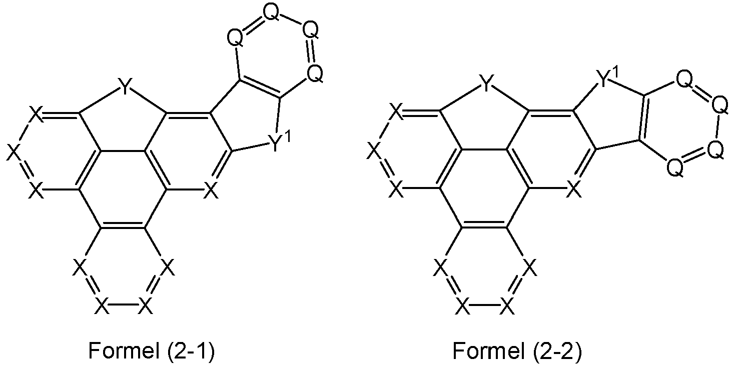 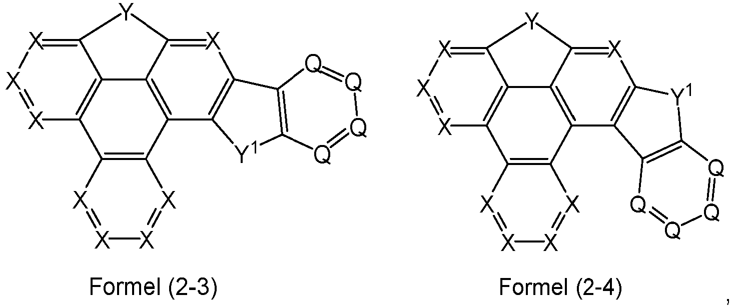 wobei die verwendeten Symbole die oben für Formel (1) genannten Bedeutungen aufweisen.

In einer bevorzugten Ausführungsform steht mindestens ein Rest R der Symbole Y, Y¹, X oder Q in einer der Formeln (1) oder (2-1) bis (2-4) für ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann.

In einer bevorzugten Ausführungsform der Erfindung steht maximal ein Symbol X pro Zyklus für N. In einer weiteren bevorzugten Ausführungsform der Erfindung steht maximal ein Symbol Q für N.

In einer besonders bevorzugten Ausführungsform der Erfindung stehen X und Q für CR.

In einer bevorzugten Ausführungsform der Erfindung weist die Verbindung nur ein ankondensiertes Ringsystem auf, welches über die mit * gekennzeichneten Bindungen ankondensiert ist.

Bevorzugt weist die Verbindung nach Formel (1) nur ein ankondensiertes Ringsystem auf, welches über die mit * gekennzeichneten Bindungen ankondensiert ist, sowie nur bis zu ein weiteres ankondensiertes Ringsystem auf, bei dem zwei benachbarte Reste R ein an den Zyklus ankondensiertes aromatisches oder heteroaromatisches Ringsystem mit 4 bis 8 Ringatomen bilden, welches mit einem oder mehreren Resten R substituiert sein kann.

Bevorzugte Ausführungsformen der Verbindungen der Formeln (2-1) bis (2-4) sind die folgenden Verbindungen der Formeln (3-1) bis (3-4): 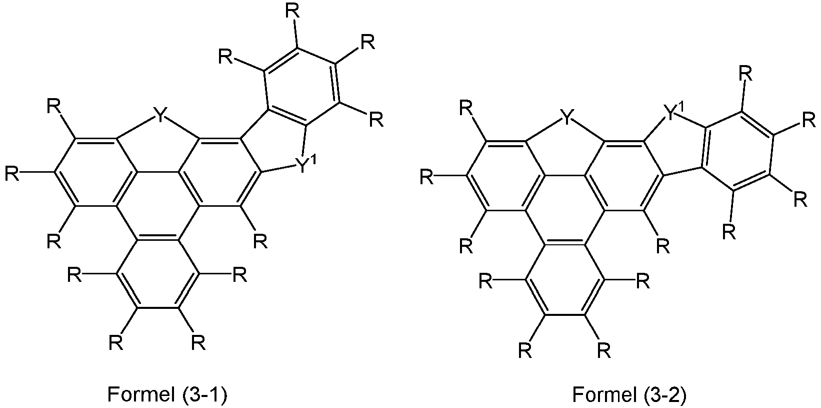 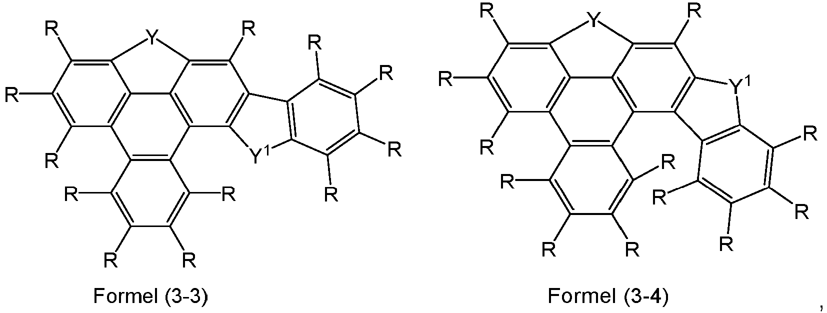 wobei die Symbole, soweit vorhanden, die für die Formeln (2-1) bis (2-4) genannten Bedeutungen aufweisen.

Bevorzugte Ausführungsformen der Verbindungen der Formeln (3-1) bis (3-4) sind die folgenden Verbindungen der Formeln (3-1a) bis (3-4a): 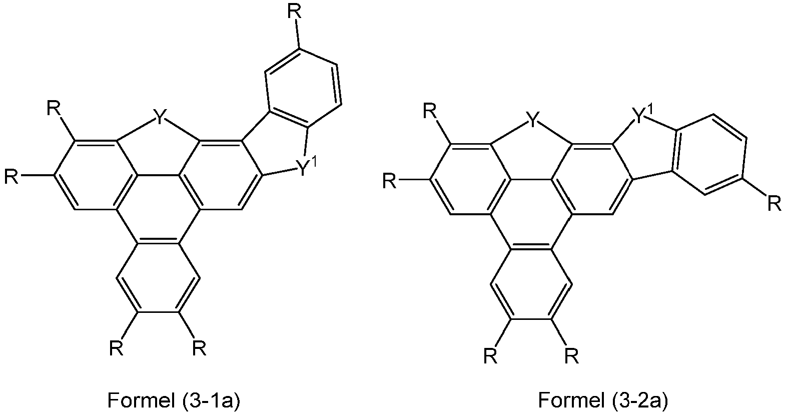 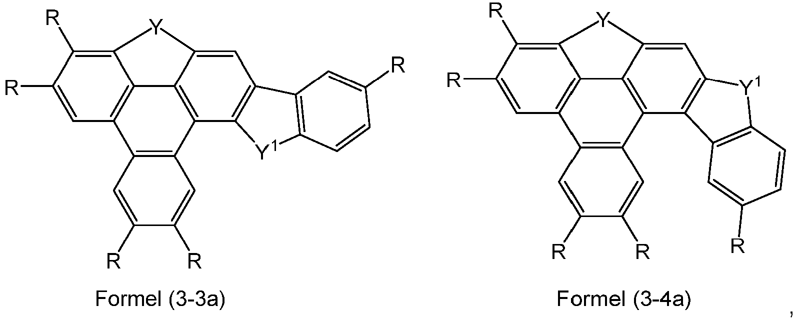 wobei die Symbole, soweit vorhanden, die für die Formeln (2-1) bis (2-4) genannten Bedeutungen aufweisen.

In einer bevorzugten Ausführungsform der Erfindung stehen maximal 5 Gruppen R in den Formeln (2-1) bis (2-4), in den Formeln (3-1) bis (3-4) und in den Formeln (3-1a) bis (3-4a) nicht für H, CN oder D, besonders bevorzugt maximal drei Gruppen R, ganz besonders bevorzugt maximal zwei Gruppen R und insbesondere bevorzugt maximal eine Gruppe R.

Im Folgenden werden bevorzugte Substituenten R, Ar', R¹ und R² beschrieben. In einer besonders bevorzugten Ausführungsform der Erfindung treten die nachfolgend genannten Bevorzugungen für R, Ar', R¹ und R² gleichzeitig auf und gelten für die Strukturen der Formel (1) sowie für alle oben aufgeführten bevorzugten Ausführungsformen.

In einer bevorzugten Ausführungsform der Erfindung ist R bei jedem Auftreten gleich oder verschieden ausgewählt aus der Gruppe bestehend aus H, D, F, CN, OR¹, einer geradkettigen Alkylgruppe mit 1 bis 10 C-Atomen oder einer Alkenylgruppe mit 2 bis 10 C-Atomen oder einer verzweigten oder zyklischen Alkylgruppe mit 3 bis 10 C-Atomen, wobei die Alkyl- bzw. Alkenylgruppe jeweils mit einem oder mehreren Resten R¹ substituiert sein kann, bevorzugt jedoch unsubstituiert ist, und wobei eine oder mehrere nicht benachbarte CH₂-Gruppen durch O ersetzt sein können, oder einem aromatischen oder heteroaromatischen Ringsystem mit 6 bis 30 aromatischen Ringatomen, das jeweils durch einen oder mehrere Reste R¹ substituiert sein kann; dabei können zwei Reste R auch miteinander ein aliphatisches, aromatisches oder heteroaromatisches Ringsystem bilden. Besonders bevorzugt ist R bei jedem Auftreten gleich oder verschieden ausgewählt aus der Gruppe bestehend aus H, D, einer geradkettigen Alkylgruppe mit 1 bis 6 C-Atomen, insbesondere mit 1, 2, 3 oder 4 C-Atomen, oder einer verzweigten oder zyklischen Alkylgruppe mit 3 bis 6 C-Atomen, wobei die Alkylgruppe jeweils mit einem oder mehreren Resten R¹ substituiert sein kann, bevorzugt aber unsubstituiert ist, oder einem aromatischen oder heteroaromatischen Ringsystem mit 6 bis 24 aromatischen Ringatomen, das jeweils durch einen oder mehrere Reste R¹, bevorzugt nicht-aromatische Reste R¹, substituiert sein kann. Ganz besonders bevorzugt ist R bei jedem Auftreten gleich oder verschieden ausgewählt aus der Gruppe bestehend aus H oder D oder einem aromatischen oder heteroaromatischen Ringsystem mit 6 bis 24 aromatischen Ringatomen, das jeweils durch einen oder mehrere Reste R², bevorzugt nicht-aromatische Reste R¹, substituiert sein kann.

Geeignete aromatische bzw. heteroaromatische Ringsysteme R sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, welches über die 1- oder 2-Position verknüpft sein kann, Indol, Benzofuran, Benzothiophen, welches über die 1-, 2-, 3- oder 4-Position verknüpft sein kann, Dibenzofuran, Carbazol, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Chinazolin, Chinoxalin, Benzimidazol, Phenanthren, Triphenylen oder einer Kombination aus zwei oder drei dieser Gruppen, welche jeweils mit einem oder mehreren Resten R¹ substituiert sein können. Wenn R für eine Heteroarylgruppe, insbesondere für Triazin, Pyrimidin, Chinoxalin oder Chinazolin steht, können auch aromatische oder heteroaromatische Reste R¹ an dieser Heteroarylgruppe bevorzugt sein.

Dabei sind die Gruppen R, wenn sie für ein aromatisches bzw. heteroaromatisches Ringsystem stehen, bevorzugt gewählt aus den Gruppen der folgenden Formeln R-1 bis R-83, 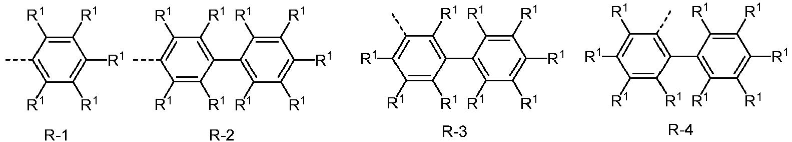 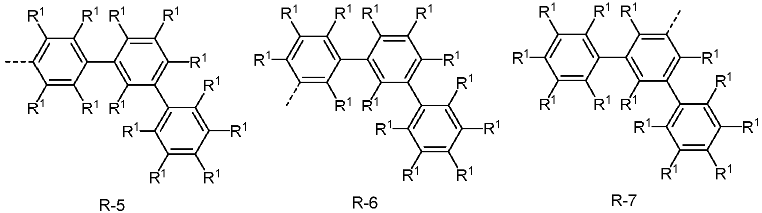 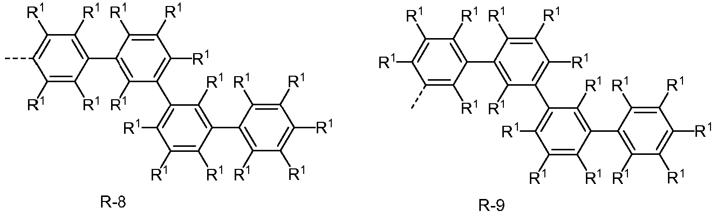 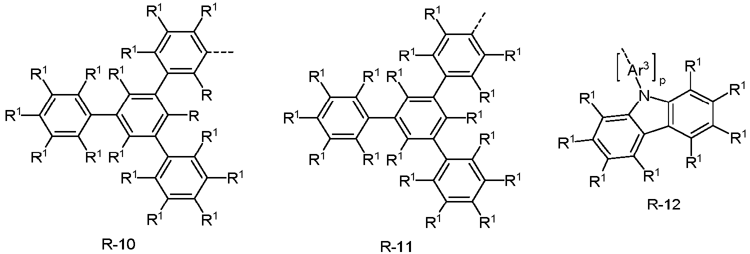 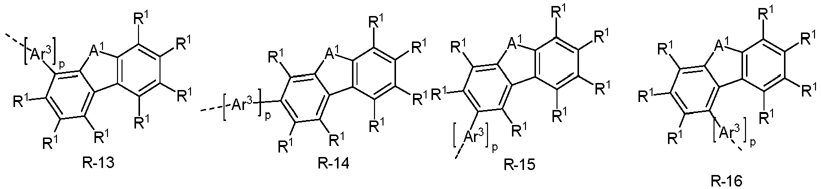 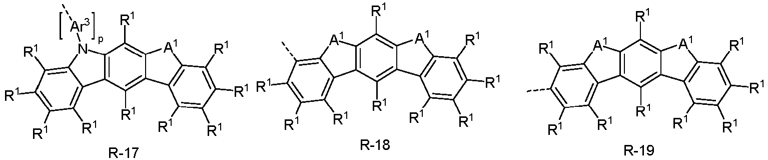 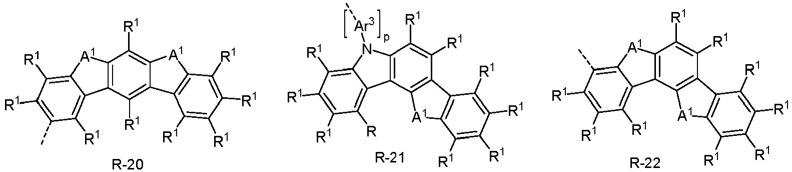 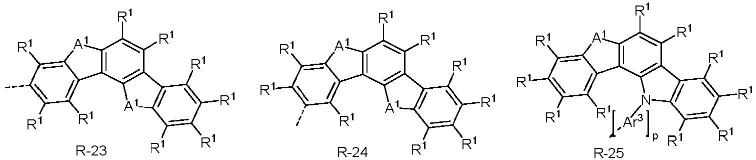 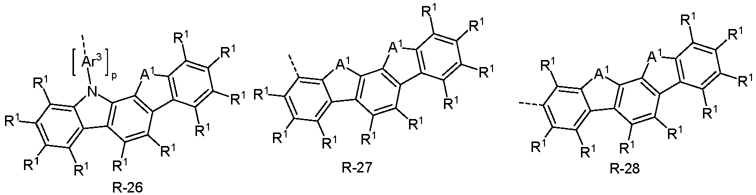 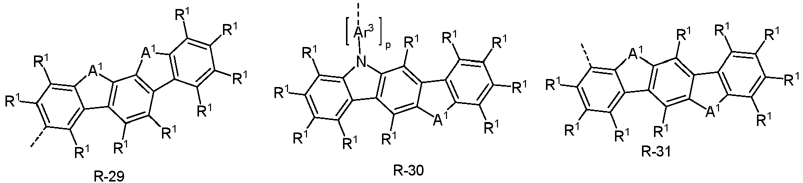 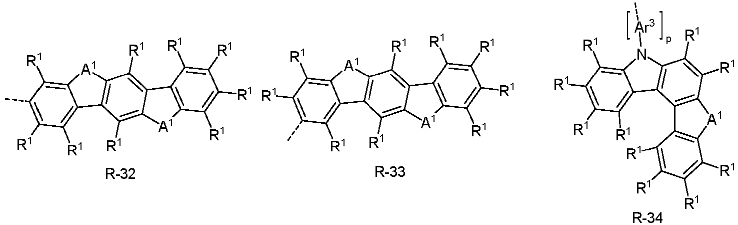 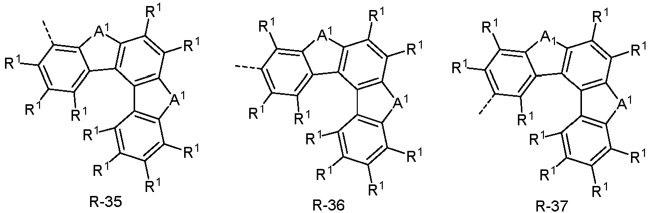 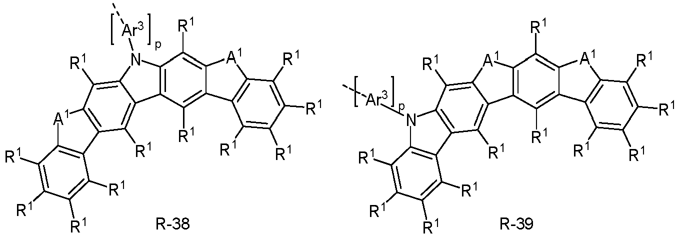 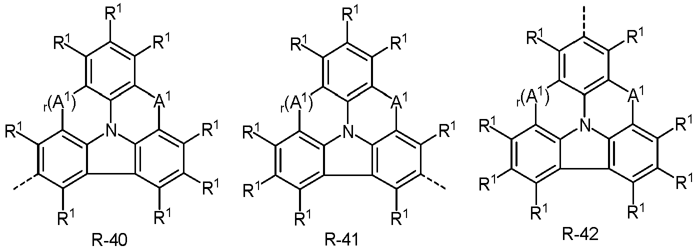 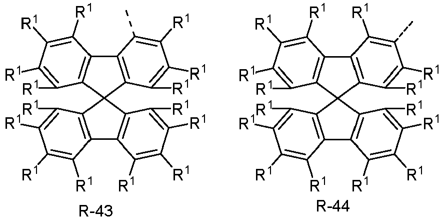 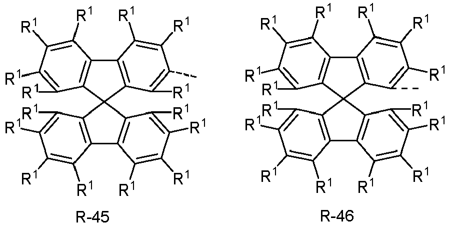 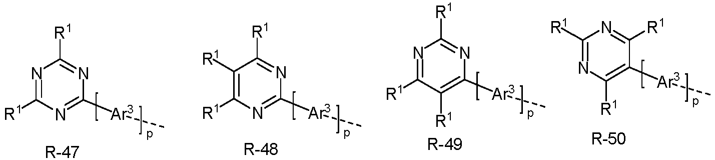 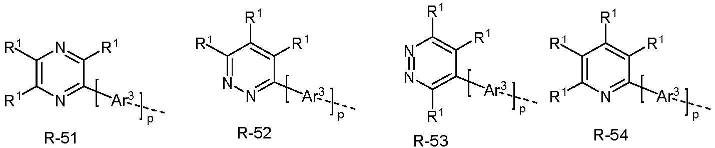 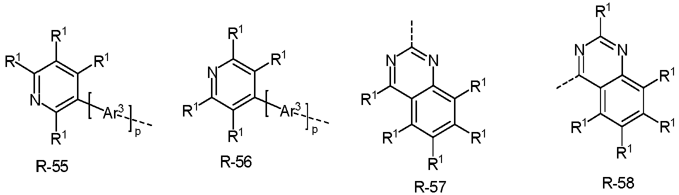 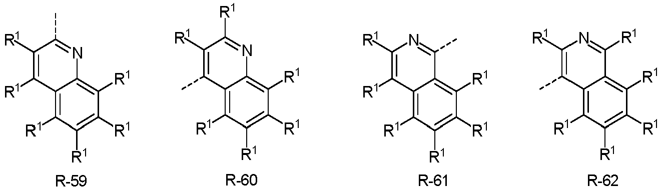 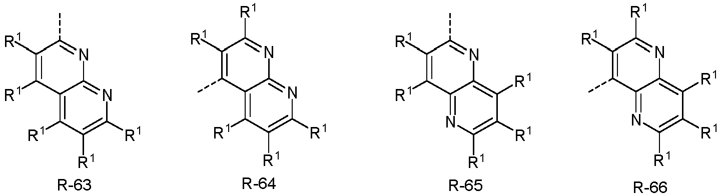 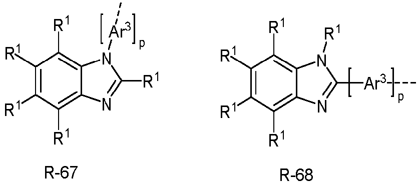 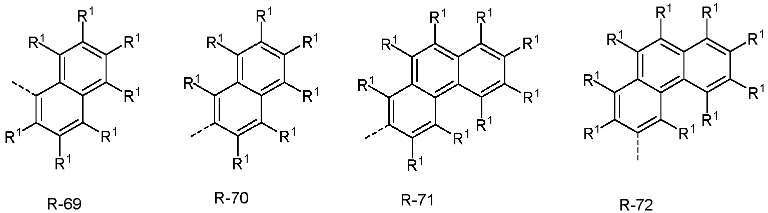 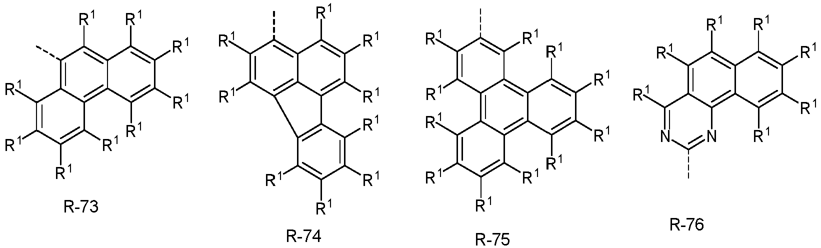 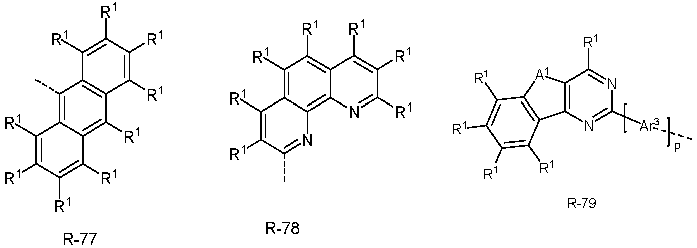 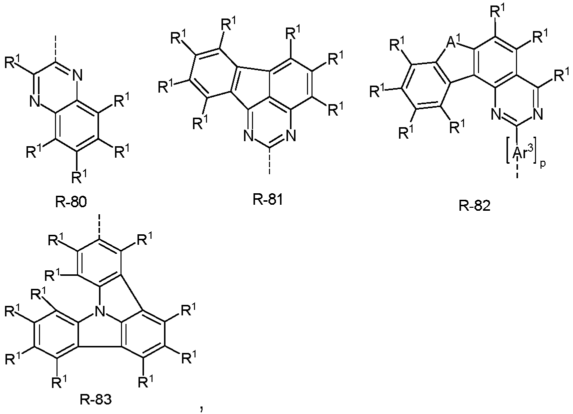 wobei R¹ die oben genannten Bedeutungen aufweist, die gestrichelte Bindung die Bindung an ein Kohlenstoffatom des Grundgerüsts in Formel (1) bzw. in den bevorzugten Ausführungsformen darstellt und weiterhin gilt:
- Ar³: ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R¹ substituiert sein kann;
- A¹: ist bei jedem Auftreten gleich oder verschieden C(R¹)₂, NR¹, O oder S, bevorzugt O oder S;
- p: ist 0 oder 1, wobei p = 0 bedeutet, dass die Gruppe Ar³ nicht vorhanden ist und dass die entsprechende aromatische bzw. heteroaromatische Gruppe direkt an ein Kohlenstoffatom des Grundgerüsts in Formel (1) gebunden ist;
- r: ist 0 oder 1, wobei r = 0 bedeutet, dass an dieser Position keine Gruppe A¹ gebunden ist und an die entsprechenden Kohlenstoffatome stattdessen Reste R¹ gebunden sind.

In einer bevorzugten Ausführungsform umfasst Ar³ bivalente aromatische oder heteroaromatische Ringsysteme basierend auf den Gruppen der R-1 bis R-83, wobei m gleich 0 gilt und die gestrichelte Bindung und ein R¹ für die Bindung zur aromatischen oder heteroaromatischen Gruppe nach R-1 bis R-83 steht.

Wenn die oben genannten Gruppen R-1 bis R-83 für R mehrere Gruppen A¹ aufweisen, so kommen hierfür alle Kombinationen aus der Definition von A¹ in Frage. Bevorzugte Ausführungsformen sind dann solche, in denen eine Gruppe A¹ für O oder S und die andere Gruppe A¹ für C(R¹)₂ steht oder in denen beide Gruppen A¹ für S oder O stehen oder in denen beide Gruppen A¹ für O bzw. S stehen.

Wenn A¹ für NR¹ steht, steht der Substituent R¹, der an das Stickstoffatom gebunden ist, bevorzugt für ein aromatisches oder heteroaromatisches Ringsystem mit 5 bis 24 aromatischen Ringatomen, welches auch durch einen oder mehrere Reste R² substituiert sein kann. In einer besonders bevorzugten Ausführungsform steht dieser Substituent R¹ gleich oder verschieden bei jedem Auftreten für ein aromatisches oder heteroaromatisches Ringsystem mit 6 bis 24 aromatischen Ringatomen, bevorzugt mit 6 bis 12 aromatischen Ringatomen, welches keine kondensierten Arylgruppen oder Heteroarylgruppen, in denen zwei oder mehr aromatische bzw. heteroaromatische 6-Ring-Gruppen direkt aneinander ankondensiert sind, aufweist, und welches jeweils auch durch einen oder mehrere Reste R² substituiert sein kann. Besonders bevorzugt sind Phenyl, Biphenyl, Terphenyl und Quaterphenyl mit Verknüpfungsmustern, wie vorne für R-1 bis R-11 aufgeführt, wobei diese Strukturen durch einen oder mehrere Reste R¹ substituiert sein können, bevorzugt aber unsubstituiert sind.

Wenn A¹ für C(R¹)₂ steht, stehen die Substituenten R¹, die an dieses Kohlenstoffatom gebunden sind, bevorzugt gleich oder verschieden bei jedem Auftreten für eine lineare Alkylgruppe mit 1 bis 10 C-Atomen oder für eine verzweigte oder zyklische Alkylgruppe mit 3 bis 10 C-Atomen oder für ein aromatisches oder heteroaromatisches Ringsystem mit 5 bis 24 aromatischen Ringatomen, welches auch durch einen oder mehrere Reste R² substituiert sein kann. Ganz besonders bevorzugt steht R¹ für eine Methylgruppe oder für eine Phenylgruppe. Dabei können die Reste R¹ auch miteinander ein Ringsystem bilden, was zu einem Spirosystem führt.

Wenn Y und/oder Y¹ für C(R)₂ steht, stehen die Substituenten R, die an dieses Kohlenstoffatom gebunden sind, bevorzugt gleich oder verschieden bei jedem Auftreten für eine lineare Alkylgruppe mit 1 bis 10 C-Atomen oder für eine verzweigte oder zyklische Alkylgruppe mit 3 bis 10 C-Atomen oder für ein aromatisches oder elektronenarmes heteroaromatisches Ringsystem mit 5 bis 24 aromatischen Ringatomen, welches auch durch einen oder mehrere Reste R¹ substituiert sein kann. Ganz besonders bevorzugt stehen diese Substituenten R für eine Methylgruppe oder für eine Phenylgruppe. Dabei können die Reste R auch miteinander ein Ringsystem bilden, was zu einem Spirosystem führt.

In einer Ausführungsform der Erfindung steht mindestens ein Rest R für ein elektronenreiches heteroaromatisches Ringsystem. Dabei ist das elektronenreiche heteroaromatische Ringsystem bevorzugt gewählt aus den oben abgebildeten Gruppen R-13 bis R-42, wobei in den Gruppen R-13 bis R-16, R-18 bis R-20, R-22 bis R-24, R-27 bis R-29, R-31 bis R-33 und R-35 bis R-37 mindestens eine Gruppe A¹ für NR¹ steht, wobei R¹ bevorzugt für ein aromatisches oder heteroaromatisches Ringsystem steht, insbesondere für ein aromatisches Ringsystem.

In einer weiteren besonders bevorzugten Ausführungsform der Erfindung steht mindestens ein Rest R für ein elektronenarmes heteroaromatisches Ringsystem. Dabei ist das elektronenarme heteroaromatische Ringsystem bevorzugt gewählt aus den oben abgebildeten Gruppen R-47 bis R-50, R-57, R-58, R-76; R-79, R-80, R-81 und R-82.

In einer weiteren bevorzugten Ausführungsform der Erfindung ist R¹ gleich oder verschieden bei jedem Auftreten ausgewählt aus der Gruppe bestehend aus H, D, F, CN, OR², einer geradkettigen Alkylgruppe mit 1 bis 10 C-Atomen oder einer Alkenylgruppe mit 2 bis 10 C-Atomen oder einer verzweigten oder zyklischen Alkylgruppe mit 3 bis 10 C-Atomen, wobei die Alkyl- bzw. Alkenylgruppe jeweils mit einem oder mehreren Resten R² substituiert sein kann und wobei eine oder mehrere nicht benachbarte CH₂-Gruppen durch O ersetzt sein können, oder einem aromatischen oder heteroaromatischen Ringsystem mit 6 bis 30 aromatischen Ringatomen, das jeweils durch einen oder mehrere Reste R² substituiert sein kann; dabei können zwei oder mehrere Reste R¹ miteinander ein aliphatisches Ringsystem bilden. In einer besonders bevorzugten Ausführungsform der Erfindung ist R¹ gleich oder verschieden bei jedem Auftreten ausgewählt aus der Gruppe bestehend aus H, D, einer geradkettigen Alkylgruppe mit 1 bis 6 C-Atomen, insbesondere mit 1, 2, 3 oder 4 C-Atomen, oder einer verzweigten oder zyklischen Alkylgruppe mit 3 bis 6 C-Atomen, wobei die Alkylgruppe mit einem oder mehreren Resten R² substituiert sein kann, bevorzugt aber unsubstituiert ist, oder einem aromatischen oder heteroaromatischen Ringsystem mit 6 bis 24 aromatischen Ringatomen, das jeweils durch einen oder mehrere Reste R² substituiert sein kann, bevorzugt aber unsubstituiert ist.

In einer weiteren bevorzugten Ausführungsform der Erfindung ist R² gleich oder verschieden bei jedem Auftreten H, D, F, eine Alkylgruppe mit 1 bis 4 C-Atomen oder eine Arylgruppe mit 6 bis 10 C-Atomen, welche mit einer Alkylgruppe mit 1 bis 4 C-Atomen substituiert sein kann, bevorzugt aber unsubstituiert ist.

In einer weiteren bevorzugten Ausführungsform der Erfindung sind alle Reste R¹, soweit sie für ein aromatisches oder heteroaromatisches Ringsystem, bzw. R² soweit sie für aromatische oder heteroaromatische Gruppen stehen, ausgewählt aus den Gruppen R-1 bis R-83, welche allerdings dann jeweils entsprechend mit R², bzw. den bei R² genannten Gruppen substituiert sind.

In einer bevorzugten Ausführungsform bilden die Reste R keine weiteren an das Grundgerüst der Formel (1) ankondensierten aromatischen oder heteroaromatischen Gruppen.

Dabei haben die Alkylgruppen in erfindungsgemäßen Verbindungen, die durch Vakuumverdampfung verarbeitet werden, bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Wenn die Verbindungen der Formel (1) bzw. die bevorzugten Ausführungsformen als Matrixmaterial für einen phosphoreszierenden Emitter oder in einer Schicht, die direkt an eine phosphoreszierende Schicht angrenzt, verwendet werden, ist es weiterhin bevorzugt, wenn die Verbindung keine kondensierten Aryl- bzw. Heteroarylgruppen enthält, in denen mehr als zwei Sechsringe direkt aneinander ankondensiert sind. Insbesondere ist es bevorzugt, dass die Reste Ar', R, R¹ und R² keine kondensierten Aryl- bzw. Heteroarylgruppen enthalten, in denen zwei oder mehr Sechsringe direkt aneinander ankondensiert sind. Eine Ausnahme hiervon bilden Phenanthren, Triphenylen, Chinoxalin und Chinazolin, die aufgrund ihrer hohen Triplettenergie trotz der Anwesenheit kondensierter aromatischer Sechsringe bevorzugt sein können.

Die oben genannten bevorzugten Ausführungsformen können beliebig innerhalb der in Anspruch 1 definierten Einschränkungen miteinander kombiniert werden. In einer besonders bevorzugten Ausführungsform der Erfindung treten die oben genannten Bevorzugungen gleichzeitig auf.

Beispiele für bevorzugte Verbindungen gemäß den oben aufgeführten Ausführungsformen sind die in der folgenden Tabelle aufgeführten Verbindungen.

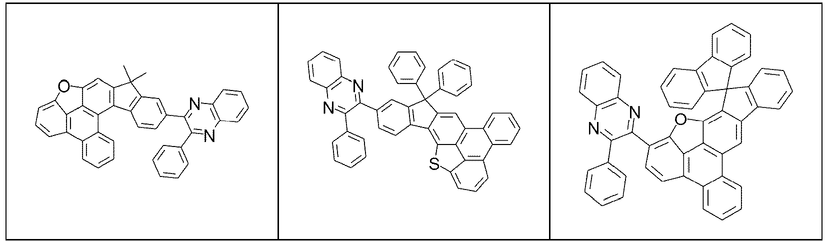

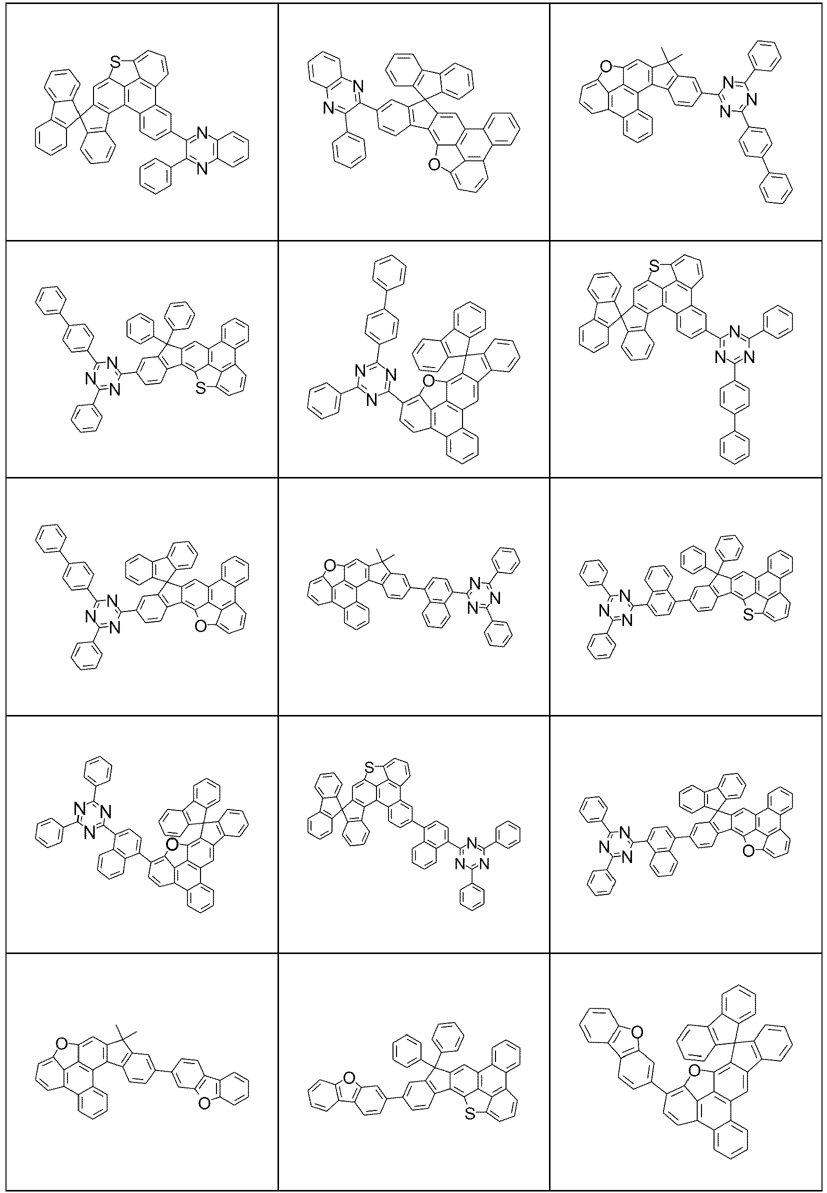

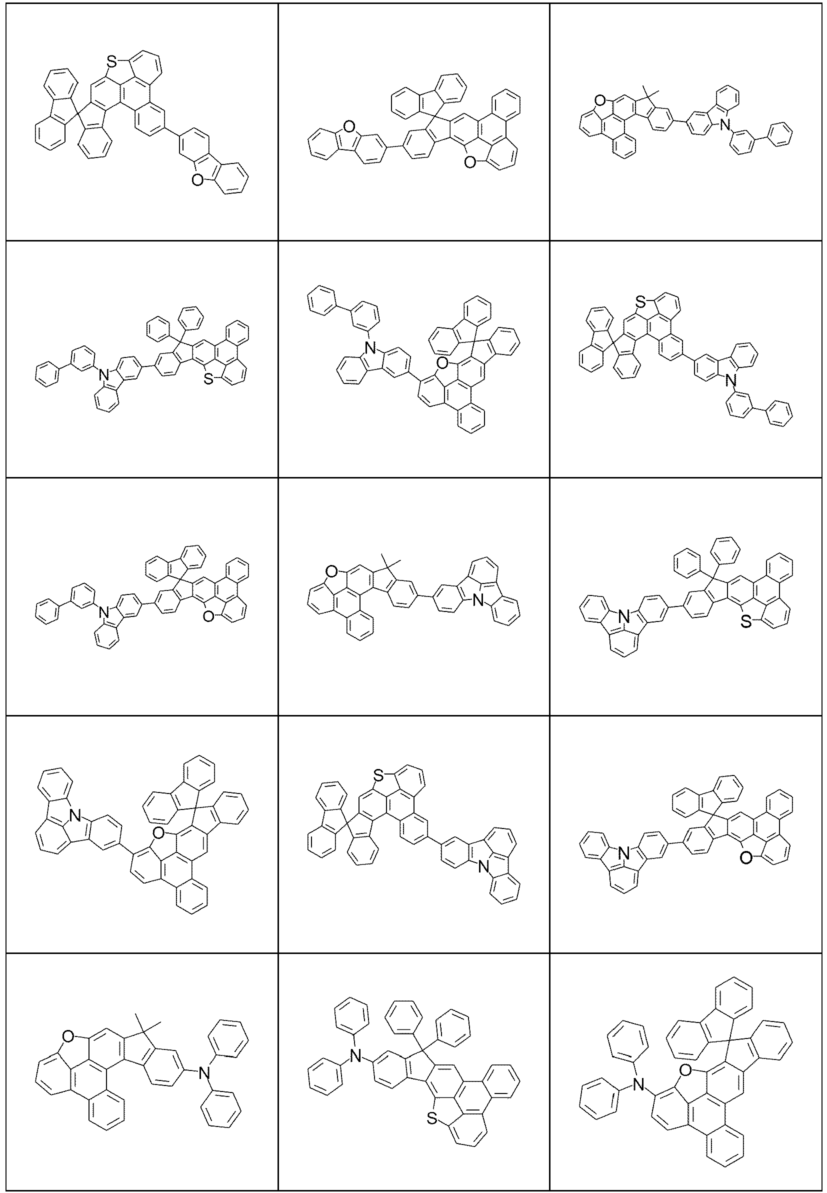

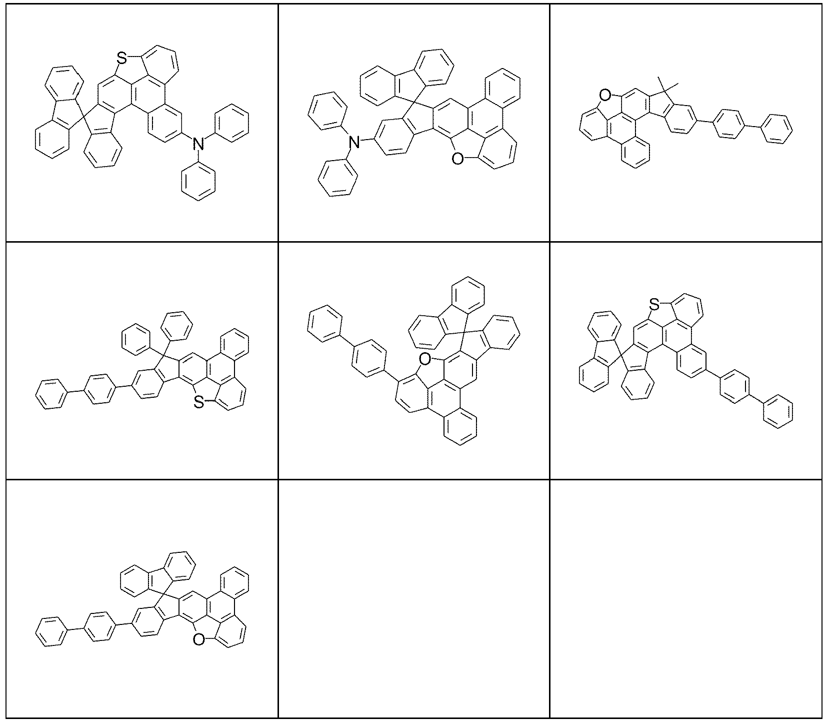

Die erfindungsgemäßen Verbindungen können nach dem Fachmann bekannten Syntheseschritten, wie z. B. Bromierung, Suzuki-Kupplung, Ullmann-Kupplung, Heck-Reaktion, Hartwig-Buchwald-Kupplung, etc., dargestellt werden.

Ein weiterer Gegenstand der vorliegenden Erfindung ist daher ein Verfahren zur Herstellung der erfindungsgemäßen Verbindungen, gekennzeichnet durch die folgenden Schritte:
(A) Synthese des Grundgerüsts nach Formel (1);
(B) Ankondensieren des aromatischen oder heteroaromatischen Ringsystems unter Bildung der mit * bezeichneten Bindungen nach Formel (1) durch Kupplungs- und Ringschlussreaktion.

Die Synthese des Grundgerüsts kann dabei beispielsweise über eine Kombination aus Ullmann-Kupplung, Suzuki-Kupplung und einer intramolekularen Heckreaktion erfolgen, wie im folgenden Schema 1 oder Schema 2 gezeigt:

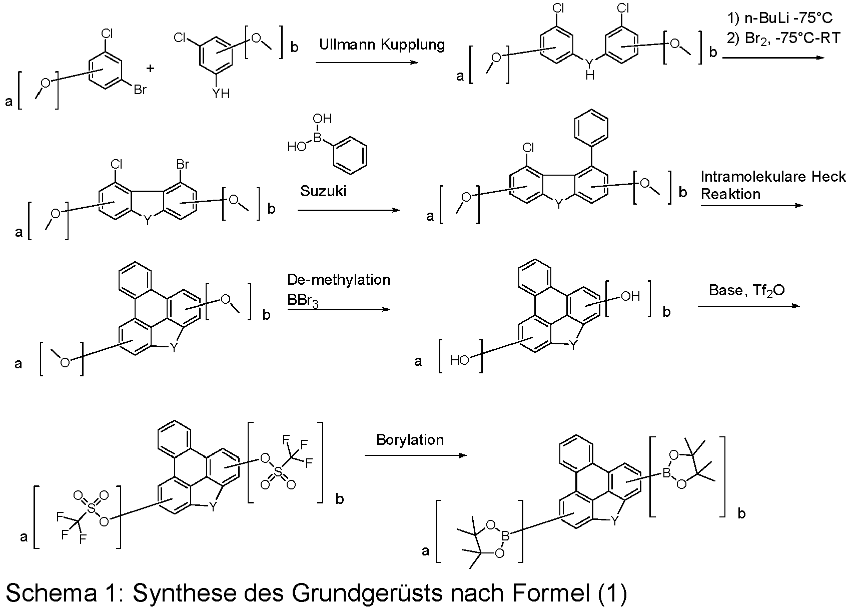

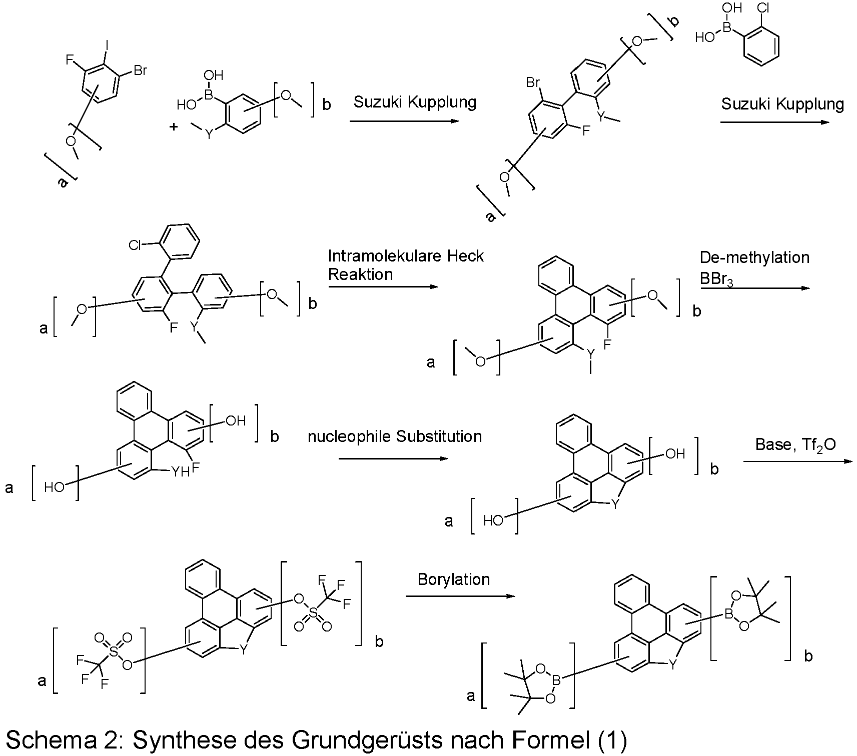

Das Ankondensieren des aromatischen oder heteroaromatischen Ringsystems kann dann durch entsprechende Kupplungs- und Ringschlussreaktionen erfolgen, wie in den folgenden Schemata 3 oder 5 dargestellt.

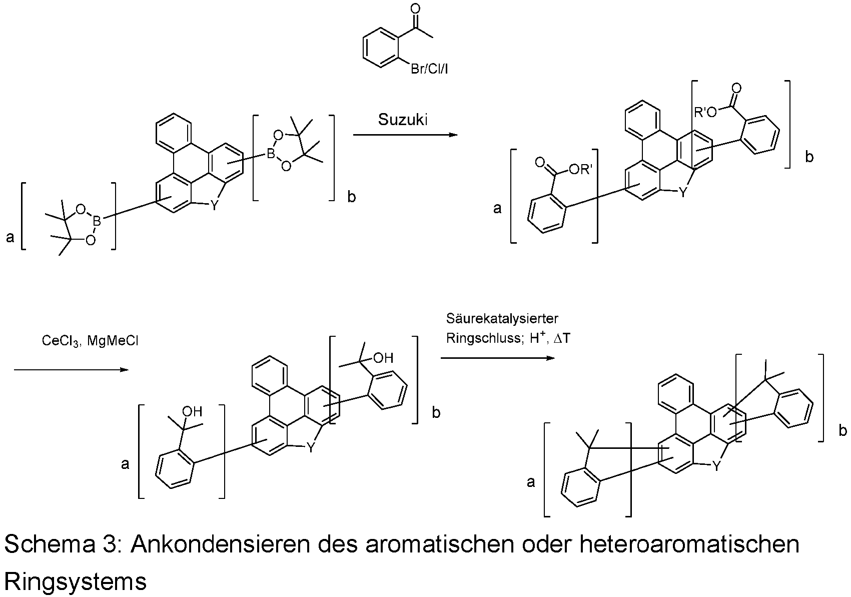

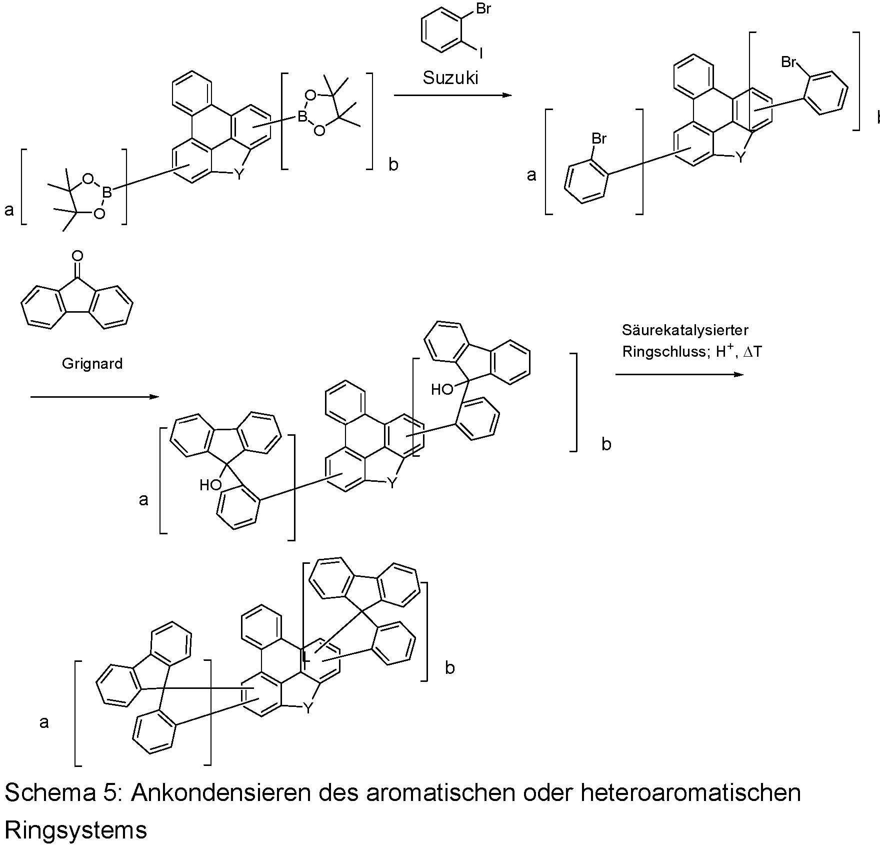

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthyl-isovalerat, Cyclohexylhexanoat oder Mischungen dieser Lösemittel.

Ein weiterer Gegenstand der vorliegenden Erfindung ist daher eine Formulierung, enthaltend mindestens eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung und/oder ein weiteres Matrixmaterial. Geeignete emittierende Verbindungen und weitere Matrixmaterialien sind hinten im Zusammenhang mit der organischen Elektrolumineszenzvorrichtung aufgeführt. Diese weitere Verbindung kann auch polymer sein.

Die erfindungsgemäßen Verbindungen eignen sich für die Verwendung in einer elektronischen Vorrichtung, insbesondere in einer organischen Elektrolumineszenzvorrichtung.

Ein weiterer Gegenstand der vorliegenden Erfindung ist daher die Verwendung einer erfindungsgemäßen Verbindung in einer elektronischen Vorrichtung, insbesondere in einer organischen Elektrolumineszenzvorrichtung.

Ein nochmals weiterer Gegenstand der vorliegenden Erfindung ist eine elektronische Vorrichtung enthaltend mindestens eine erfindungsgemäße Verbindung.

Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), farbstoffsensibilisierten organischen Solarzellen (DSSCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices", bevorzugt aber organischen Elektrolumineszenzvorrichtungen (OLEDs), besonders bevorzugt phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, sodass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen. Es kann sich bei der erfindungsgemäßen organischen Elektrolumineszenzvorrichtung auch um eine Tandem-OLED handeln, insbesondere für weiß emittierende OLEDs.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. die oben ausgeführten bevorzugten Ausführungsformen in einer emittierenden Schicht als Matrixmaterial für phosphoreszierende Emitter, für fluoreszierende Emitter oder für Emitter, die TADF (thermally activated delayed fluorescence) zeigen, insbesondere für phosphoreszierende Emitter. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält. Weiterhin kann die erfindungsgemäße Verbindung auch in einer Elektronentransportschicht und/oder in einer Lochblockierschicht und/oder in einer Lochtransportschicht und/oder in einer Exzitonenblockierschicht eingesetzt werden.

Wenn die erfindungsgemäße Verbindung als Matrixmaterial für eine phosphoreszierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der erfindungsgemäßen Verbindung und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der erfindungsgemäßen Verbindung bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der erfindungsgemäßen Verbindung als Matrixmaterial für einen phosphoreszierenden Emitter in Kombination mit einem weiteren Matrixmaterial. Geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, Triphenylenderivate, z. B. gemäß WO 2012/048781, oder Dibenzofuranderivate, z. B. gemäß WO 2015/169412, WO 2016/015810, WO 2016/023608, WO 2017/148564 oder WO 2017/148565. Ebenso kann ein weiterer phosphoreszierender Emitter, welcher kürzerwellig als der eigentliche Emitter emittiert, als Co-Host in der Mischung vorhanden sein oder eine Verbindung, die nicht oder nicht in wesentlichem Umfang am Ladungstransport teilnimmt, wie beispielsweise in WO 2010/108579 beschrieben.

In einer bevorzugten Ausführungsform der Erfindung werden die Materialien in Kombination mit einem weiteren Matrixmaterial eingesetzt. Bevorzugte Co-Matrixmaterialien, insbesondere wenn die erfindungsgemäße Verbindung mit einem elektronenarmen heteroaromatischen Ringsystem substituiert ist, sind gewählt aus der Gruppe der Biscarbazole, der verbrückten Carbazole, der Triarylamine, der Dibenzofuran-Carbazol-Derivate bzw. Dibenzofuran-Amin-Derivate und der Carbazolamine.

Bevorzugte Biscarbazole sind die Strukturen der folgenden Formeln (4) und (5), 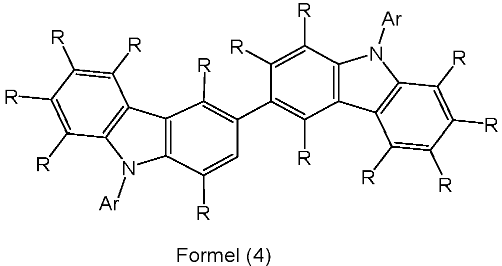 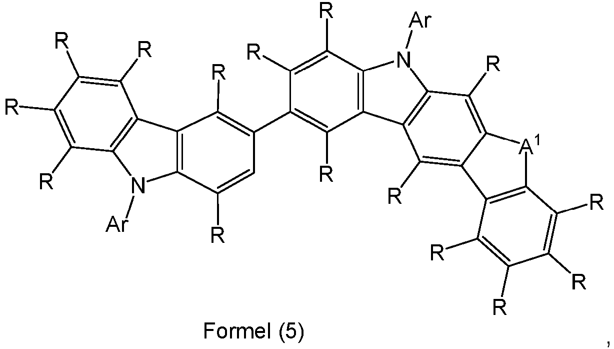 wobei für Ar, R und A¹ folgendes gilt:
- A¹: ist gleich oder verschieden bei jedem Auftreten NAr, O, S oder C(R)₂;
- Ar: ist bei jedem Auftreten gleich oder verschieden ein aromatisches oder heteroaromatisches Ringsystem mit 5 bis 60 aromatischen Ringatomen, das jeweils durch einen oder mehrere Reste R substituiert sein kann;
- R: ist bei jedem Auftreten gleich oder verschieden H, D, F, Cl, Br, I, B(OR¹)₂, CHO, C(=O)R', CR¹=C(R¹)₂, CN, C(=O)OR¹, C(=O)N(R¹)₂, Si(R¹)₃, N(R¹)₂, NO₂, P(=O)(R¹)₂, OSO₂R¹, OR¹, S(=O)R¹, S(=O)₂R¹, SR¹, eine geradkettige Alkylalkylgruppe mit 1 bis 20 C-Atomen oder eine Alkenyl- oder Alkinylgruppe mit 2 bis 20 C-Atomen oder eine verzweigte oder zyklische Alkylgruppe mit 3 bis 20 C-Atomen, wobei die Alkyl-, Alkenyl- oder Alkinylgruppe jeweils mit einem oder mehreren Resten R¹ substituiert sein kann, wobei eine oder mehrere nicht benachbarte CH₂-Gruppen durch -R¹C=CR¹-, -C=C-, Si(R¹)₂, C=O, C=S, C=NR¹, -C(=O)O-, -C(=O)NR¹-, NR¹, P(=O)(R¹), -O-, -S-, SO oder SO₂ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann, wobei zwei oder mehr Reste R miteinander verknüpft sein können und einen Ring bilden können;

In einer bevorzugten Ausführungsform der Erfindung steht A¹ für CR₂.

Bevorzugt steht Ar im Falle der Formel (4) und (5) für ein aromatisches oder heteroaromatisches Ringsystem, bevorzugt bei jedem Auftreten gleich oder verschieden gewählt aus den Gruppen der folgenden Formeln Ar-1 bis Ar-76, 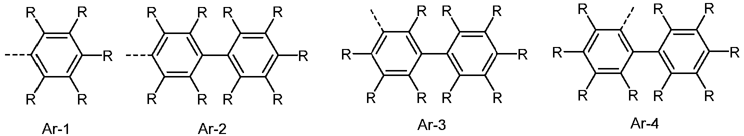 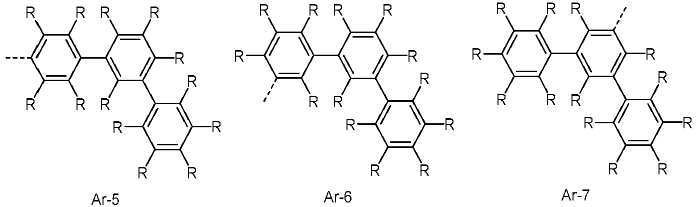 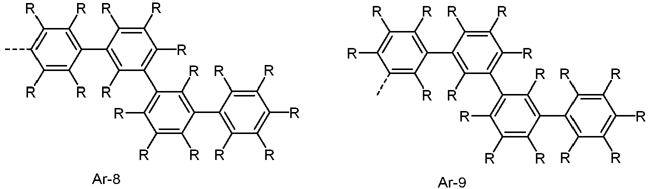 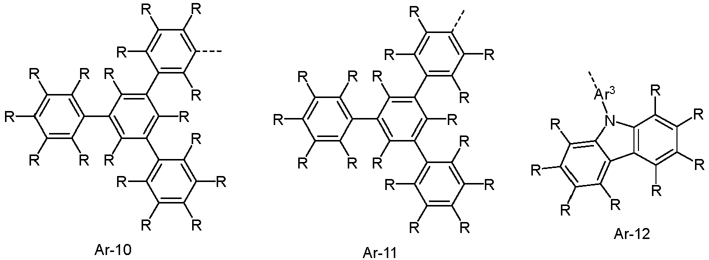 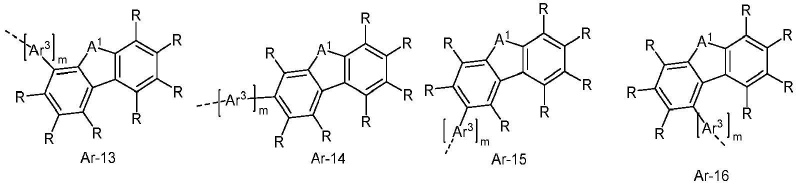 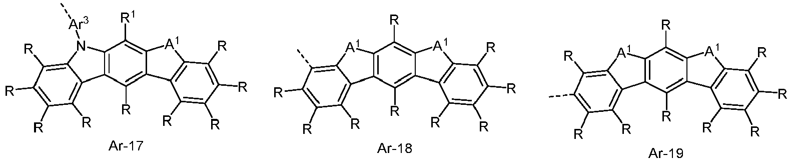 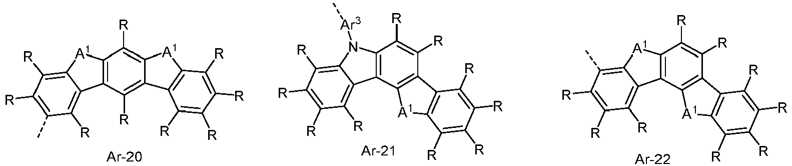 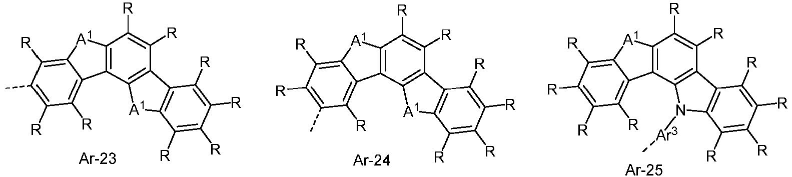 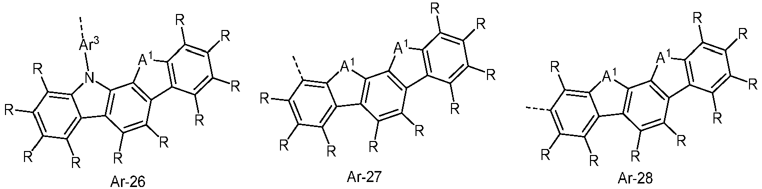 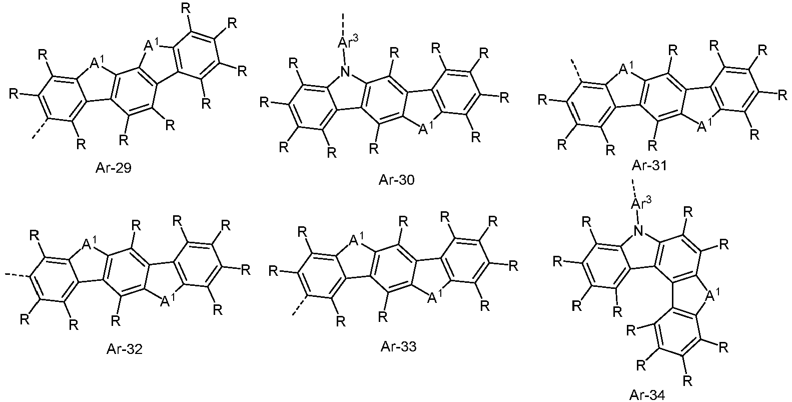 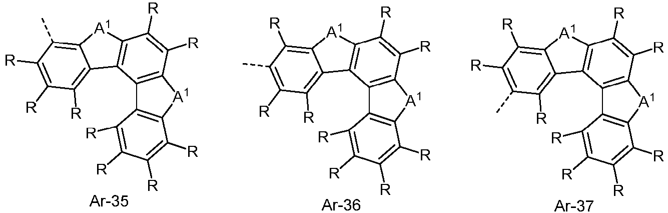 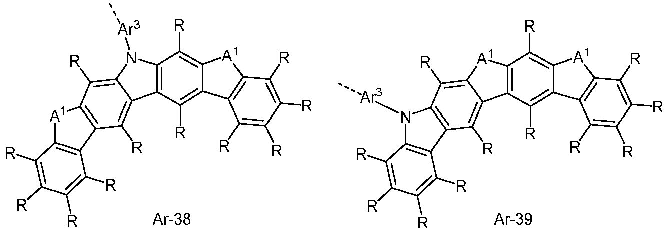 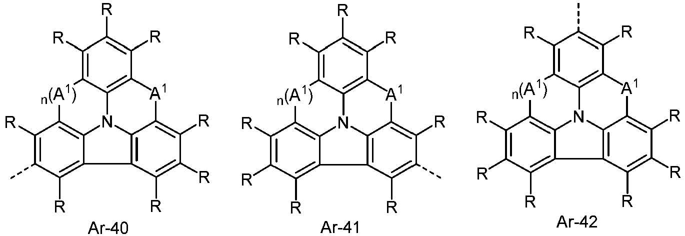 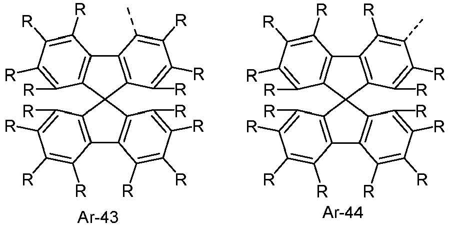 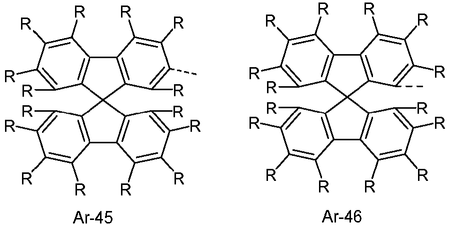 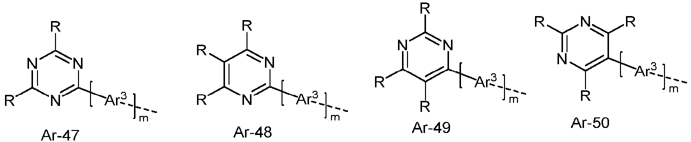 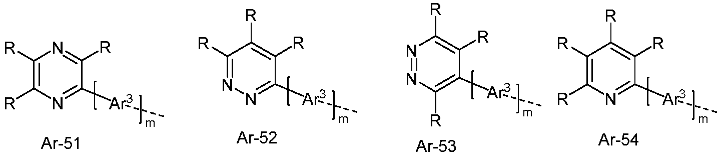 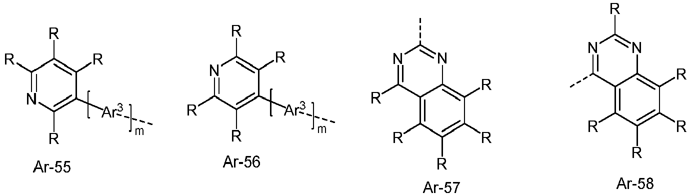 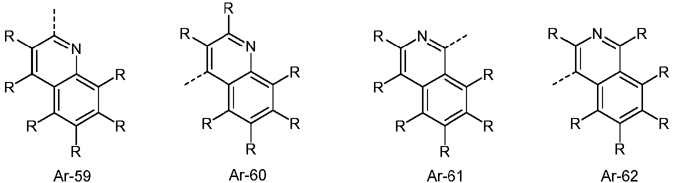 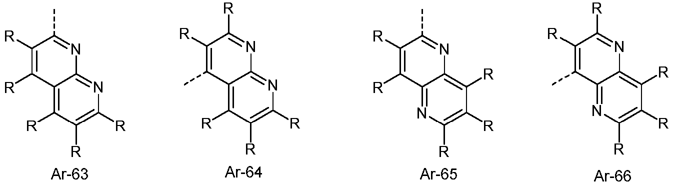 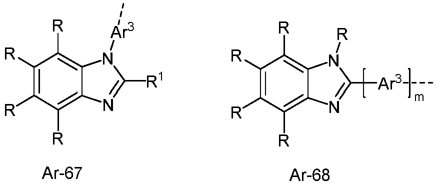 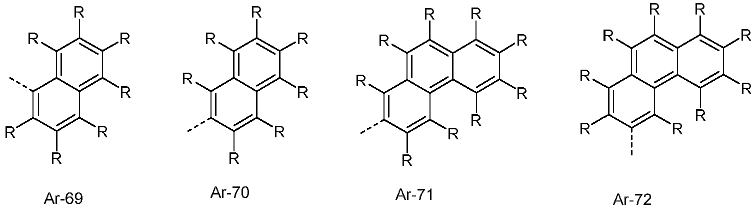 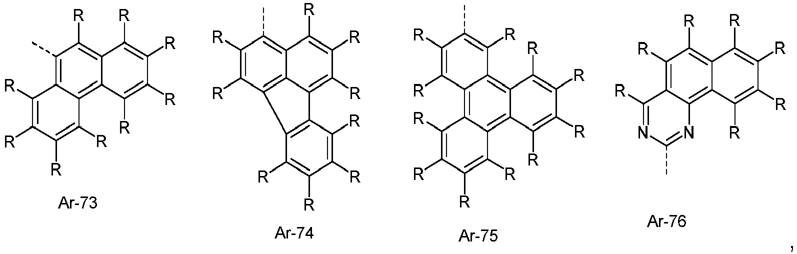 wobei die gestrichelte Linie die Bindung an das Grundgerüst darstellt und weiterhin gilt:
- Ar³: ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R substituiert sein kann;
- Ar²: ist ein aromatisches oder heteroaromatisches Ringsystem mit 5 bis 40 aromatischen Ringatomen, welches mit einem oder mehreren Resten R substituiert sein kann;
- A¹: ist gleich oder verschieden bei jedem Auftreten NAr², O, S oder C(R)₂;
- n: ist 0 oder 1, wobei n = 0 bedeutet, dass an dieser Position keine Gruppe A¹ gebunden ist und an die entsprechenden Kohlenstoffatome stattdessen Reste R gebunden sind;
- m: ist 0 oder 1, wobei m = 0 bedeutet, dass die Gruppe Ar³ nicht vorhanden ist und dass die entsprechende aromatische bzw. heteroaromatische Gruppe direkt an das Stickstoffatom gebunden ist.

Bevorzugte Ausführungsformen der Verbindungen der Formeln (4) bzw. (5) sind die Verbindungen der folgenden Formeln (4a) bzw. (5a), 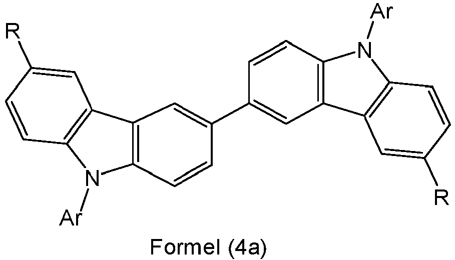 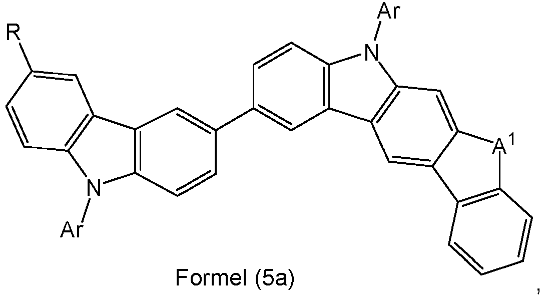 wobei die verwendeten Symbole die oben genannten Bedeutungen gemäß Formel (4) und Formel (5) aufweisen.

Beispiele für geeignete Verbindungen gemäß Formel (4) oder (5) sind die nachfolgend abgebildeten Verbindungen.

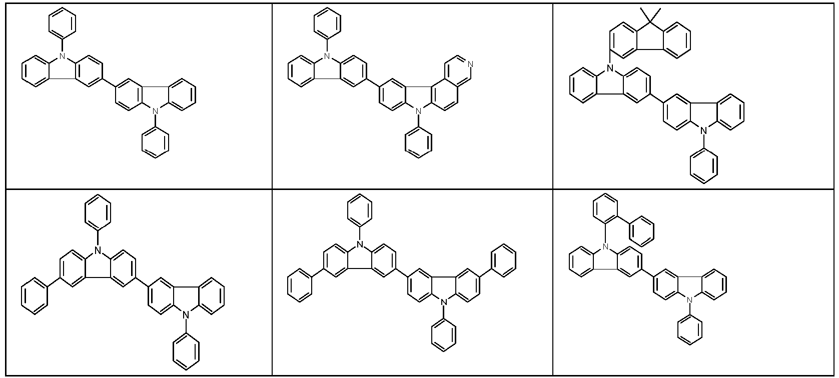

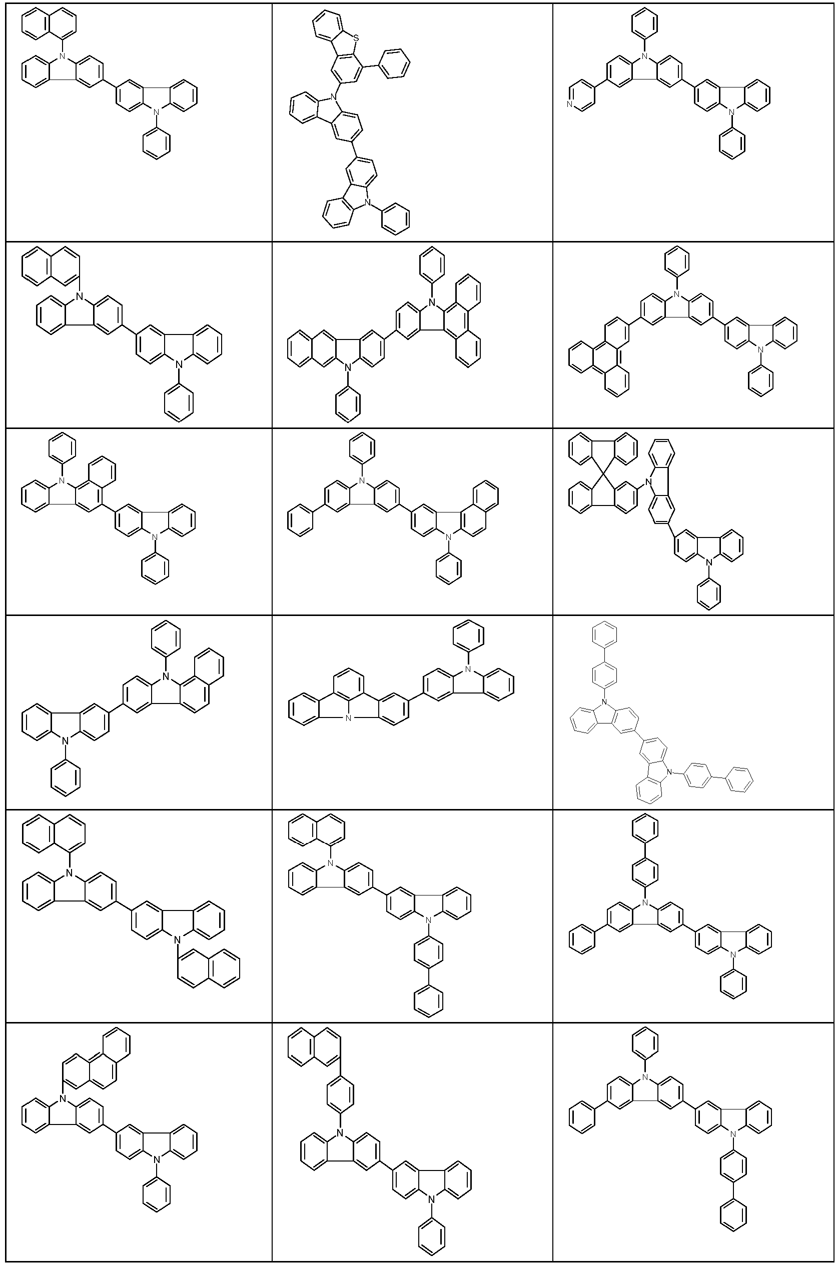

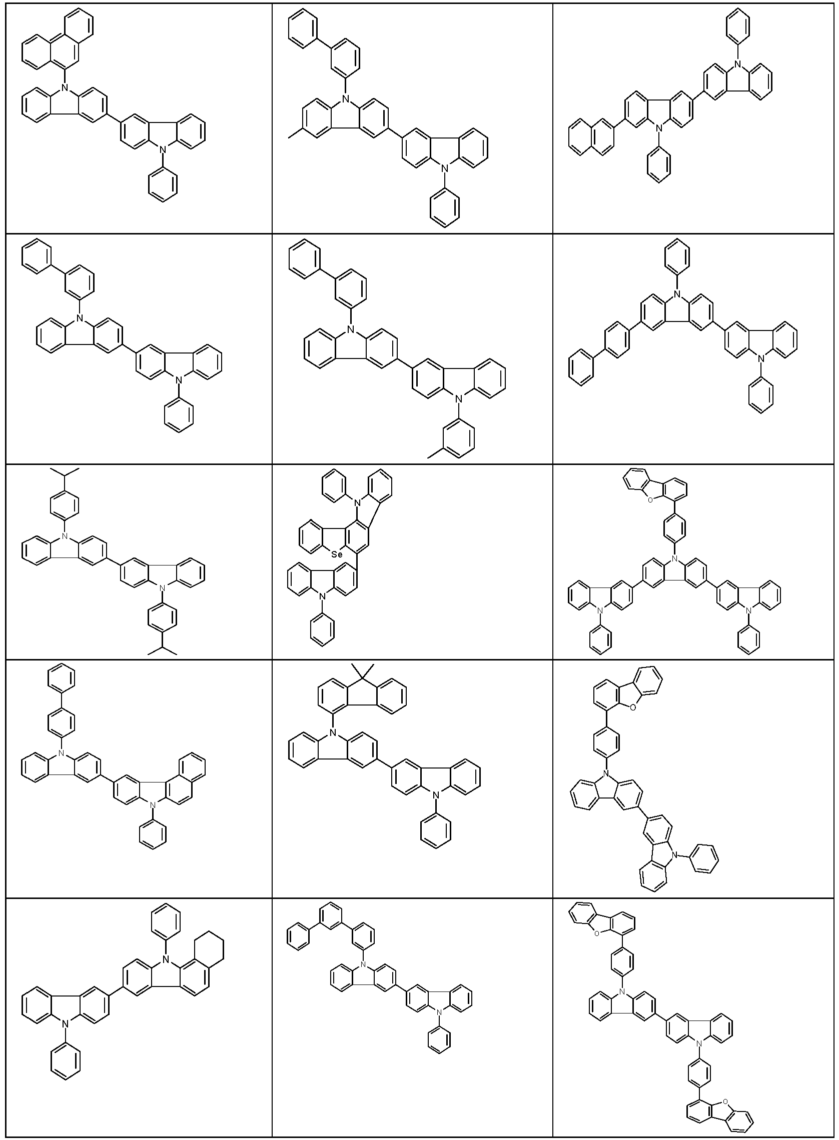

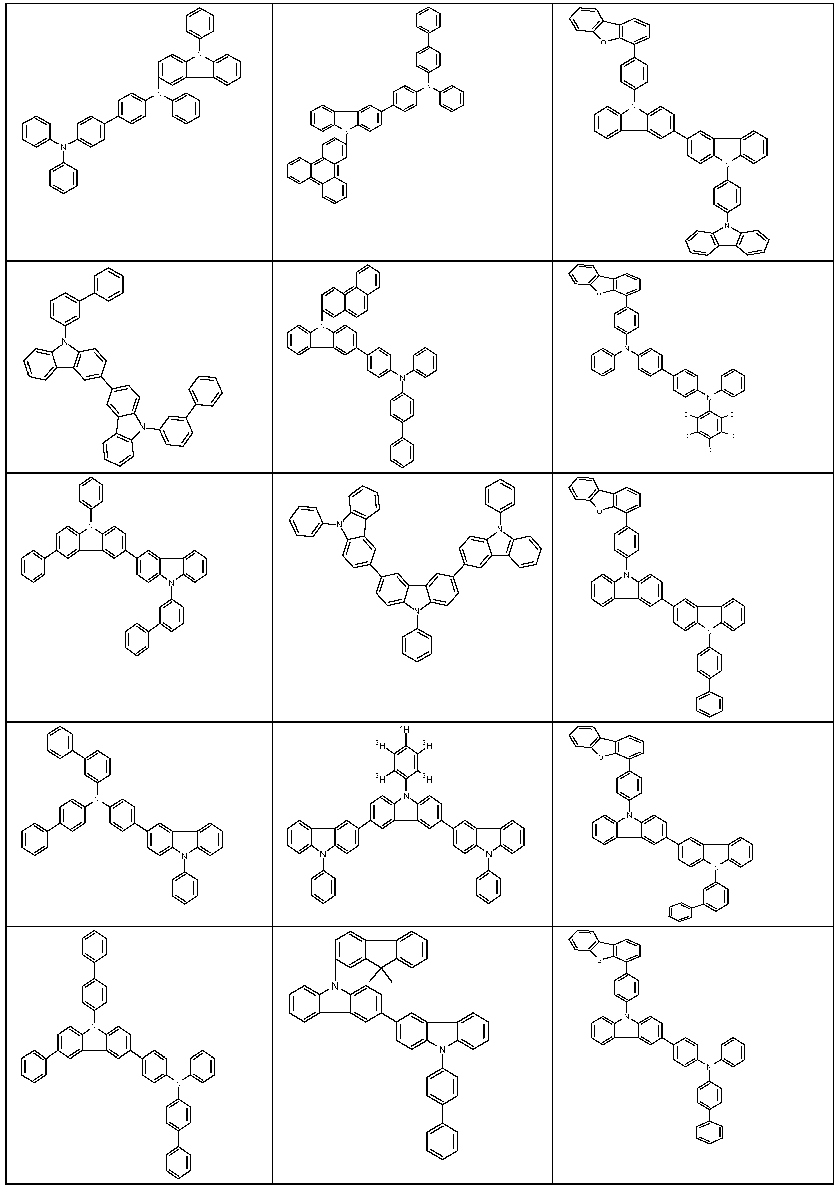

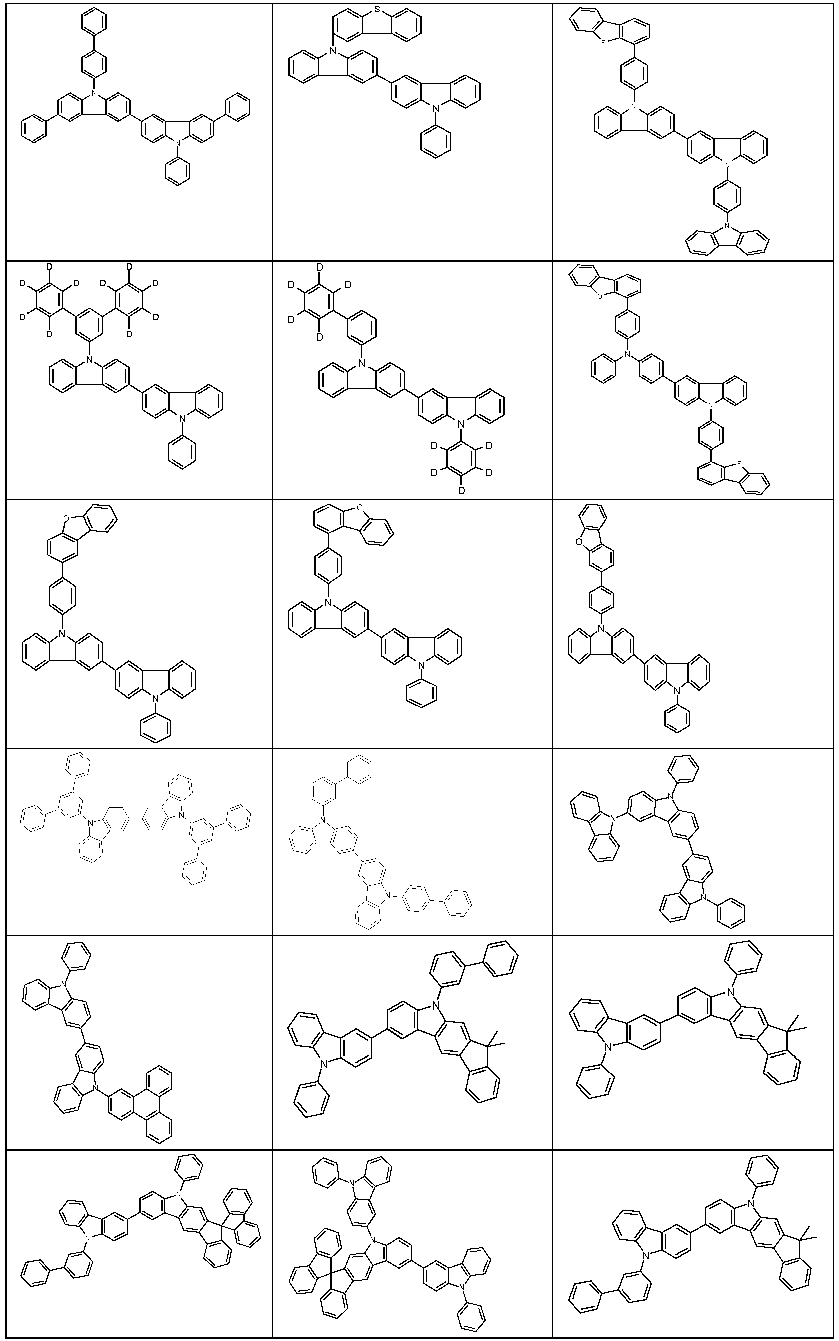

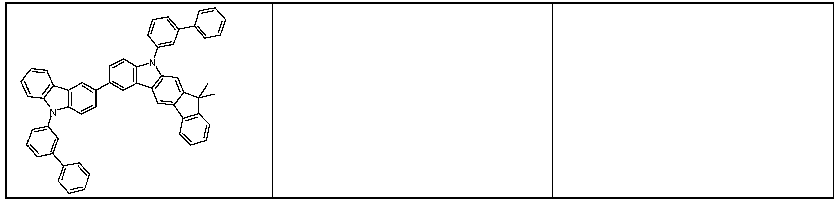

Bevorzugte verbrückte Carbazole sind die Strukturen der folgenden Formel (6), 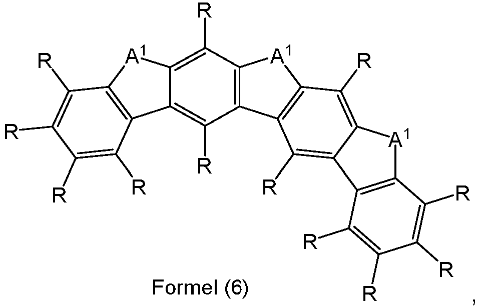 wobei A¹ und R die oben genannten Bedeutungen aufweisen gemäß der Formeln (4) und (5) und A¹ bevorzugt gleich oder verschieden bei jedem Auftreten ausgewählt ist aus der Gruppe bestehend aus NAr und CR₂.

Bevorzugte Dibenzofuran-Derivate sind die Verbindungen der folgenden Formel (7), 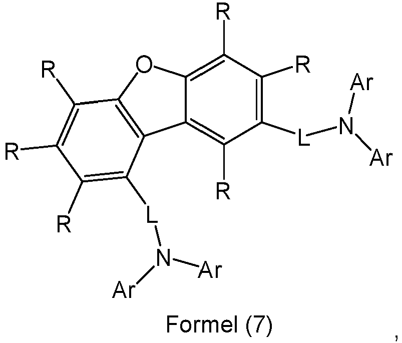 wobei der Sauerstoff auch durch Schwefel ersetzt sein kann, sodass ein Dibenzothiophen entsteht, L für eine Einfachbindung oder ein aromatisches oder heteroaromatisches Ringsystem mit 5 bis 30 aromatischen Ringatomen steht, welches auch durch eine oder mehrere Reste R substituiert sein kann, und R und Ar die oben genannten Bedeutungen aufweisen. Dabei können die beiden Gruppen Ar, die an dasselbe Stickstoffatom binden, oder eine Gruppe Ar und eine Gruppe L, die an dasselbe Stickstoffatom binden, auch miteinander verbunden sein, beispielsweise zu einem Carbazol.

Beispiele für geeignete Dibenzofuran-Derivate sind die nachfolgend abgebildeten Verbindungen.

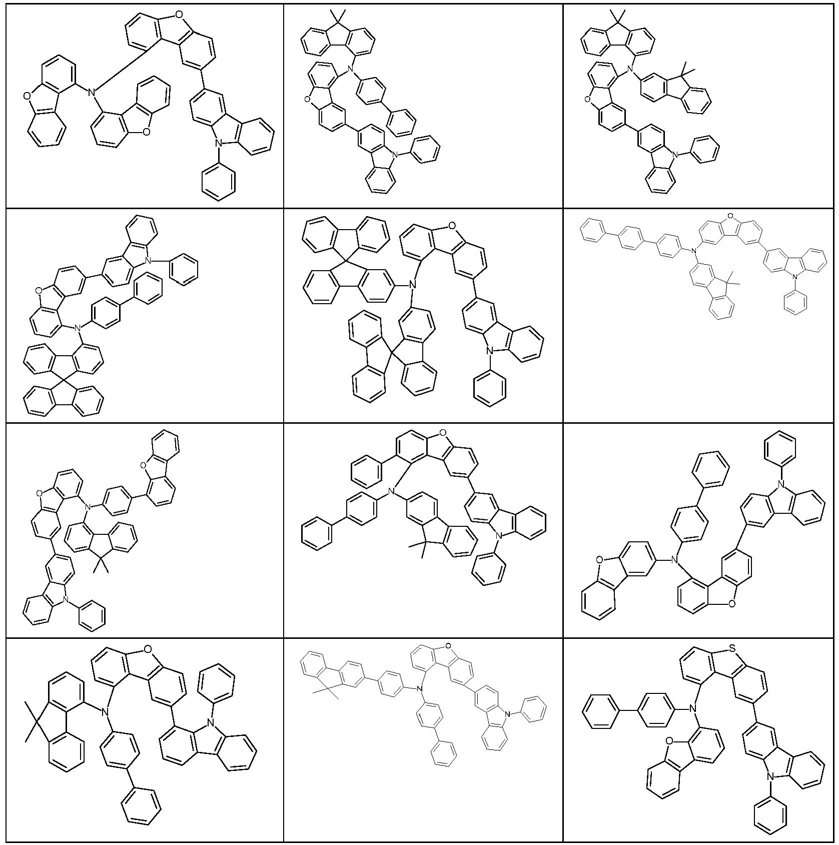

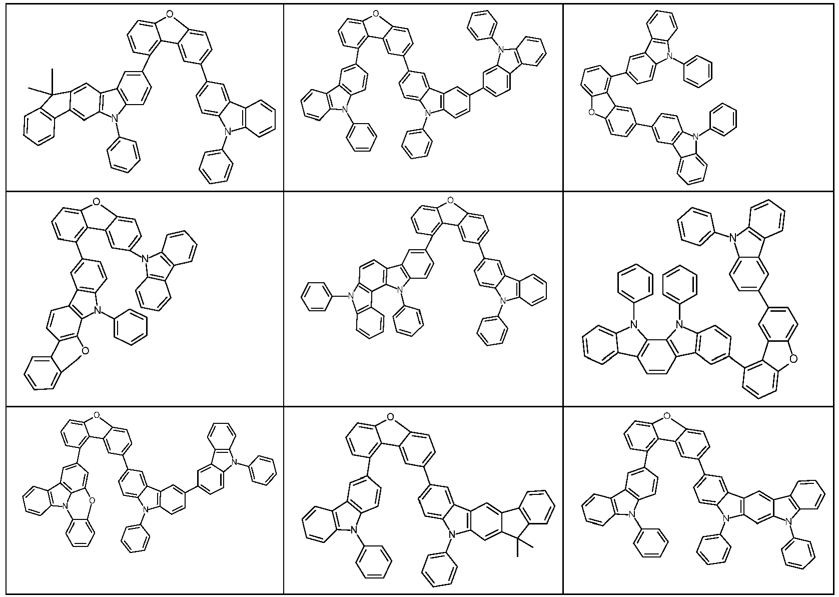

Bevorzugte Carbazolamine sind die Strukturen der folgenden Formeln (8), (9) und (10), 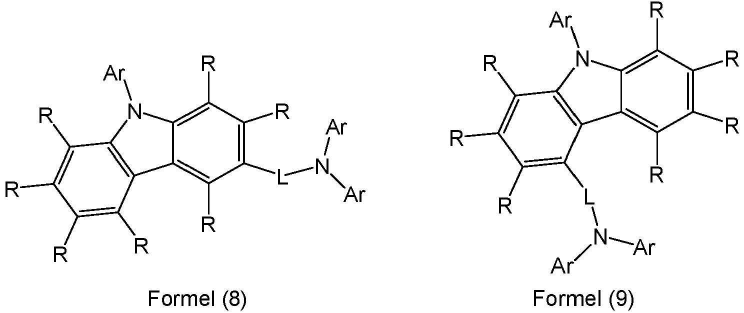 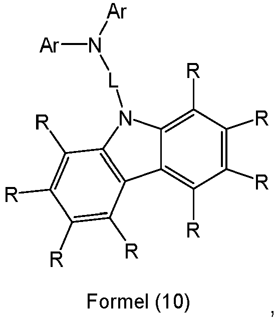 wobei L für ein aromatisches oder heteroaromatisches Ringsystem mit 5 bis 30 aromatischen Ringatomen steht, welches mit einem oder mehreren Resten R substituiert sein kann, und R und Ar die oben genannten Bedeutungen gemäß Formel (4) oder Formel (5) aufweisen.

Beispiele für geeignete Carbazolamin-Derivate sind die nachfolgend abgebildeten Verbindungen.

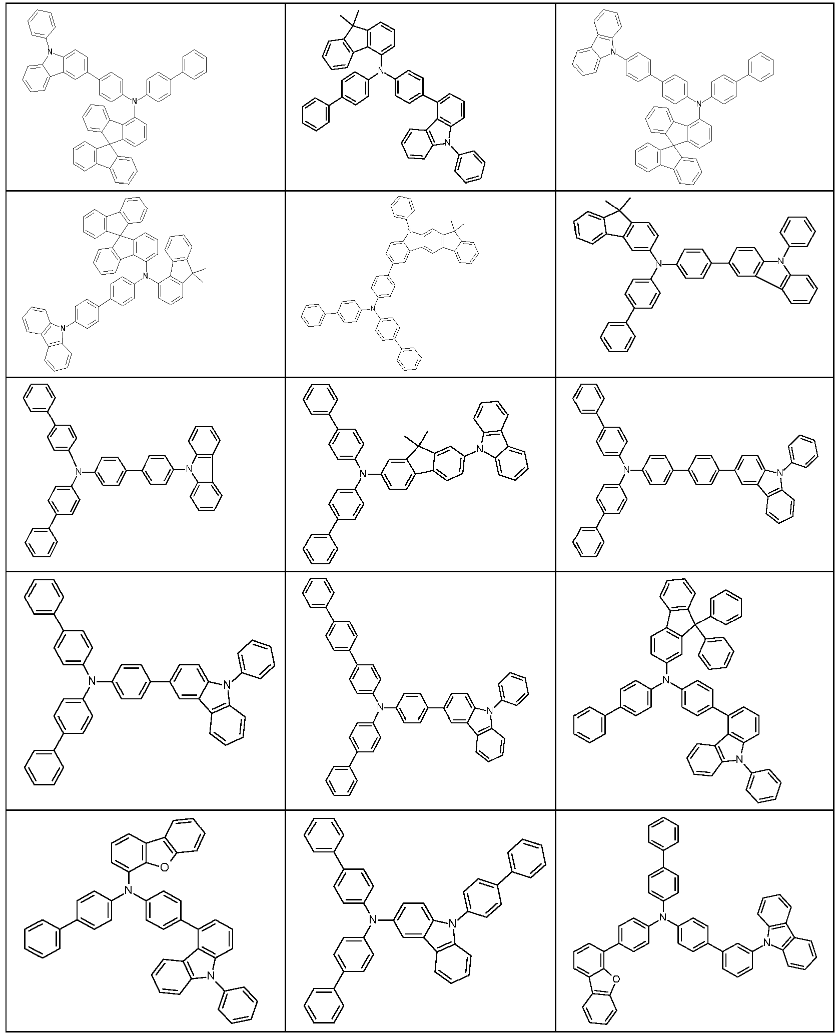

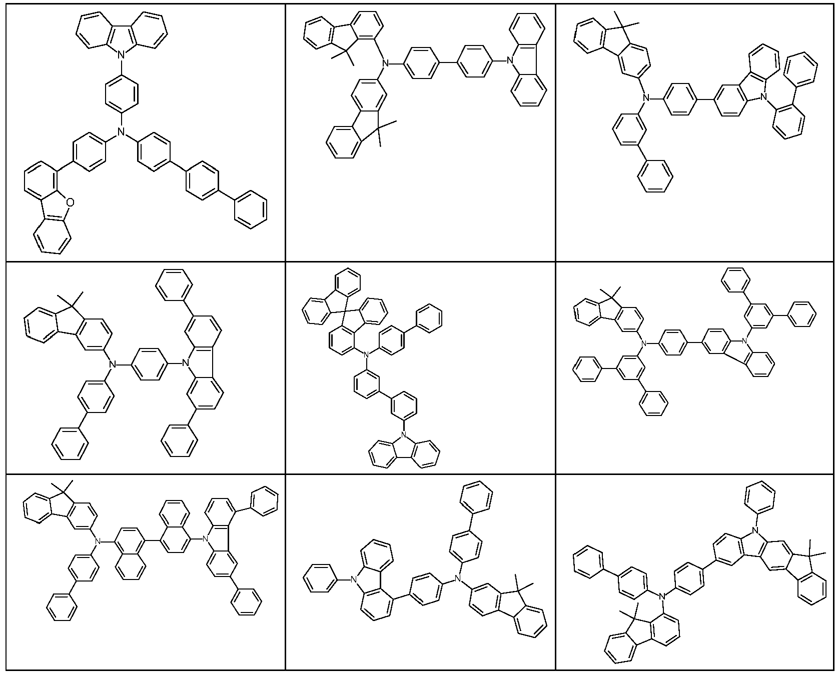

Weitere besonders bevorzugte Co-Matrixmaterialien, insbesondere lochtransportierende Co-Hosts, insbesondere wenn die erfindungsgemäße Verbindung mit einem elektronenarmen heteroaromatischen Ringsystem substituiert ist, sind in folgender Tabelle gezeigt:

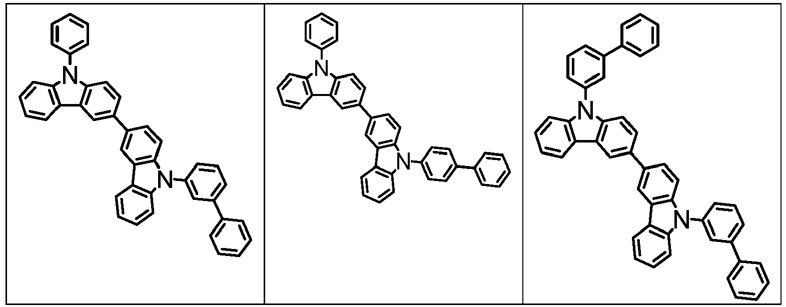

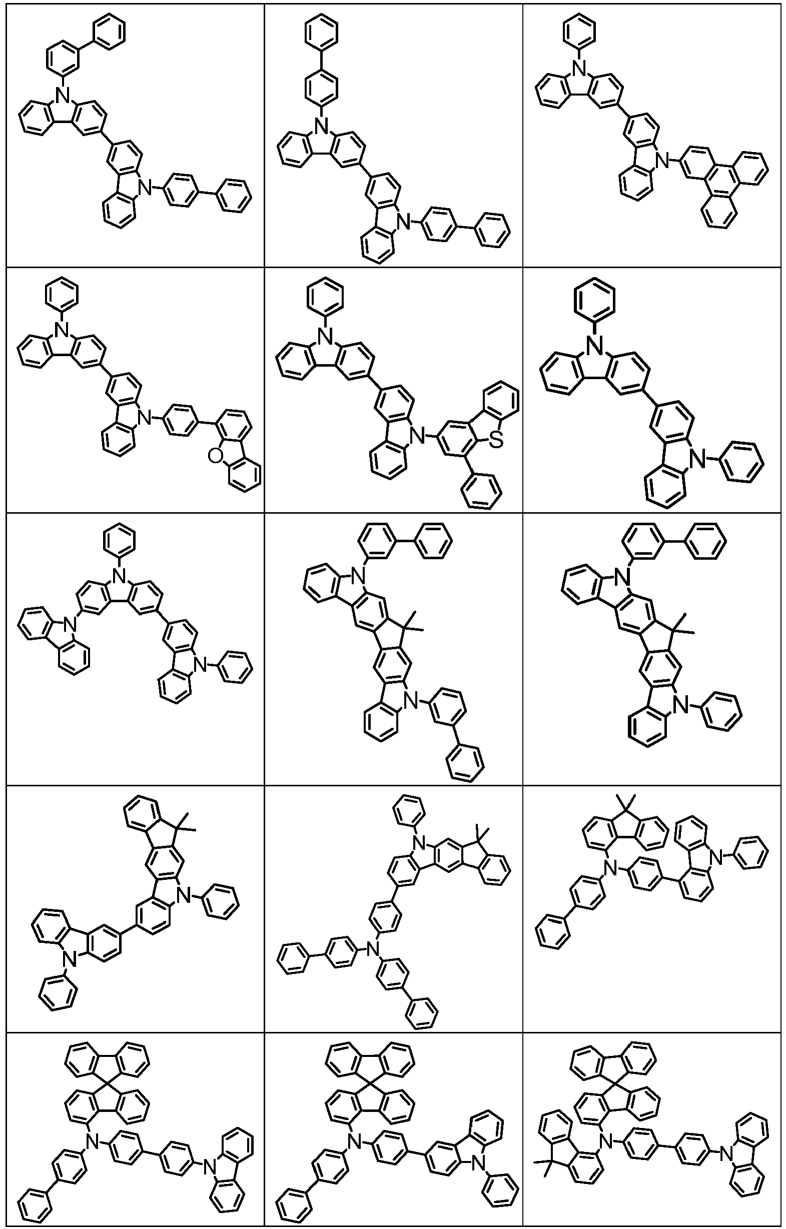

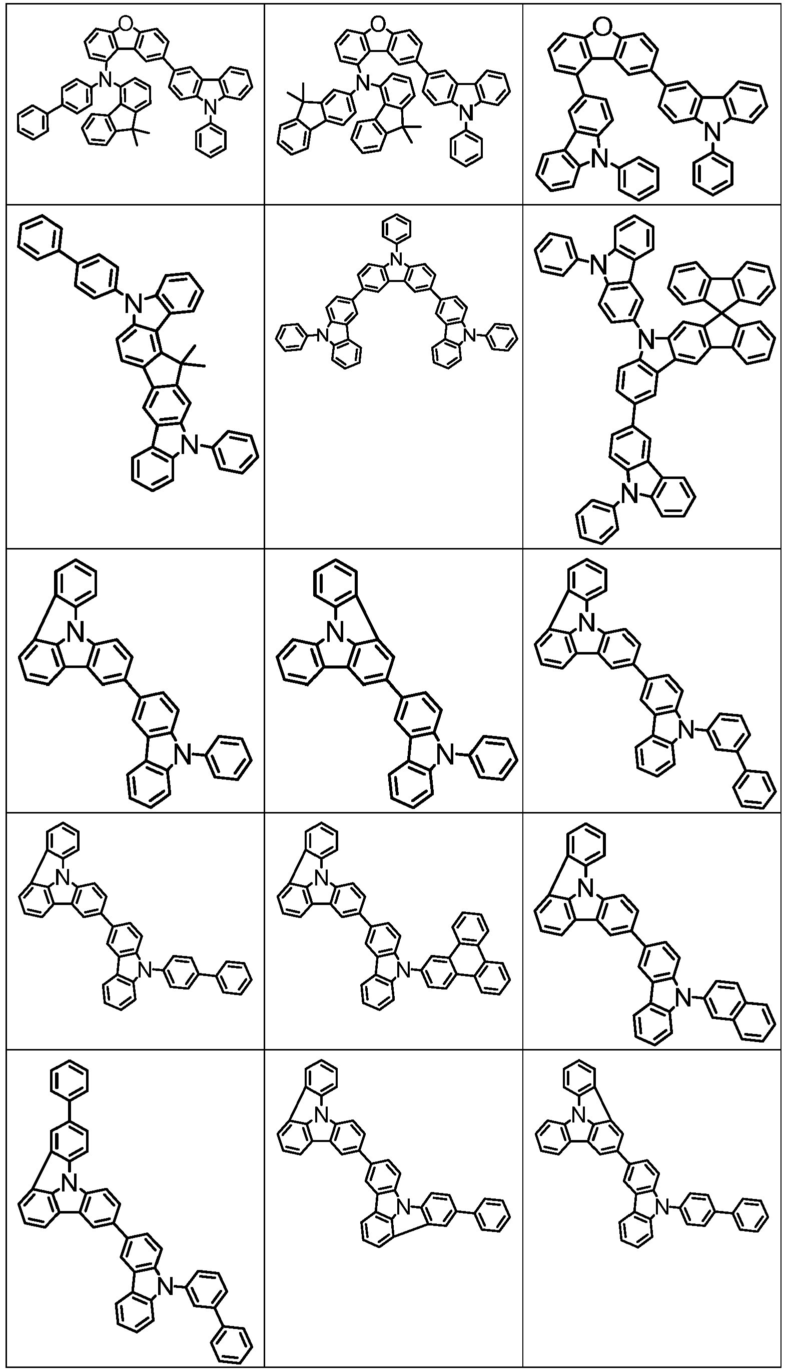

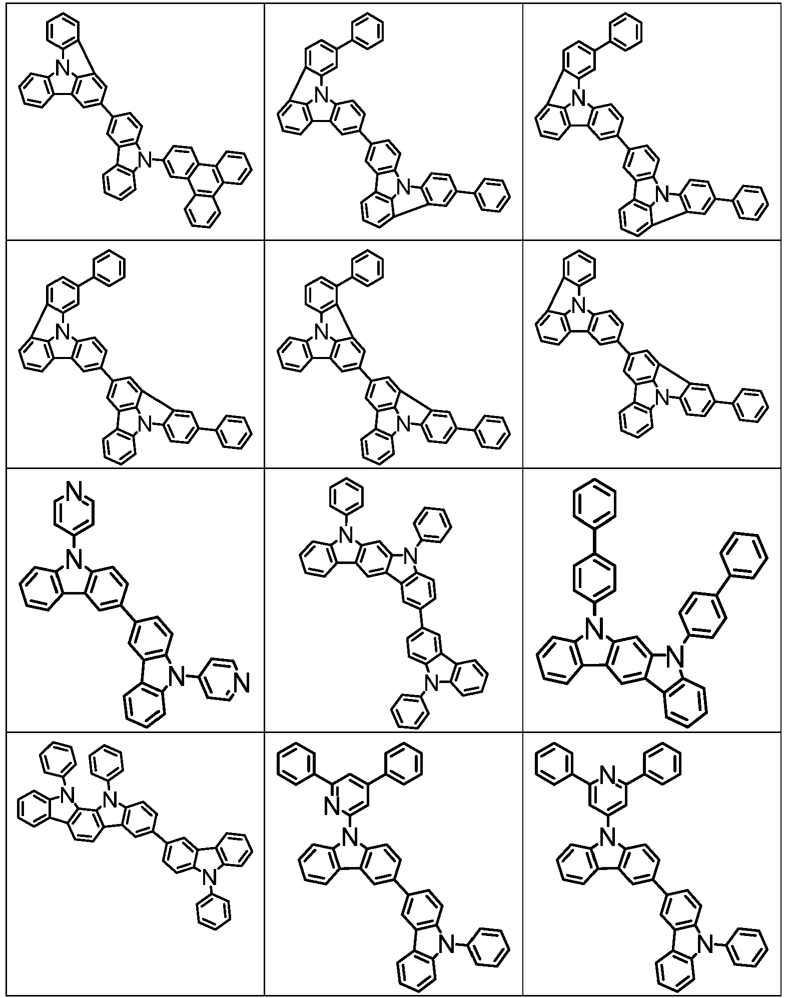

Bevorzugte Co-Matrix-Materialien, insbesondere wenn die erfindungsgemäße Verbindung mit einem elektronenreichen heteroaromatischen Ringsystem, beispielsweise einer Carbazolgruppe, substituiert ist, sind weiterhin ausgewählt aus der Gruppe bestehend aus Triazin-Derivaten, Pyrimidin-Derivaten, Chinazolin-Derivaten und Chinoxalin-Derivaten. Bevorzugte Triazin-, Chinazolin-, Chinoxalin- bzw. Pyrimidinderivate, welche als Mischung zusammen mit den erfindungsgemäßen Verbindungen eingesetzt werden können, sind die Verbindungen der folgenden Formeln (11), (12), (13) und (14), 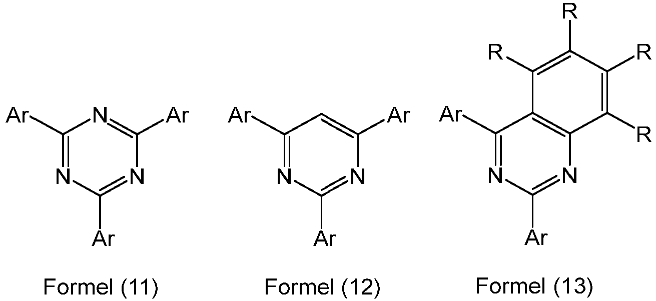 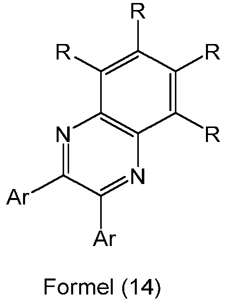 wobei Ar und R die oben genannten Bedeutungen gemäß der Formeln (4) und (5) aufweisen.

Besonders bevorzugt sind die Triazinderivate der Formel (11) und die Chinazolinderivate der Formel (13), insbesondere die Triazinderivate der Formel (11).

In einer bevorzugten Ausführungsform der Erfindung ist Ar in den Formeln (11), (12), (13) und (14) bei jedem Auftreten gleich oder verschieden ein aromatisches oder heteroaromatisches Ringsystem mit 6 bis 30 aromatischen Ringatomen, insbesondere mit 6 bis 24 aromatischen Ringatomen, das durch einen oder mehrere Reste R substituiert sein kann. Dabei sind geeignete aromatische bzw. heteroaromatische Ringsysteme Ar die gleichen, wie sie oben als Ausführungsformen für Ar ausgeführt sind, insbesondere die Strukturen Ar-1 bis Ar-76.

Beispiele für geeignete Triazinverbindungen, welche als Matrixmaterialien zusammen mit den erfindungsgemäßen Verbindungen eingesetzt werden können, sind die in der folgenden Tabelle abgebildeten Verbindungen.

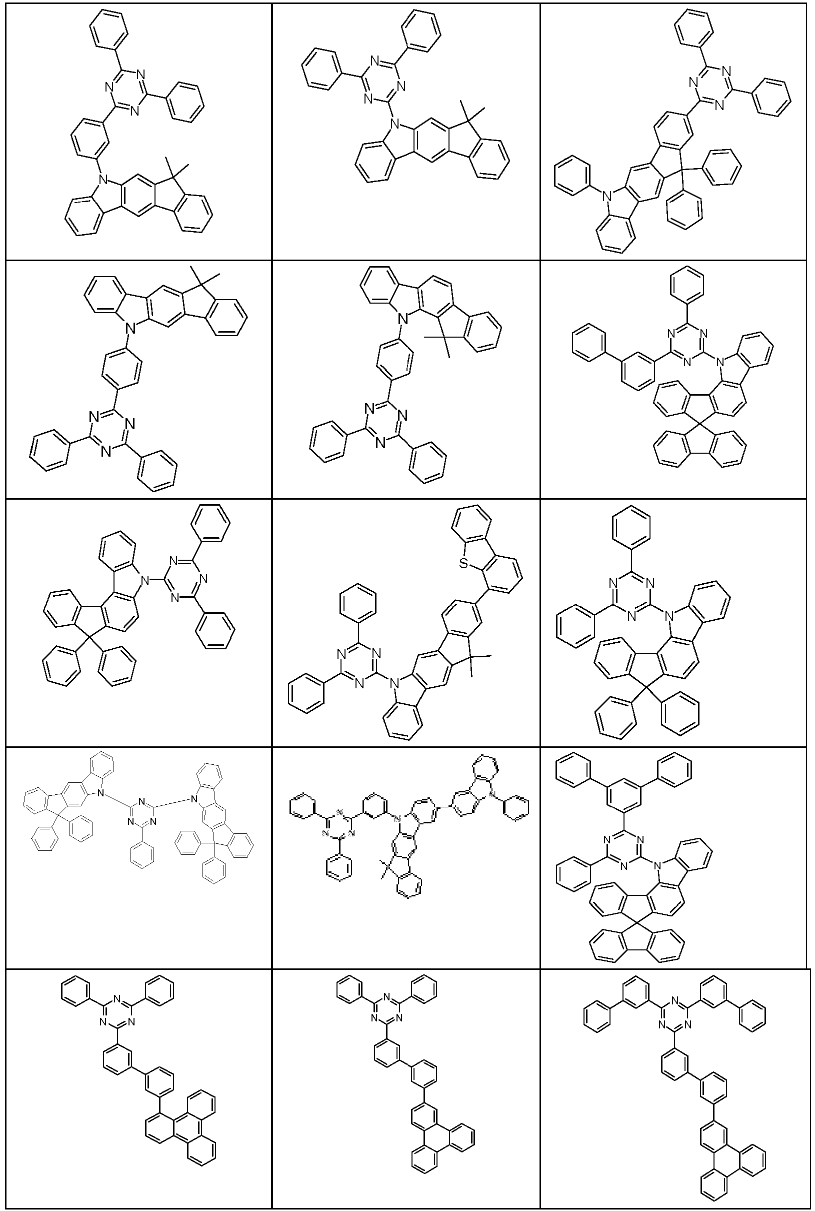

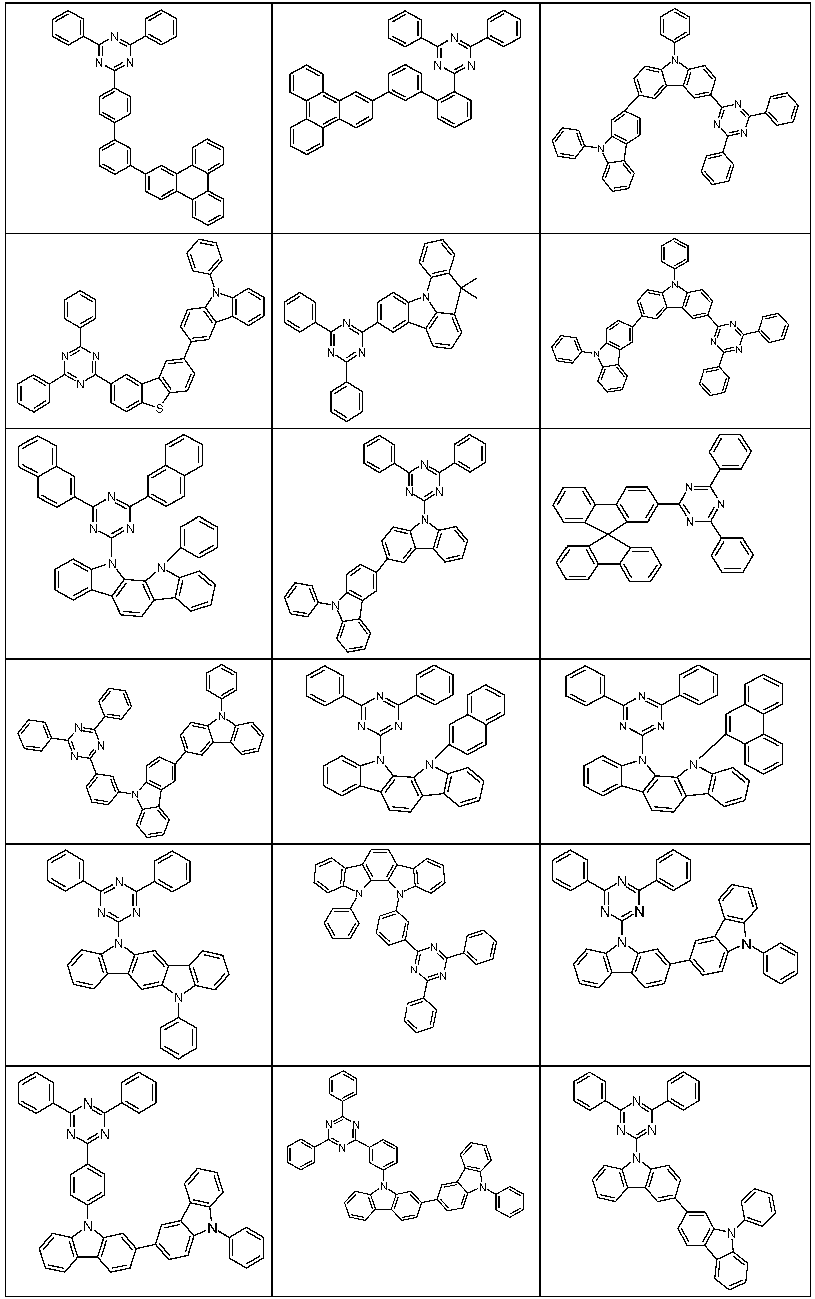

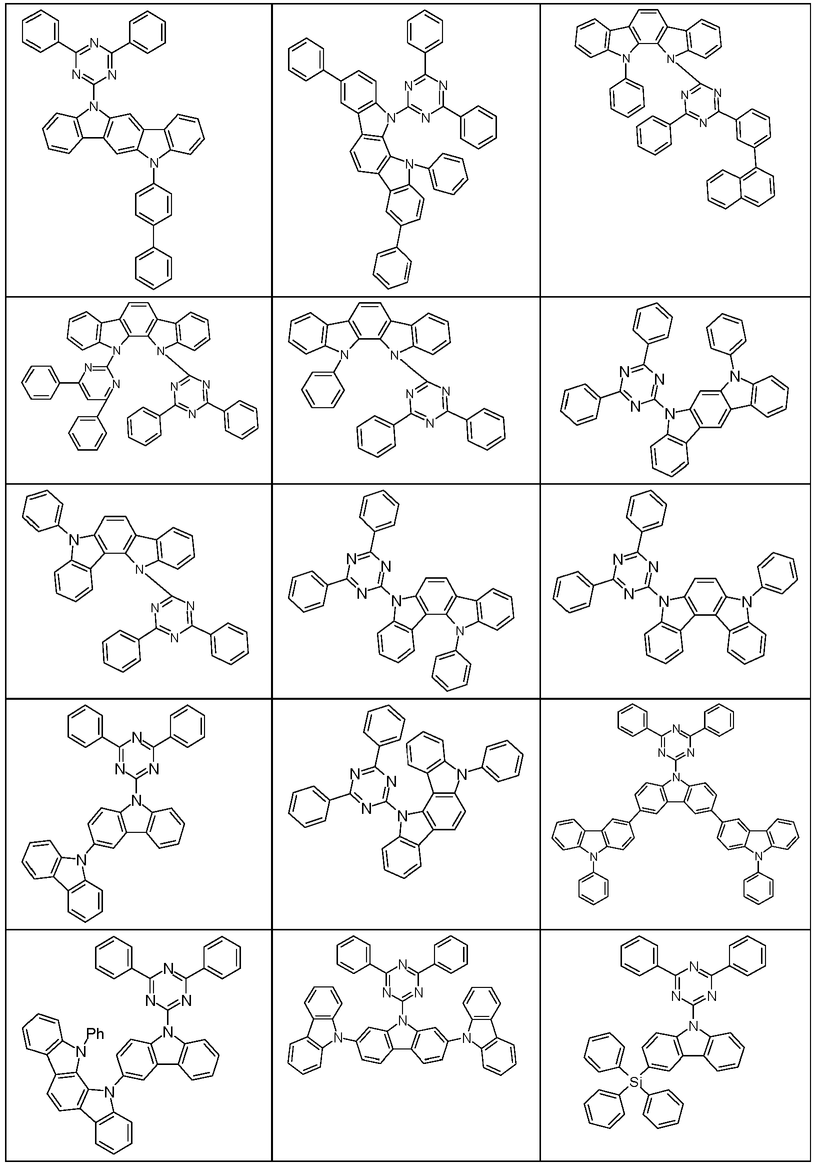

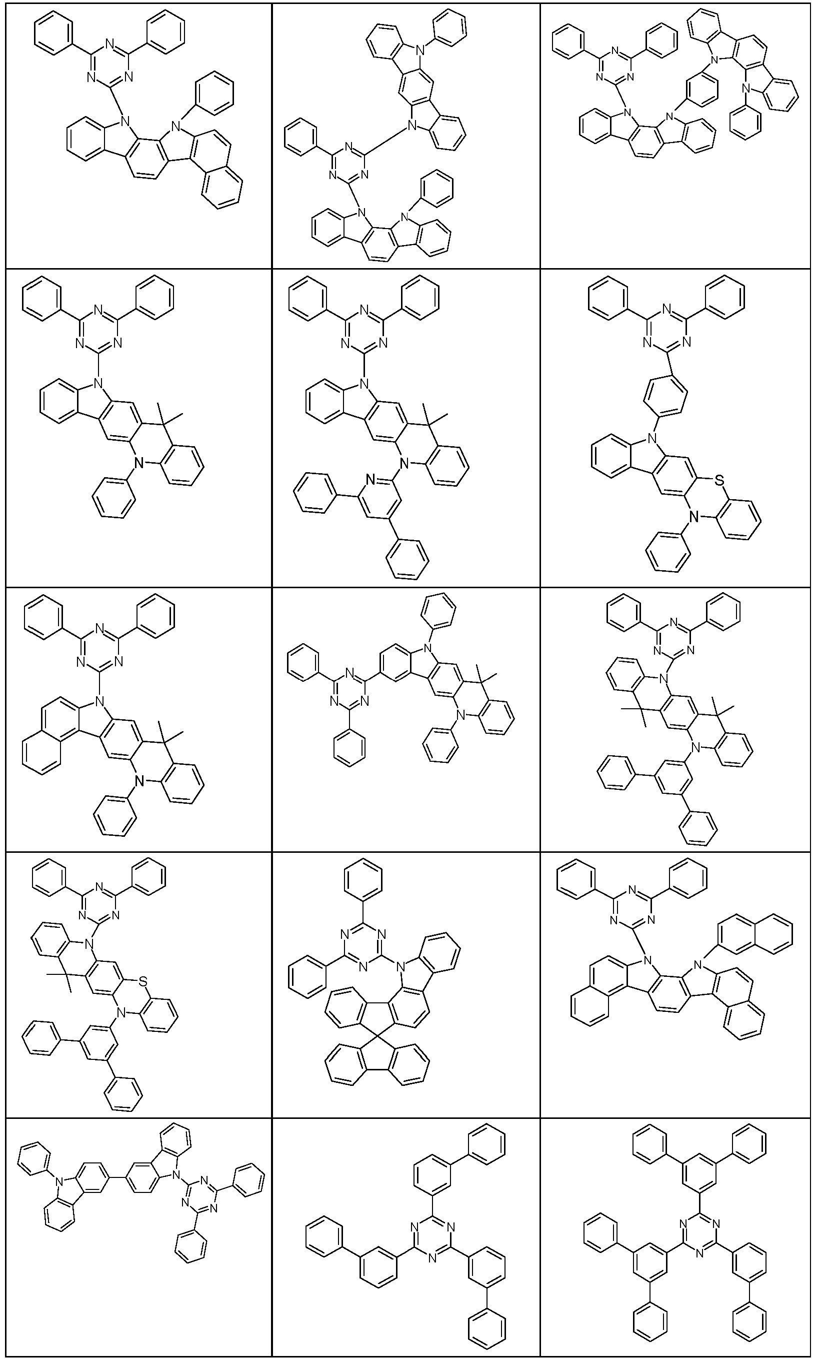

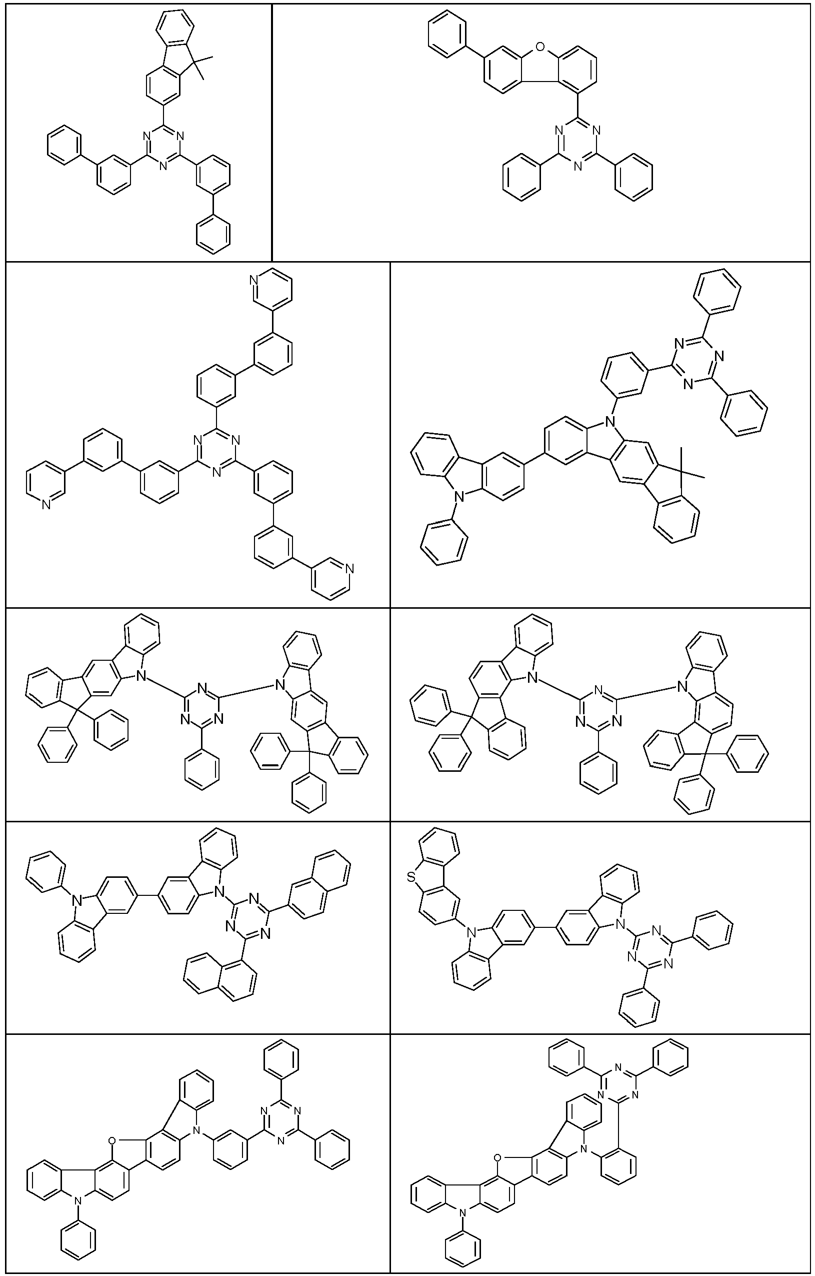

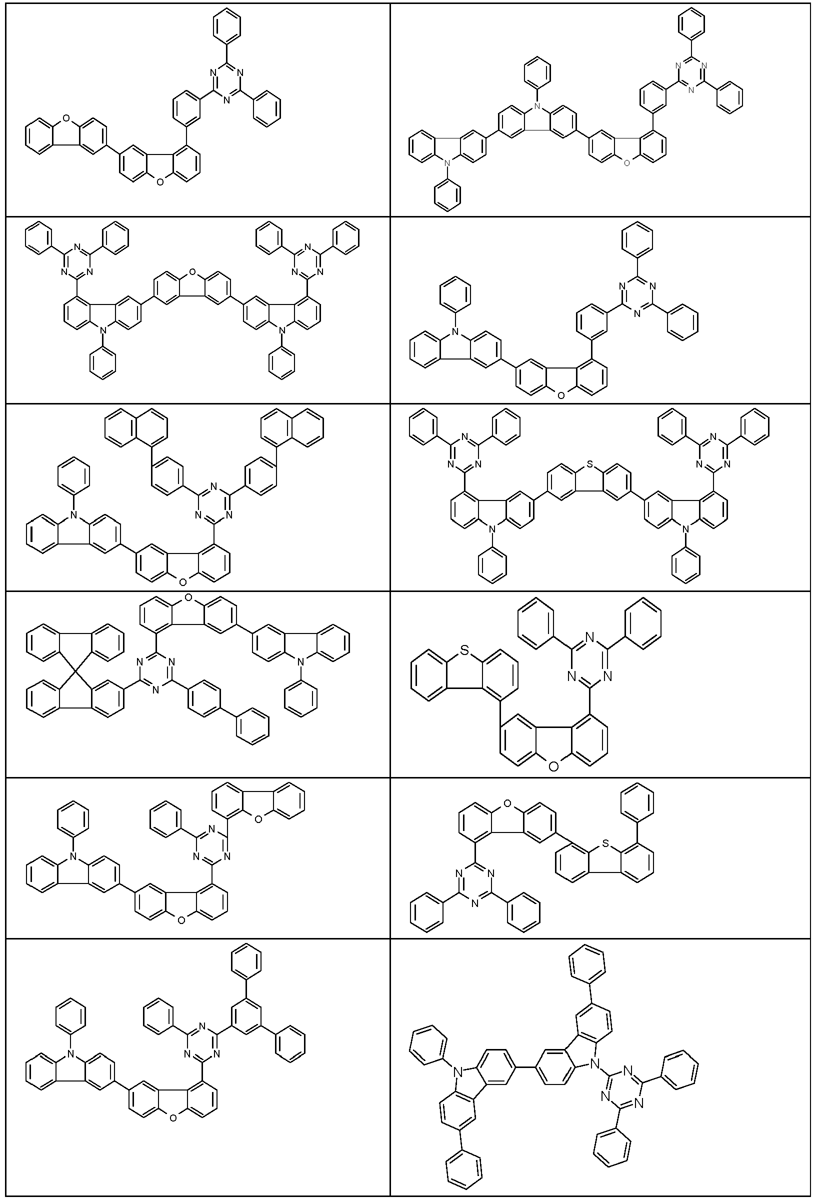

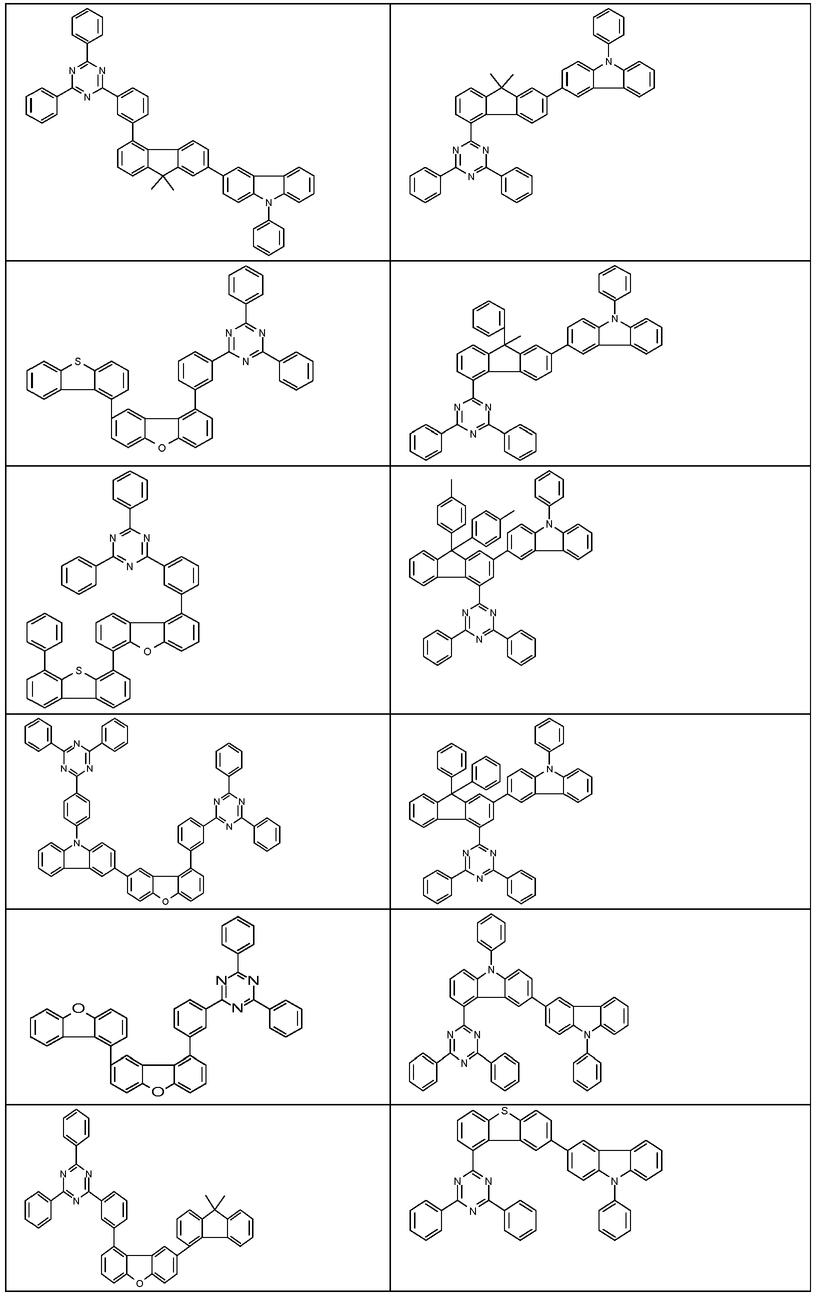

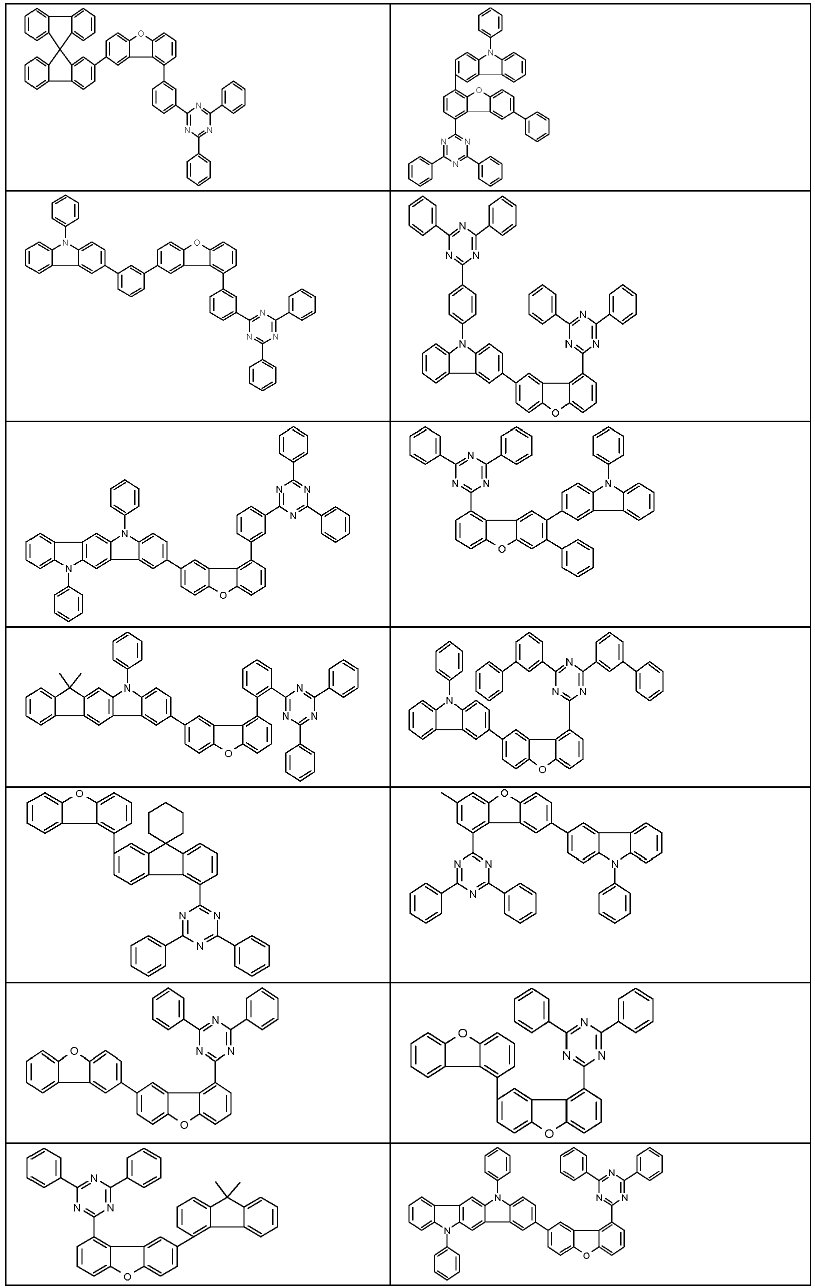

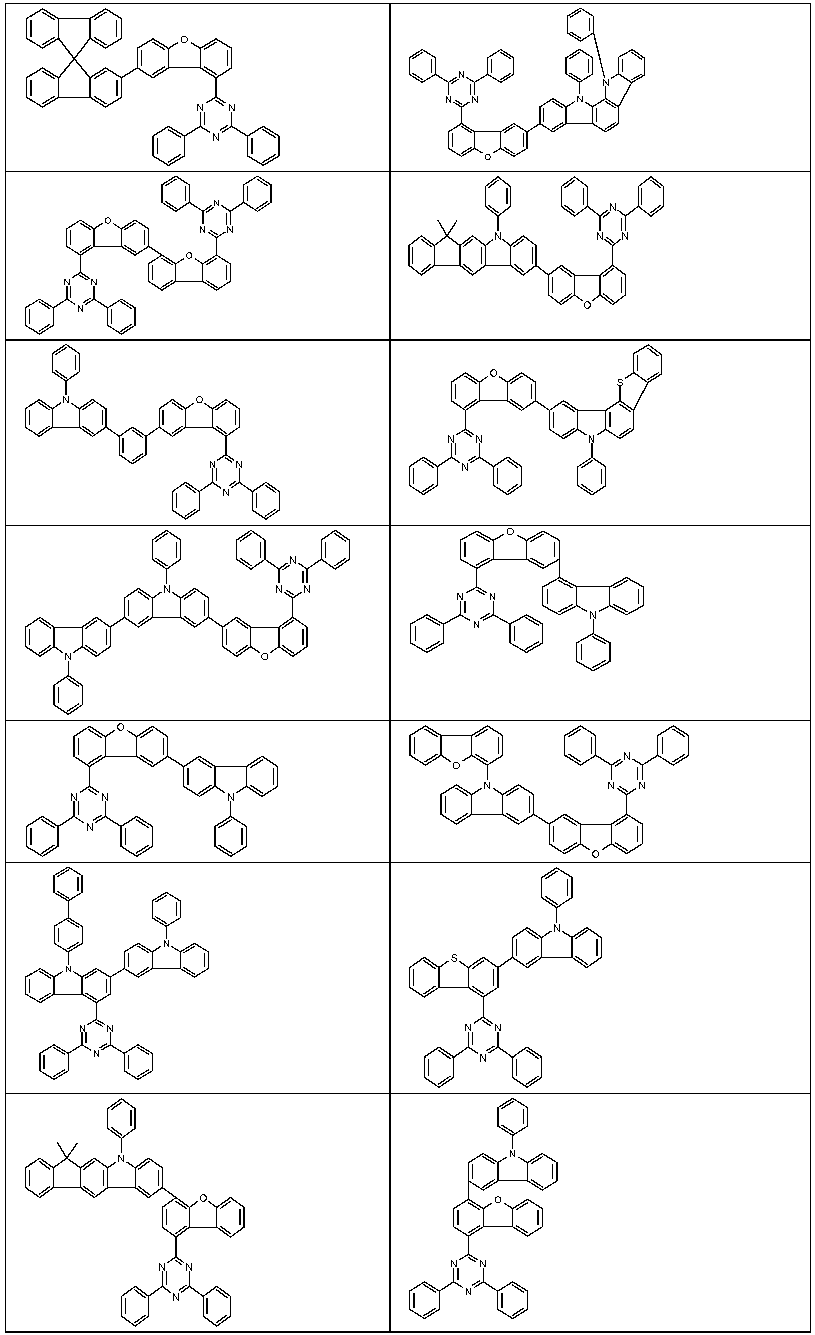

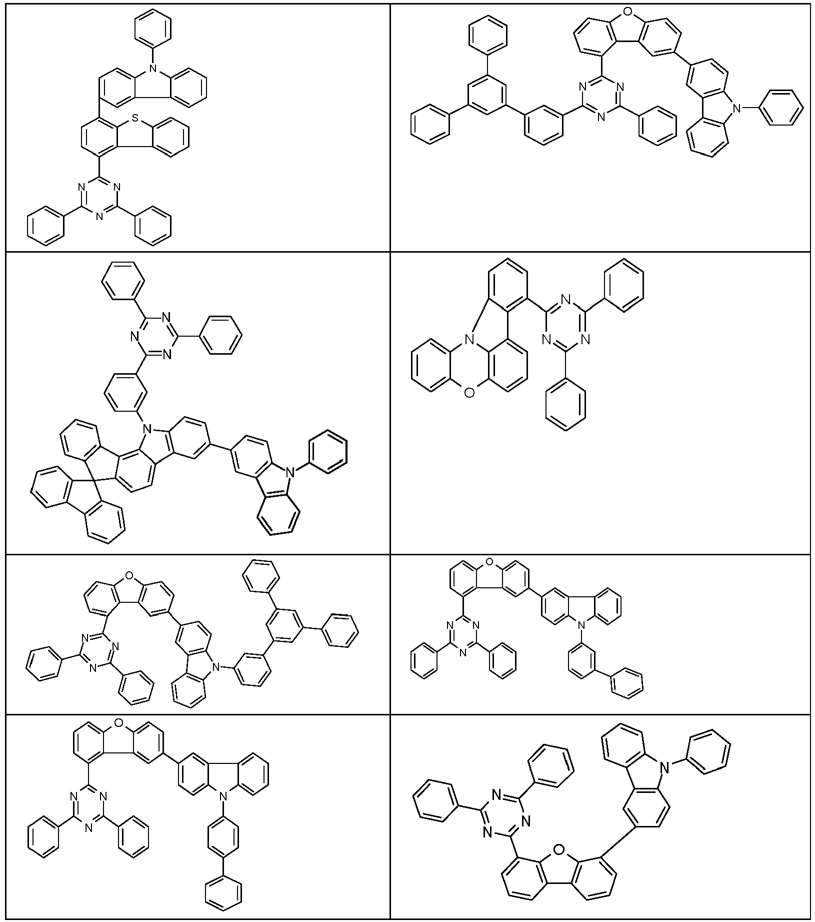

Beispiele für geeignete Chinazolinverbindungen sind die in der folgenden Tabelle abgebildeten Verbindungen:

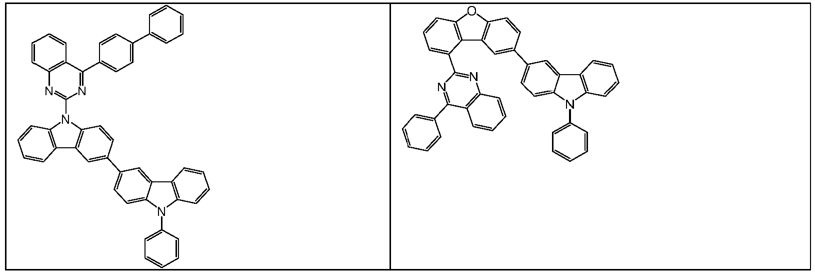

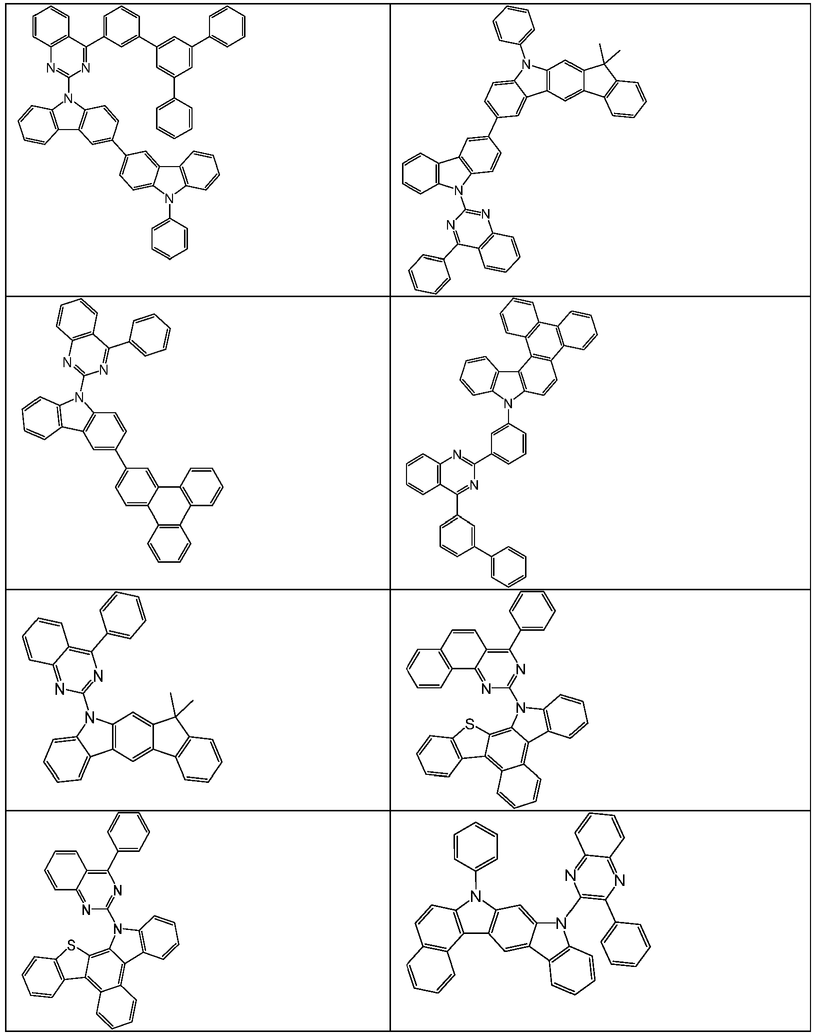

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439, WO 2018/011186, WO 2018/041769, WO 2019/020538, WO 2018/178001, WO 2019/115423 und WO 2019/158453 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Beispiele für phosphoreszierende Dotanden sind nachfolgend aufgeführt.

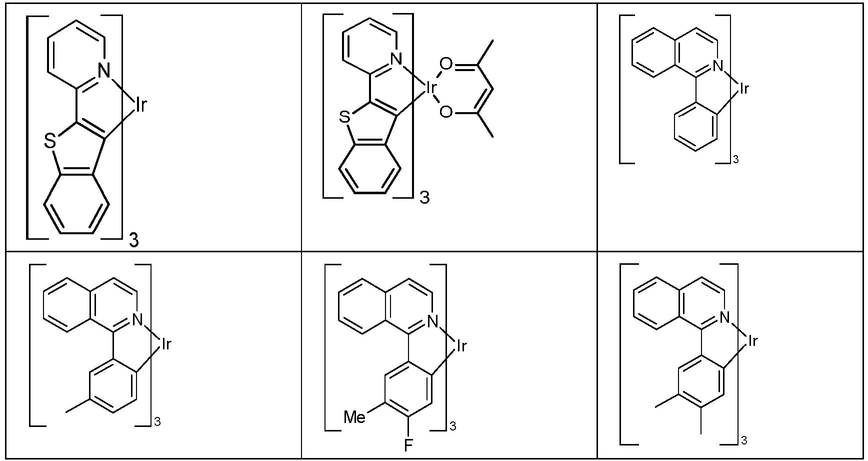

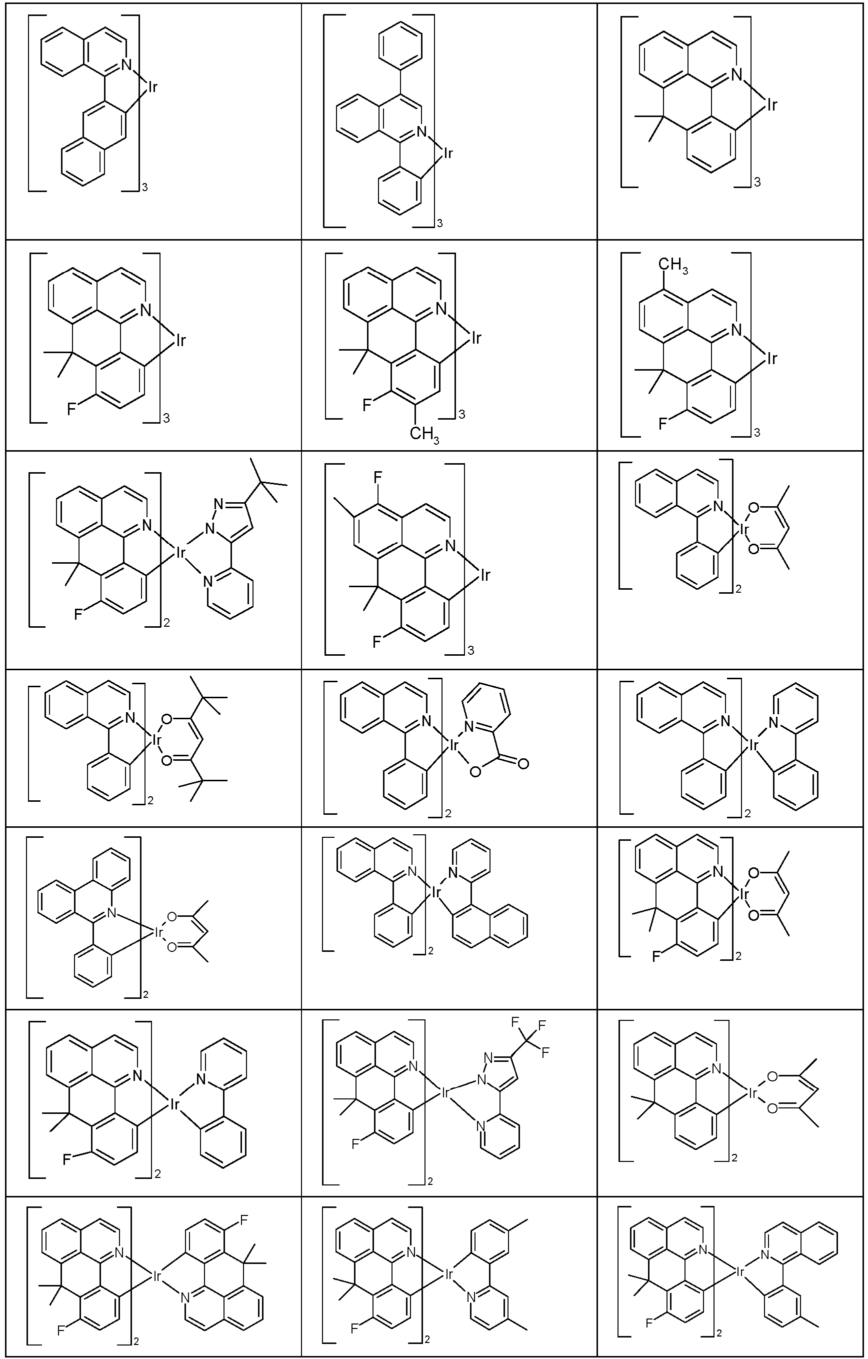

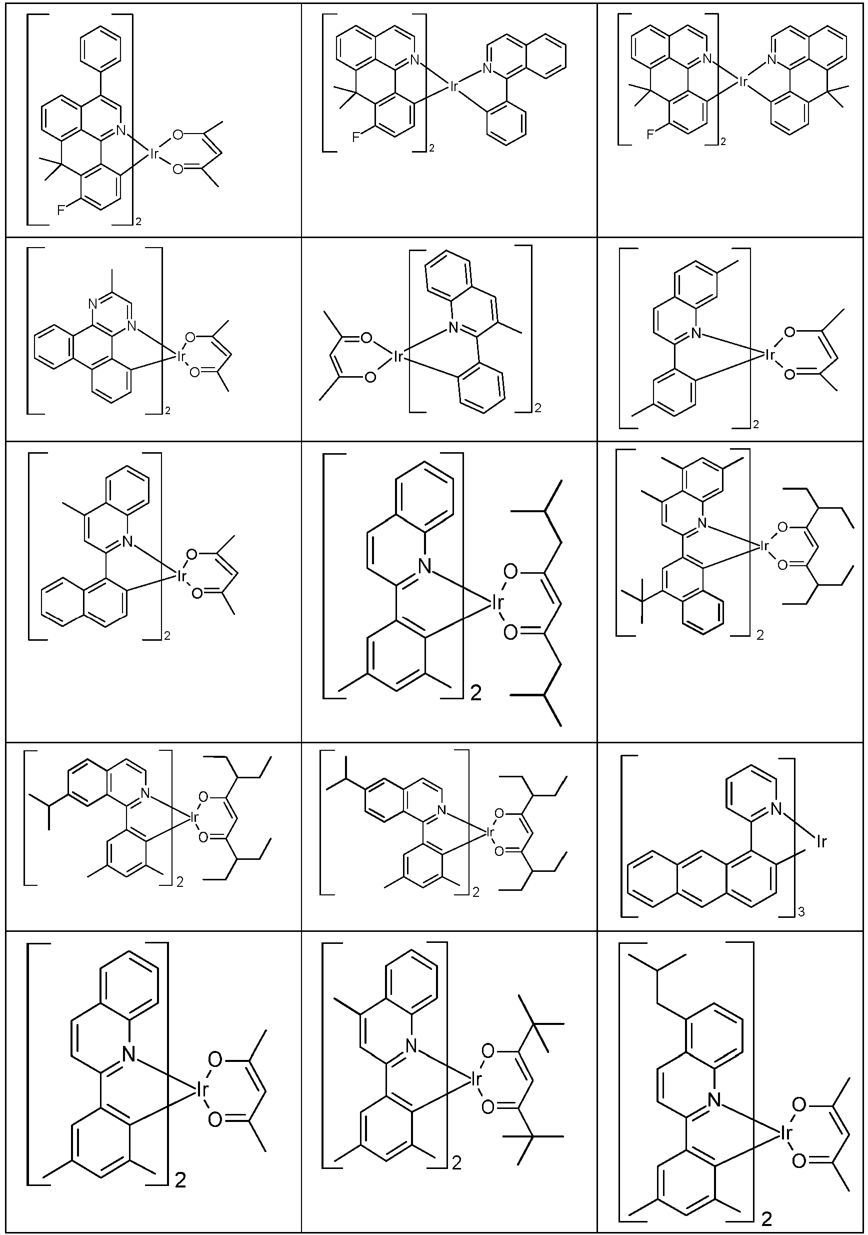

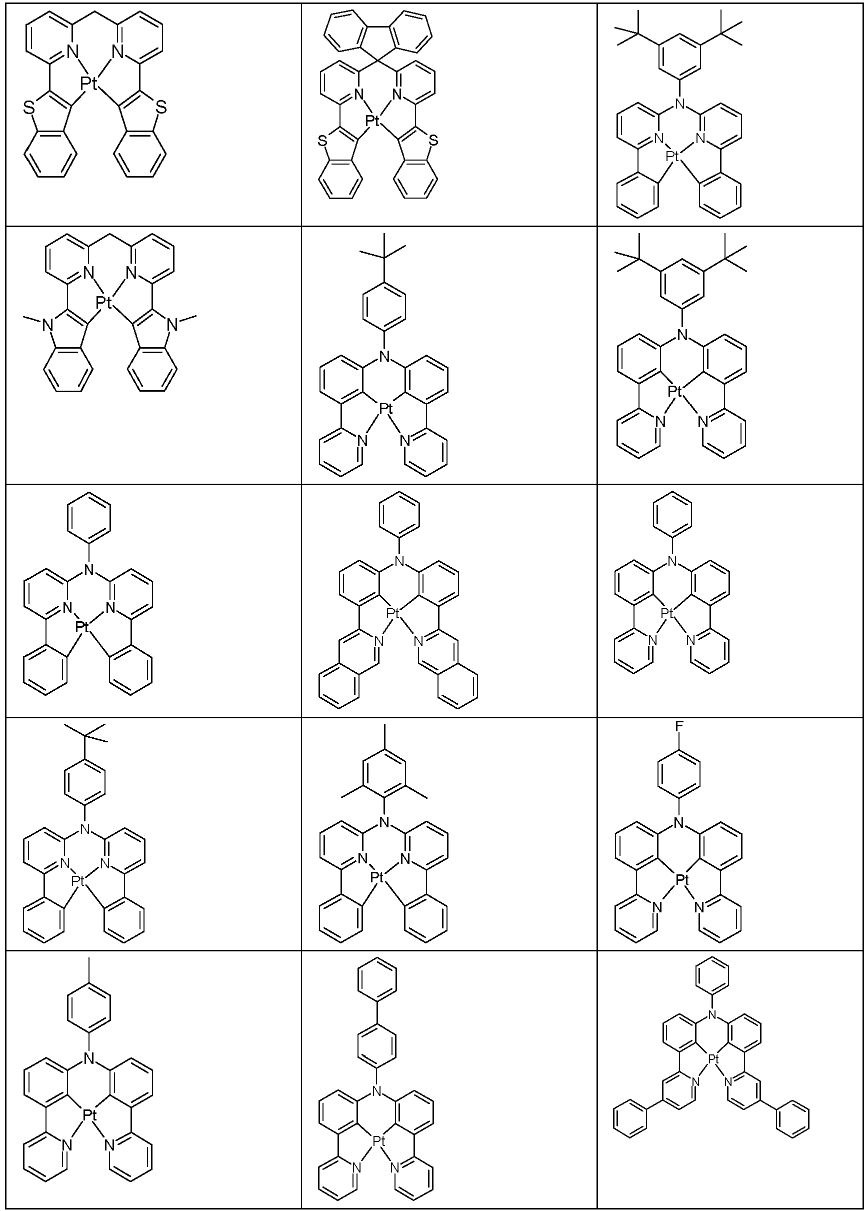

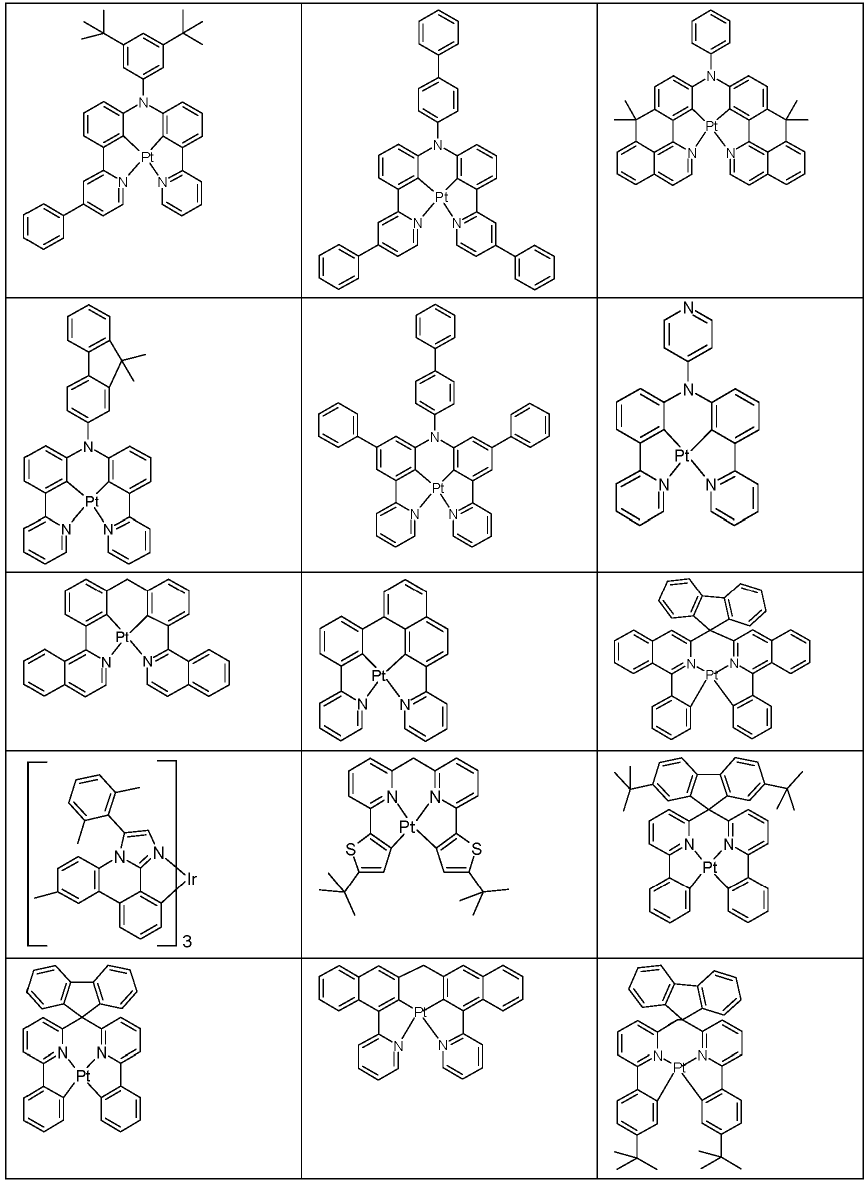

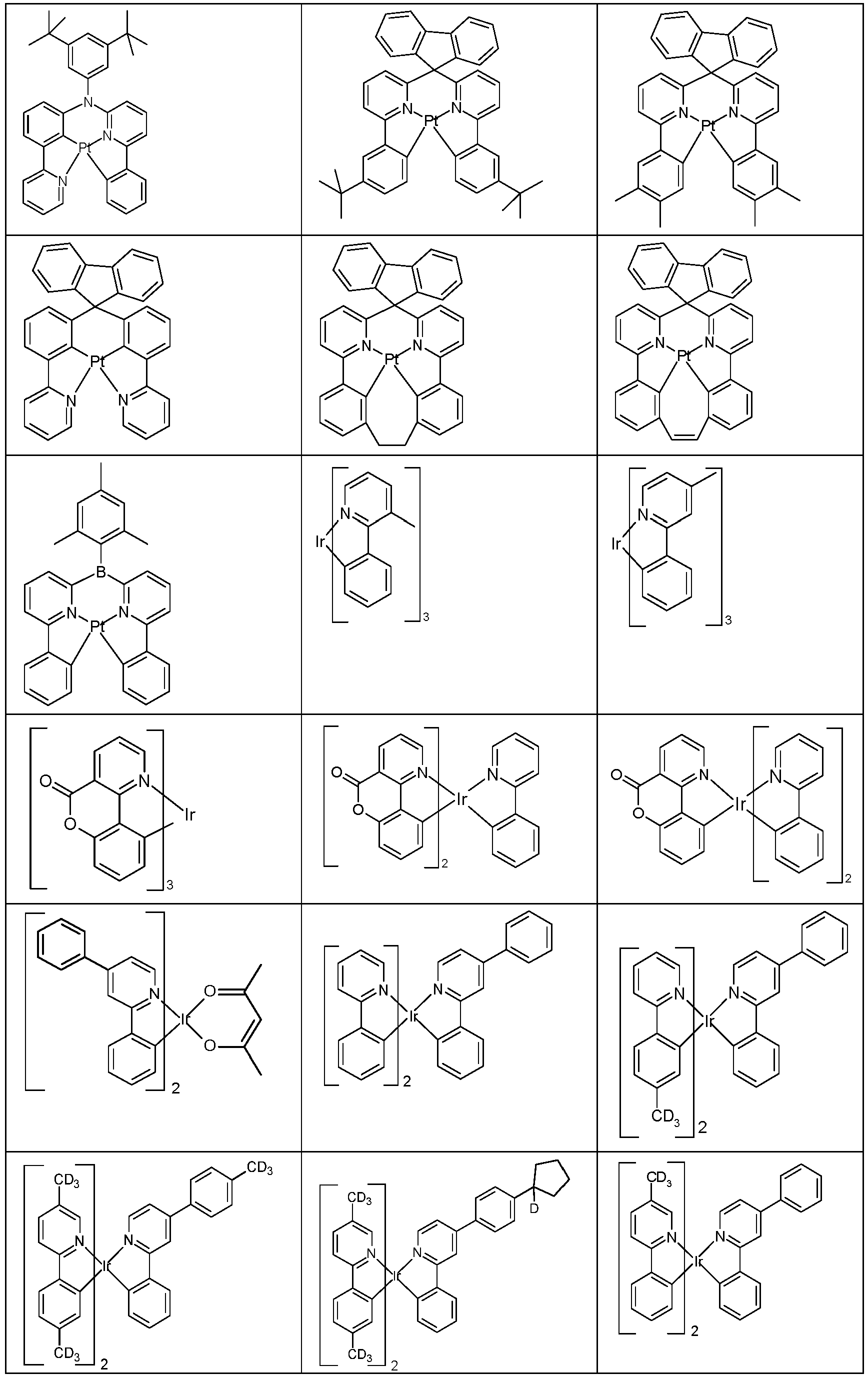

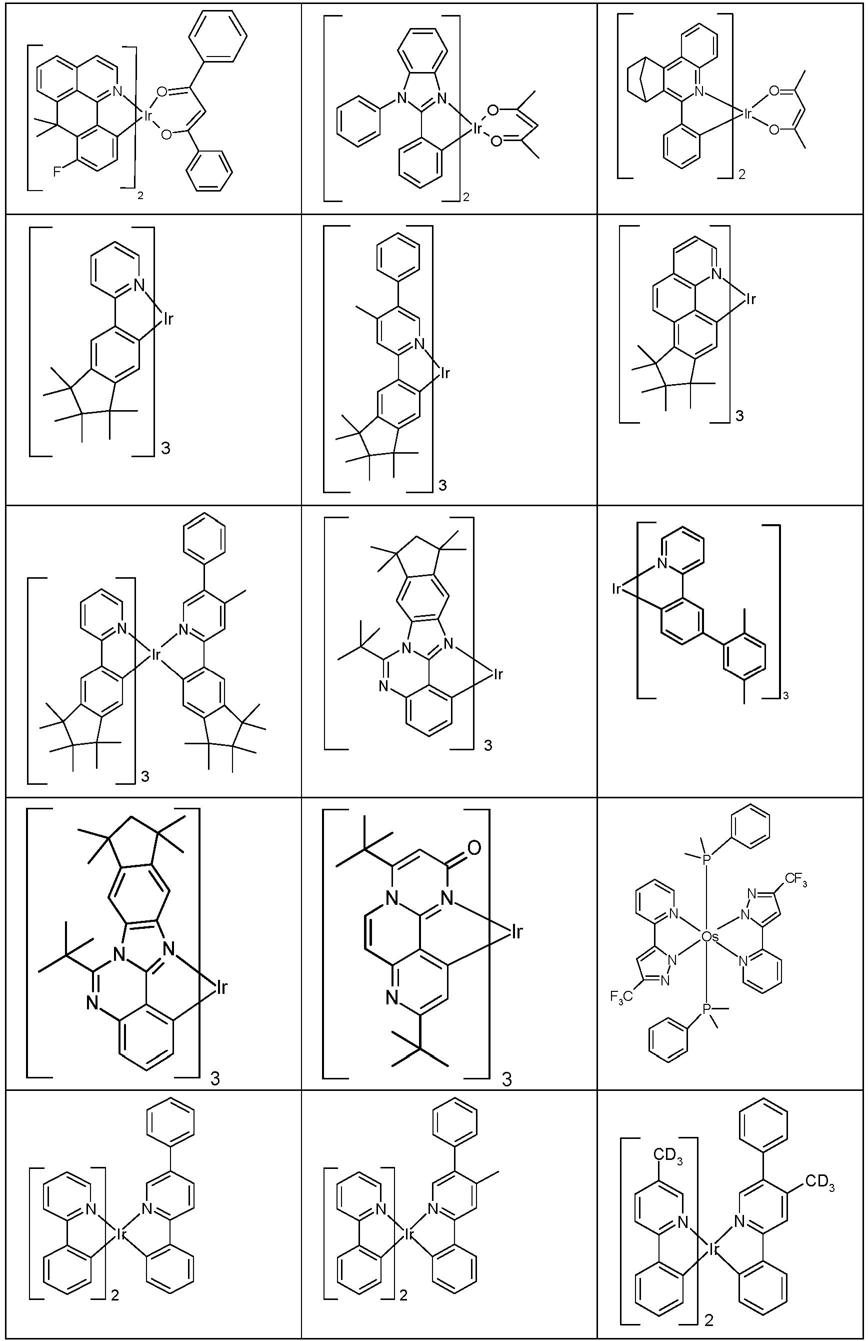

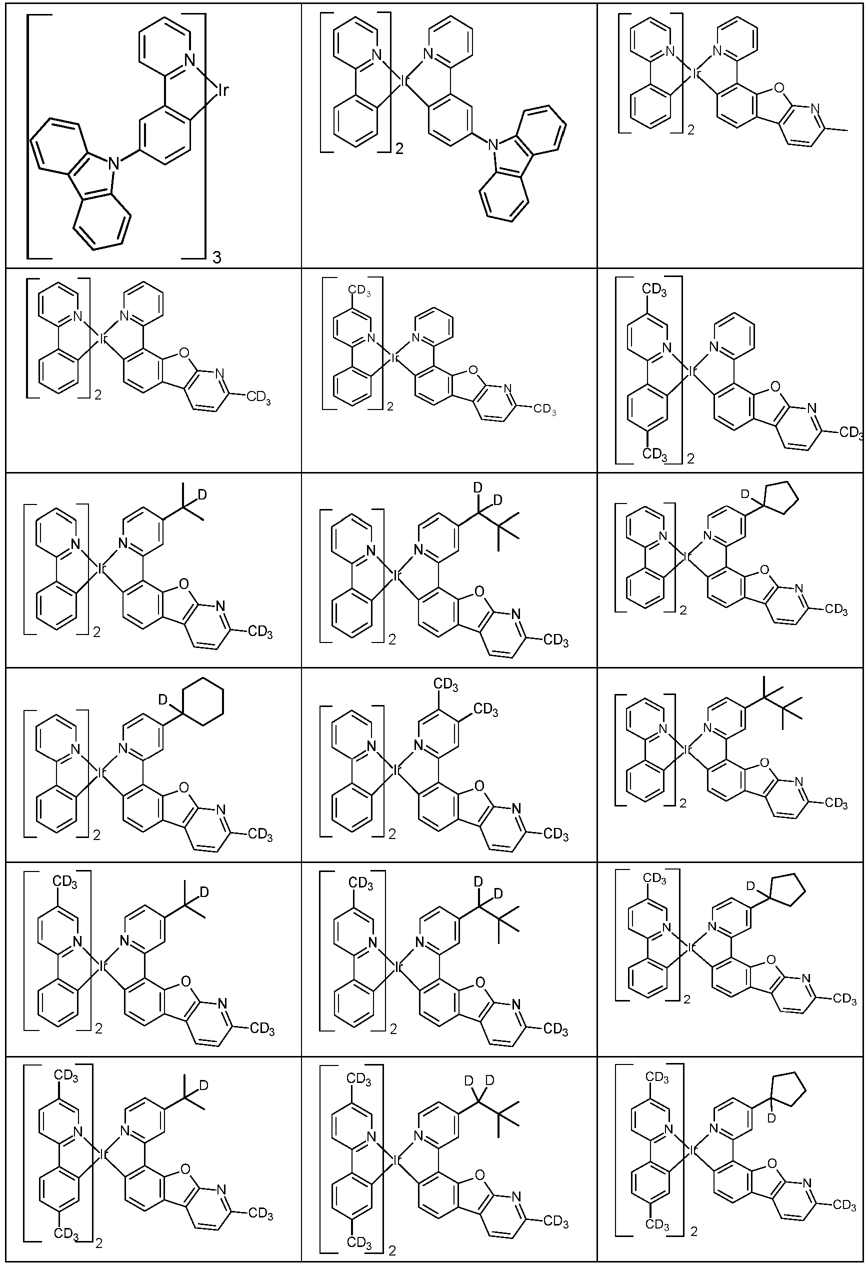

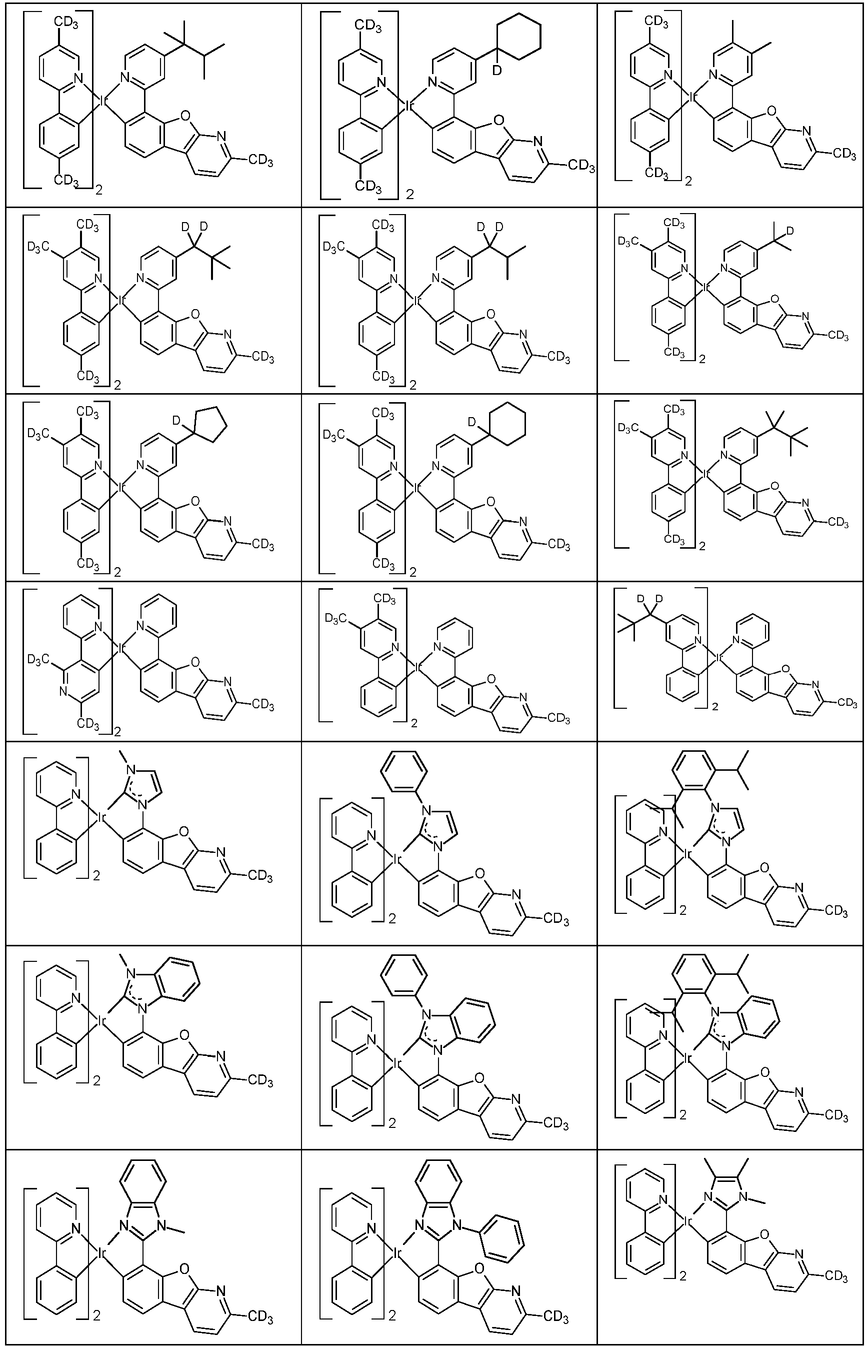

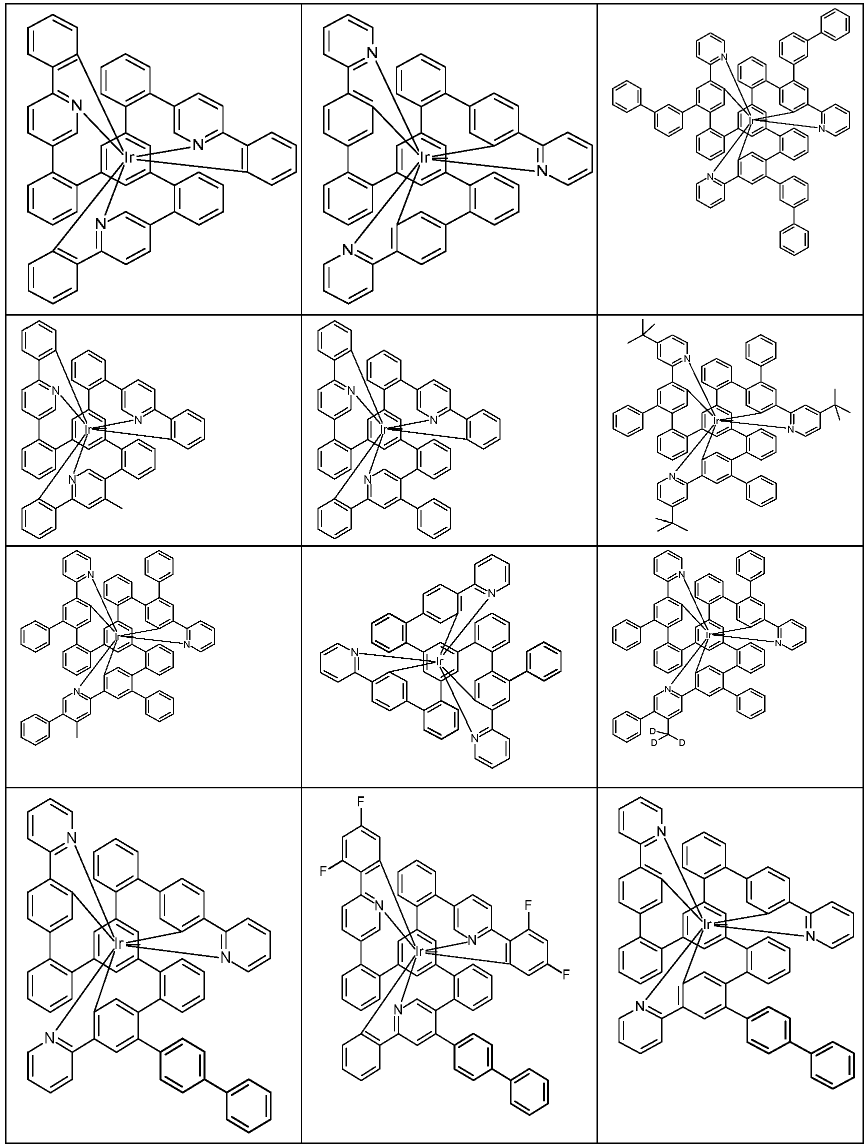

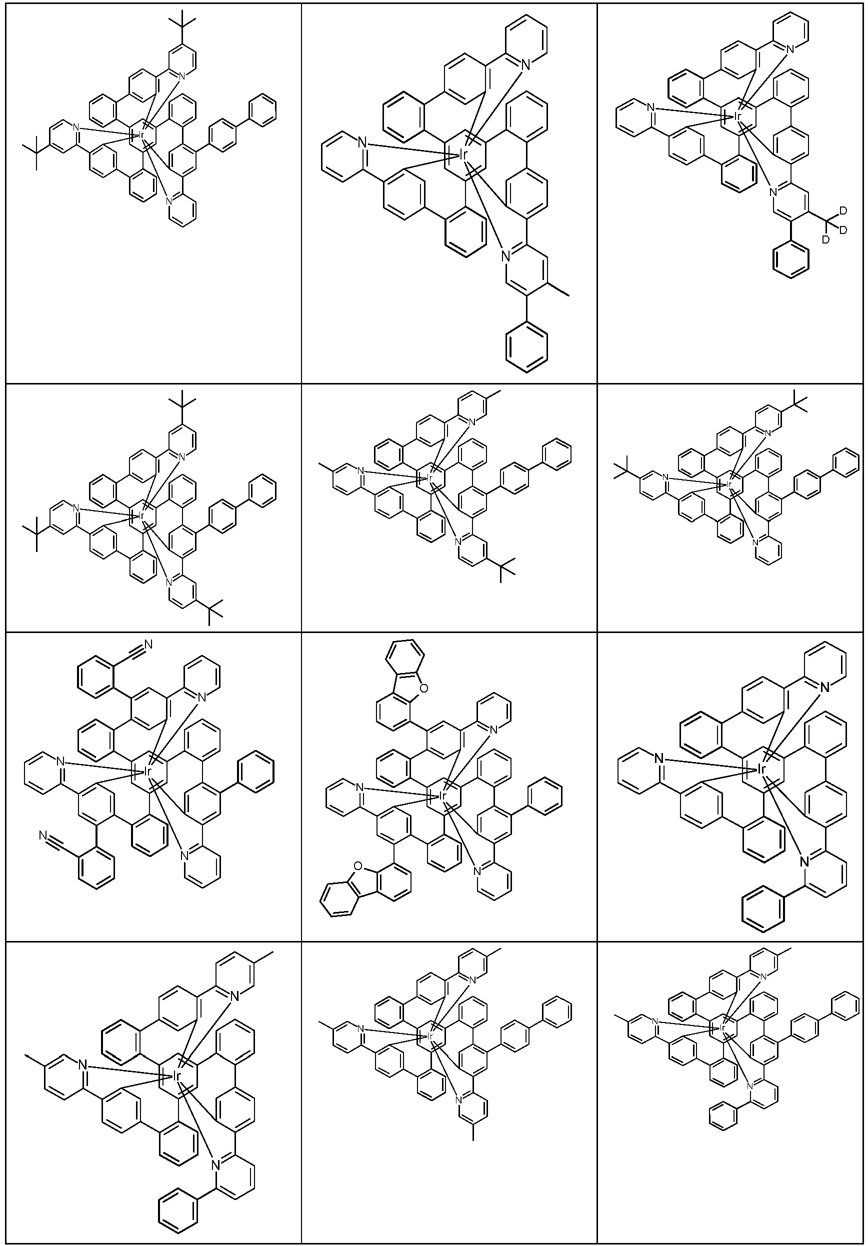

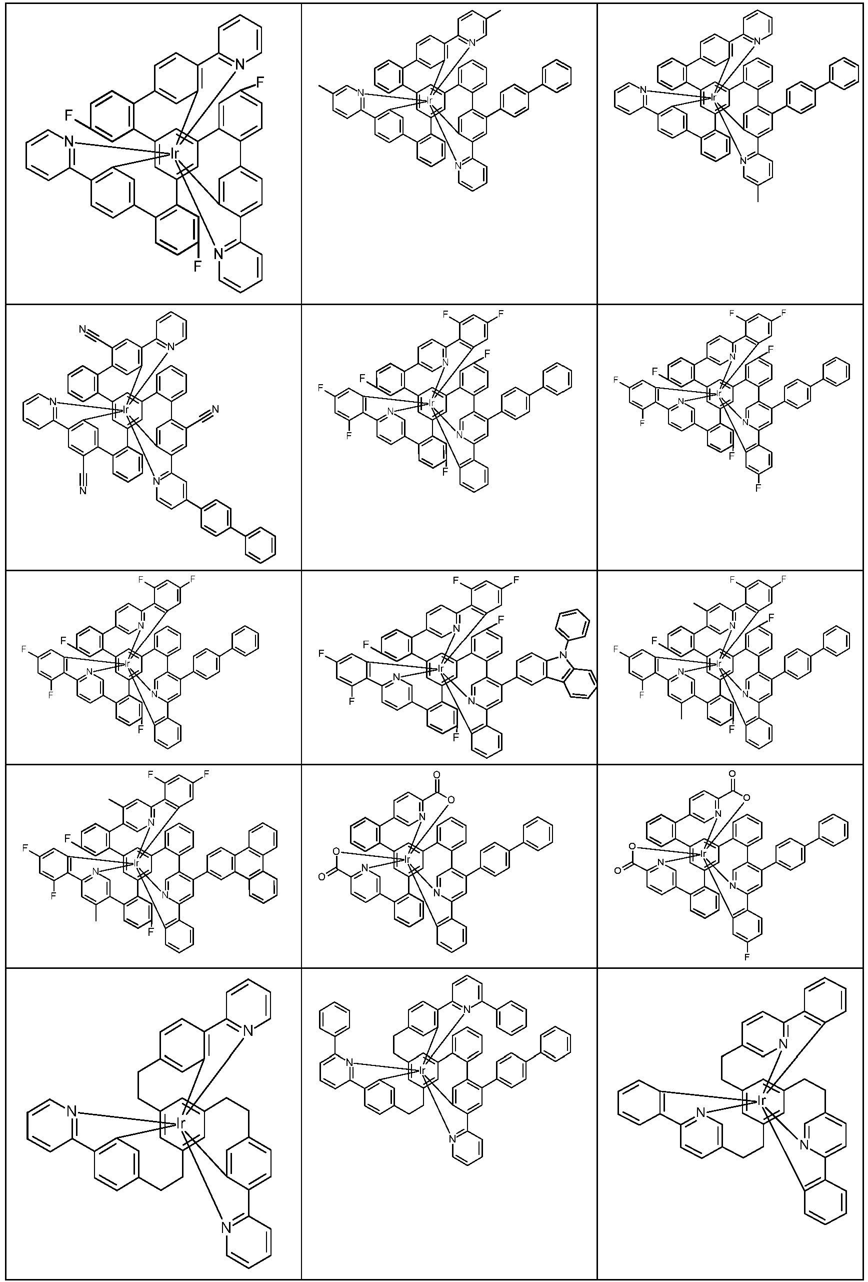

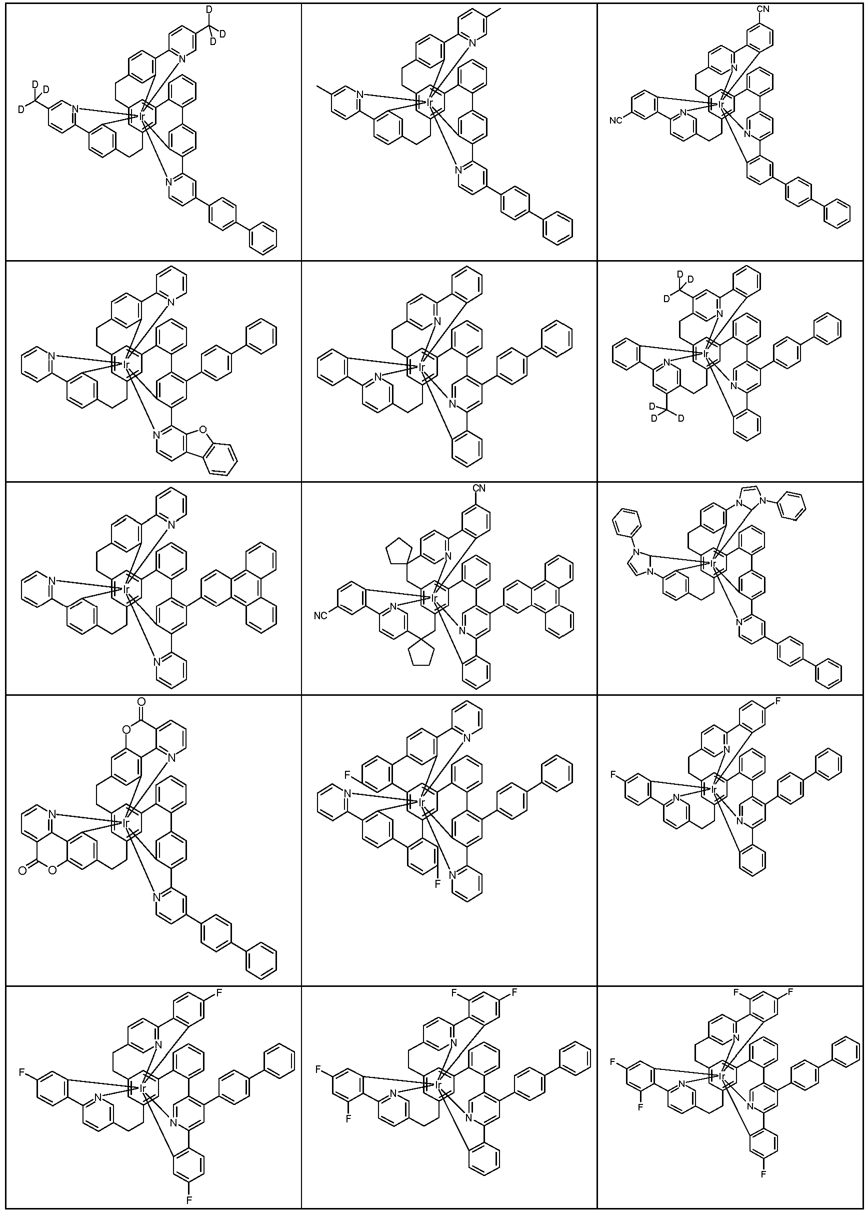

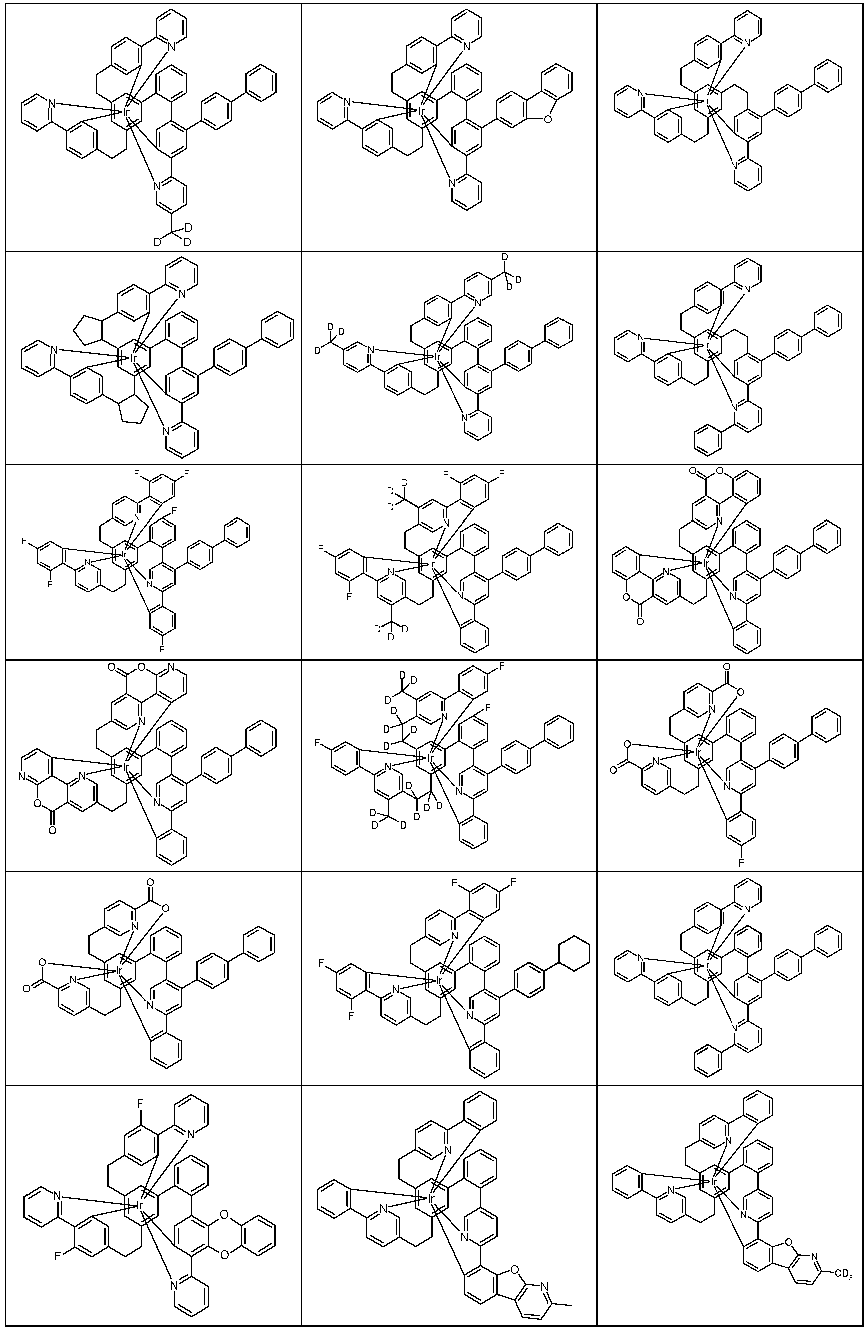

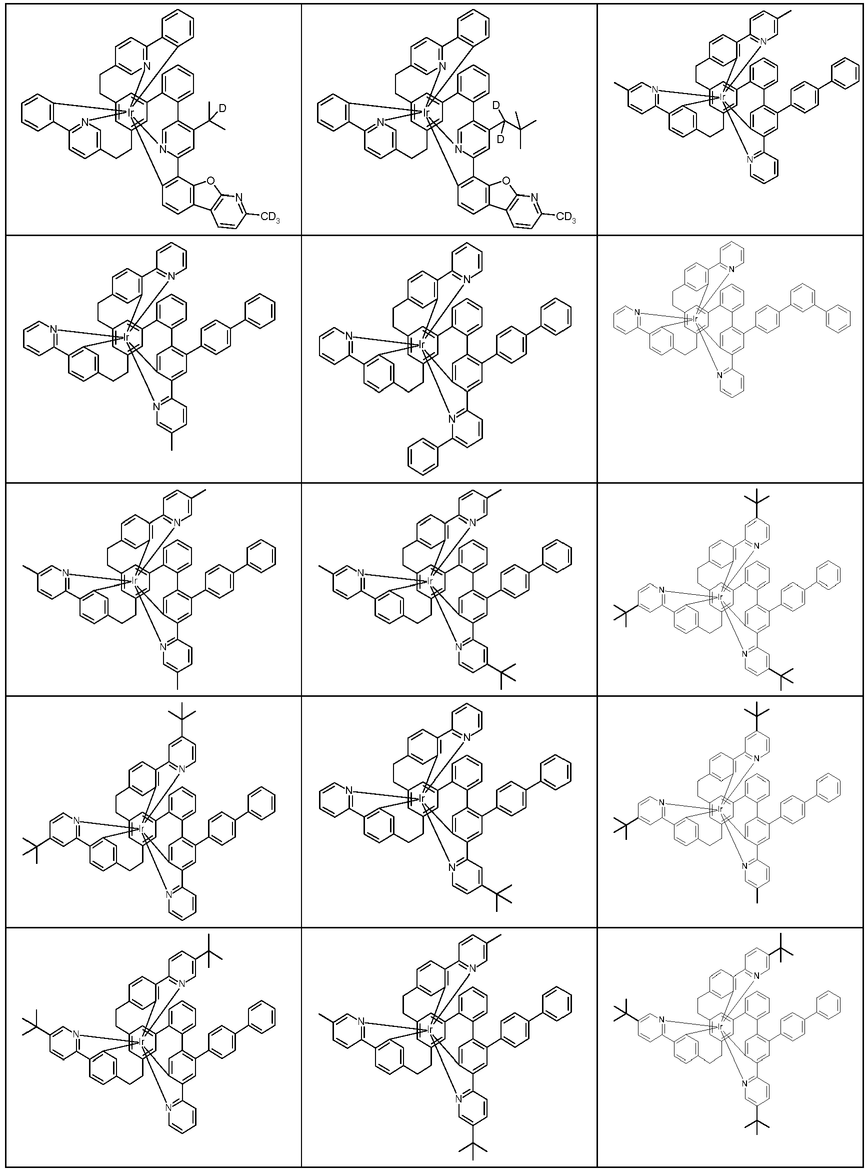

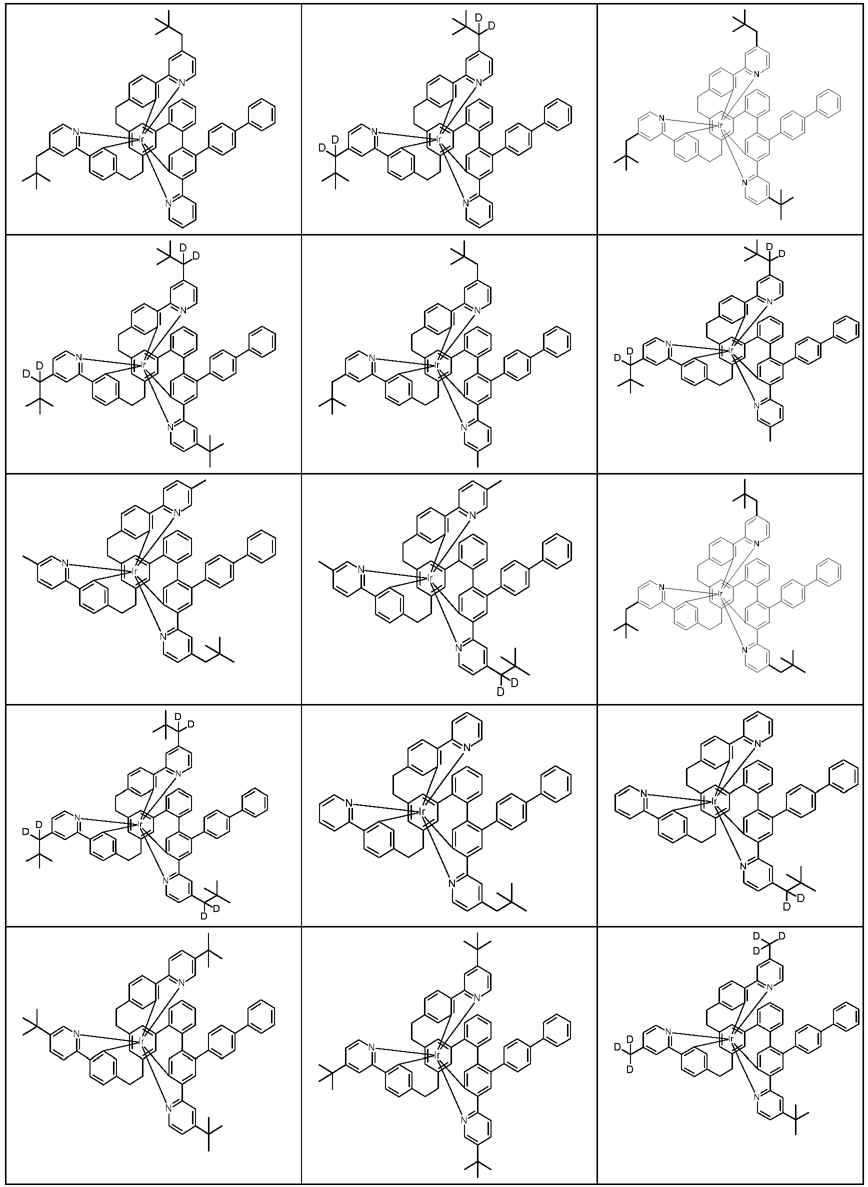

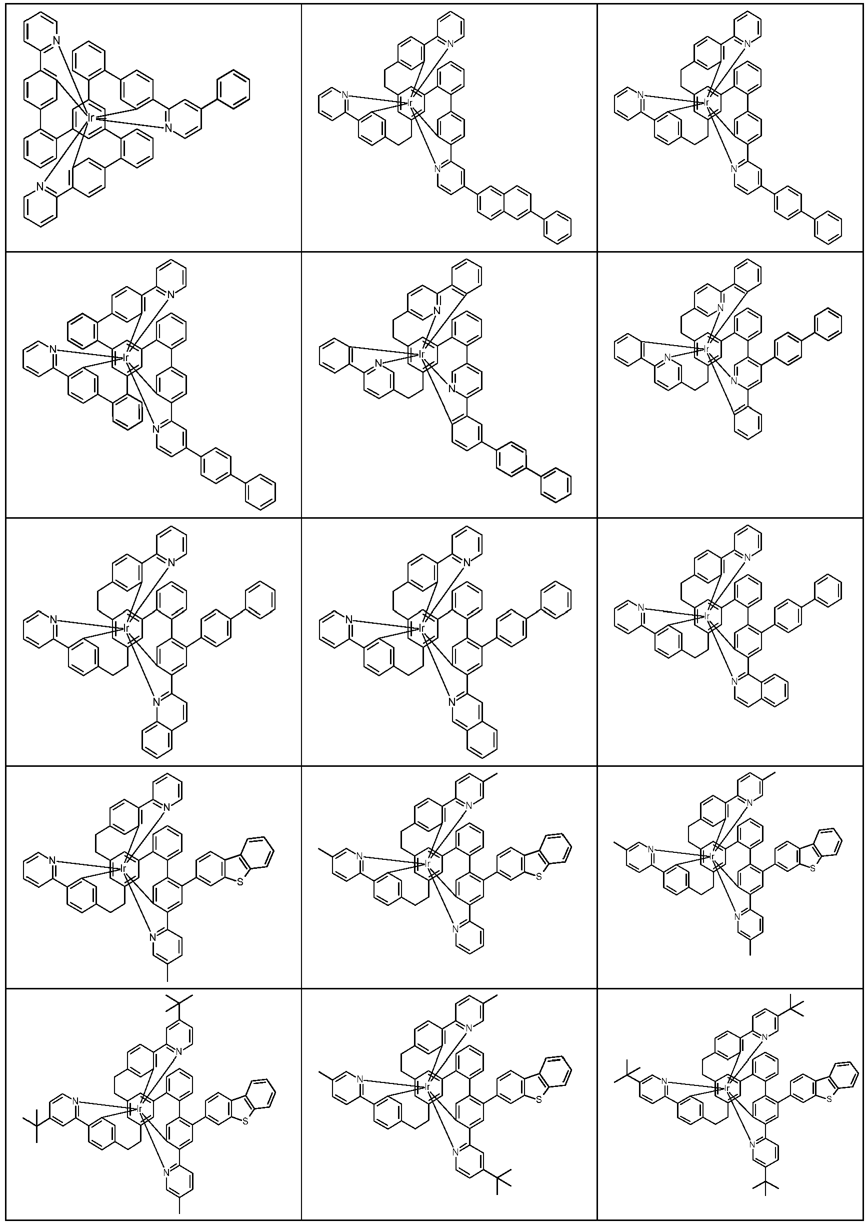

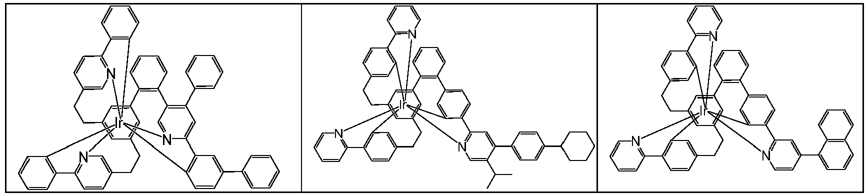

In den weiteren Schichten der erfindungsgemäßen organischen Elektrolumineszenzvorrichtung können alle Materialien verwendet werden, wie sie üblicherweise gemäß dem Stand der Technik eingesetzt werden.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind bekannt und können auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich durch einen oder mehrere der folgenden Eigenschaften aus:
1. Die erfindungsgemäßen Verbindungen, eingesetzt als Matrixmaterial für phosphoreszierende Emitter, führen zu langen Lebensdauern.
2. Die erfindungsgemäßen Verbindungen führen zu hohen Effizienzen, insbesondere zu einer hohen EQE. Dies gilt insbesondere, wenn die Verbindungen als Matrixmaterial für einen phosphoreszierenden Emitter eingesetzt werden.
3. Die erfindungsgemäßen Verbindungen führen zu geringen Betriebsspannungen. Dies gilt insbesondere, wenn die Verbindungen als Matrixmaterial für einen phosphoreszierenden Emitter eingesetzt werden.

Die Erfindung wird durch die nachfolgenden Beispiele näher erläutert.

### Beispiele

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Zu den literaturbekannten Verbindungen sind jeweils auch die entsprechenden CAS-Nummern angegeben.

### S1a:

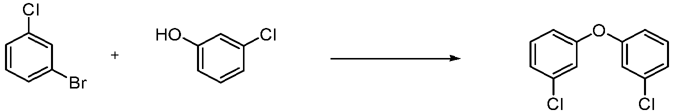

Unter inerter Atmosphäre werden DMSO (50 mL), K₃PO₄ (53.08 g 250 mmol), Pyridin-2-carbonsäure (1.53 g, 12.44 mmol) und Cul (1.19 g, 6.22 mmol) vorgelegt. Anschließend werden 3-Chlorphenol (19.20 g, 150 mmol) [108-43-0] und 3-Brom-1-chlorbenzol (23.93 g, 125 mmol) [108-37-2] nacheinander langsam zugegeben und das Reaktionsgemisch bei 85 °C für 16 h gerührt. Nach dem Abkühlen wird das Reaktionsgemisch extraktiv mit wässriger Ammoniaklösung und Methyl-tert-butylether aufgearbeitet. Die organische Phase wird fünfmal mit Wasser und zweimal mit gesättigter NaCl-Lösung gewaschen, die vereinigten Phasen über Na₂SO₄ getrocknet und das Lösungsmittel am Rotationsverdampfer abgezogen. Das Rohprodukt wird über fraktionierte Destillation weiter aufgereinigt. Ausbeute: 26.88 g (106 mmol), 85%; Reinheit 96%ig nach ¹H-NMR.

Analog können folgende Verbindungen dargestellt werden. Zur Aufreinigung kann neben Destillation auch Säulenchromatographie oder zur Umkristallisation können andere gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4-Dioxan, Dimethylsulfoxid, N,N-Dimethylformamid, N,N-Dimethylacetamid, N-Methylpyrrolidon, etc. verwendet werden.

| Edukt 1 | Edukt 2 | Produkt | Ausbeute |
|---|---|---|---|
| 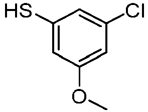 779198-52-6 | 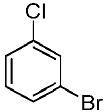 108-37-2 | 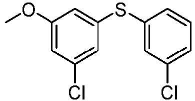 S2a | 80% |
| 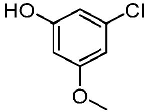 | 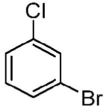 108-37-2 | 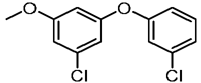 S3a | 86% |
| 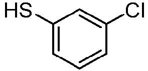 | 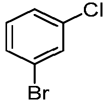 108-37-2 | 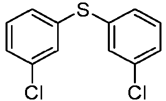 S4a | 74% |
| 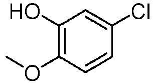 3743-23-5 | 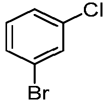 108-37-2 | 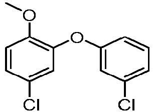 S5a | 75% |
| 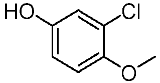 210346-76-2 | 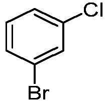 108-37-2 | 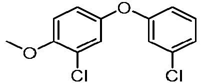 S6a | 68% |

### S1b:

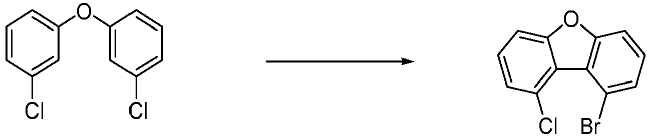

S1a (23.90 g 100 mmol) wird in THF (150 mL) unter inerter Atmosphäre vorgelegt und auf -75 °C abgekühlt. Anschließend wird langsam n-Butyllithium (2.5 mol/L in Hexan, 80 mL, 200 mol) so zugetropft, dass die Innentemperatur -65 °C nicht übersteigt. Es wird für 4 h bei -75 °C weitergerührt und dann wird Brom (5.6 mL, 109.3 mmol) so zugetropft, dass die Innentemperatur -65 °C nicht übersteigt. Nach beendeter Zugabe wird der Ansatz für 1 h bei -75 °C gerührt, dann innerhalb von 1 h langsam auf 10 °C aufgewärmt und 1 h bei 10 °C gerührt. Anschließend wird auf 0 °C gekühlt und der Ansatz mit gesättigter Na₂SO₃-Lösung (50 mL) vorsichtig gequencht. Der Ansatz wird extraktiv mit Toluol und Wasser aufgearbeitet, die vereinigten organischen Phasen dreimal mit Wasser und einmal mit gesättigter NaCl-Lösung gewaschen, über Na₂SO₄ getrocknet und das Lösungsmittel am Rotationsverdampfer entfernt. Das Rohprodukt wird zweimal mit 2-Propanol unter Rückfluss ausgerührt. Ausbeute: 24.21 g (86 mmol, 86%); Reinheit 97%ig nach ¹H-NMR.

Analog können folgende Verbindungen dargestellt werden. Zur Aufreinigung kann neben Ausrühren auch Destillation oder Säulenchromatographie oder zur Umkristallisation können andere gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4-Dioxan, Dimethylsulfoxid, N,N-Dimethylformamid, N,N-Dimethylacetamid, N-Methylpyrrolidon, etc. verwendet werden.

| Edukt 1 | Produkt | Ausbeute |
|---|---|---|
| 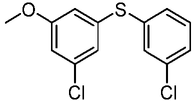 S2a | 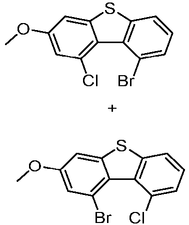 S2b | 70% |
| 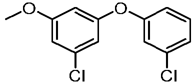 S3a | 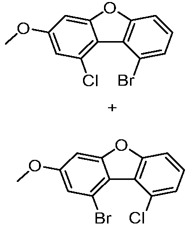 S3b | 79% |
| 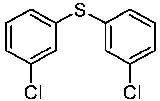 S4a | 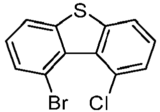 S4b | 79% |
| 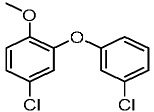 S5a | 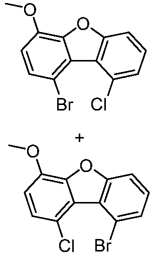 S5b | 74% |
| 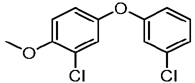 S6a | 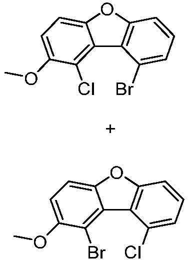 S6b | 53% |

### S1c:

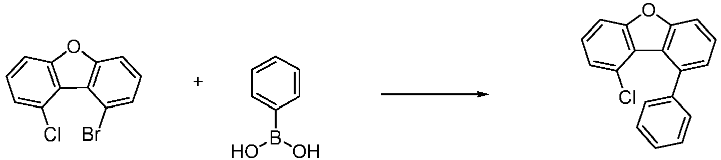

S1b (39.19 g, 140.0 mmol), Phenylboronsäure (18.29 g, 150.0 mmol) [5720-07-0] und K₂CO₃ (38.70 g, 280.0 mmol) werden in THF (100 mL) und Wasser (170 mL) vorgelegt und 30 min. inertisiert. Anschließend wird Tetrakis(triphenylphosphin)palladium [14221-01-3] (1.78 g, 1.54 mmol) zugegeben und das Reaktionsgemisch 20 h unter Rückfluss gerührt. Der Ansatz wird extraktiv mit Toluol und Wasser aufgearbeitet, die vereinigten organischen Phasen mit Wasser und gesättigter NaCl-Lösung gewaschen, über Na₂SO₄ getrocknet und das Lösungsmittel am Rotationsverdampfer abgezogen. Das Rohprodukt wird aus Ethanol umkristallisiert. Ausbeute: 30.1 g (108 mmol, 72%); Reinheit 97%ig nach ¹H-NMR.

Analog können folgende Verbindungen dargestellt werden. Zur Aufreinigung kann Säulenchromatographie oder zur Umkristallisation können andere gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4-Dioxan, Dimethylsulfoxid, N,N-Dimethylformamid, N,N-Dimethylacetamid, N-Methylpyrrolidon, etc. verwendet werden.

| Edukt 1 | Edukt 2 | Produkt | Ausbeute |
|---|---|---|---|
| 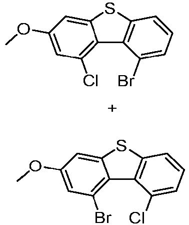 S2b | 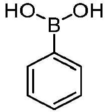 98-80-6 | 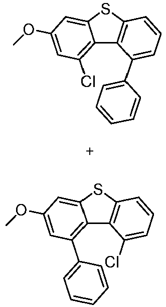 S2c | 64% |
| 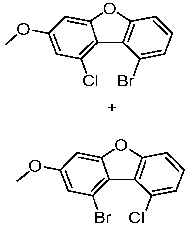 S3b | 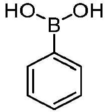 98-80-6 | 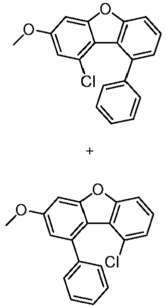 S3c | 69% |
| 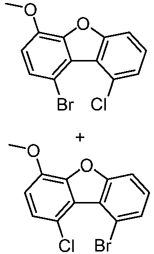 S5b | 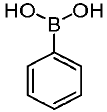 98-80-6 | 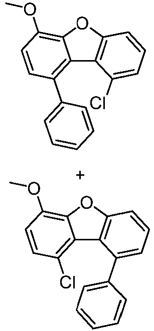 S4c | 59% |
| 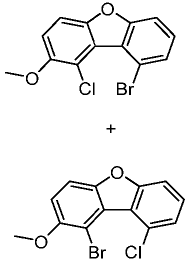 S6b | 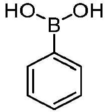 98-80-6 | 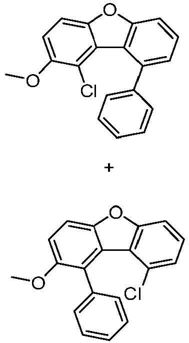 S5c | 51% |

### S1d:

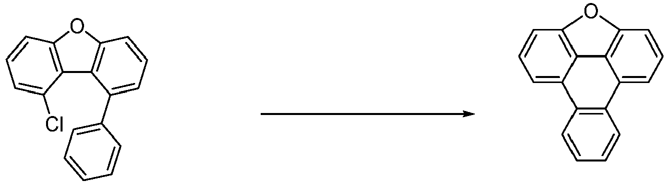

S1c (27.87 g, 100 mmol) und K₂CO₃ (41.46 g, 300 mmol) werden unter inerter Atmosphäre vorgelegt, mit DMAc (400 mL) versetzt und 30 min. inertisiert. Anschließend werden Pd(OAc)₂ (447 mg, 1.99 mmol) und 1,3-Bis-(2,6-diisopropyl-phenly)3-*H*-imidazol-1-ium chlorid (1.69 g, 3.98 mmol) zugegeben und das Reaktionsgemisch 18 h bei 140 °C gerührt. Nach dem Abkühlen wird der Ansatz in Ethanol/Wasser (1:1, 500 mL) gegossen und 30 min. nachgerührt. Der ausgefallene Feststoff wird abgesaugt und fünfmal mit Wasser und dreimal mit Ethanol gewaschen. Das Rohprodukt wird mit 2-Propanol unter Rückfluss ausgerührt und der Feststoff nach dem Abkühlen abgesaugt. Ausbeute: 22.9 g (84 mmol, 84%), Reinheit 98%ig nach ¹H-NMR.

Analog können folgende Verbindungen dargestellt werden. Dabei kann neben 1,3-Bis-(2,6-diisopropyl-phenly)3-*H*-imidazol-1-iumchlorid auch Tritert-Butylphosphin oder Tricyclohexylphosphin verwendet werden oder als Pd-Quelle neben Pd(OAc)₂ auch Pd₂(dba)₃. Zur Aufreinigung kann Säulenchromatographie oder zur Umkristallisation können andere gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4-Dioxan, Dimethylsulfoxid, N,N-Dimethylformamid, N,N-Dimethylacetamid, N-Methylpyrrolidon, etc. verwendet werden.

| Edukt 1 | Produkt | Ausbeute |
|---|---|---|
| 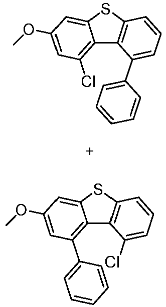 S2c | 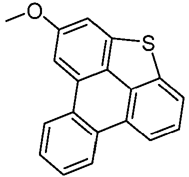 S2d | 75% |
| 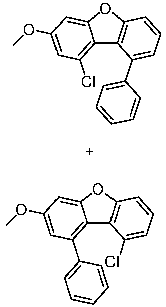 S3c | 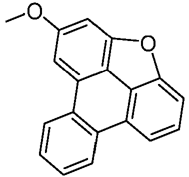 S3d | 73% |
| 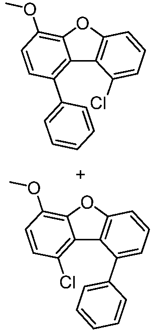 S4c | 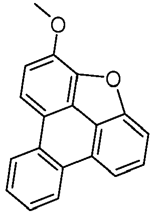 S4d | 80% |
| 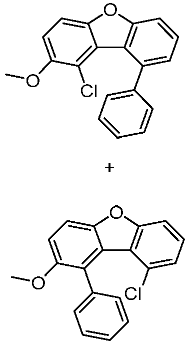 S5c | 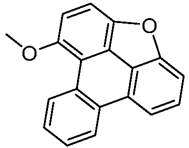 S5d | 63% |
| 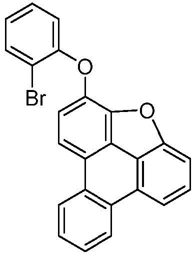 S1r | 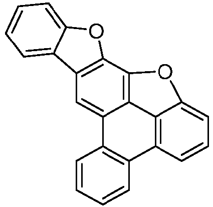 S6d | 75% |

### S1e:

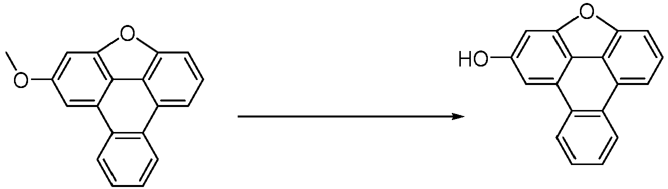

S3d (27.23 g, 100 mmol) wird in Dichlormethan (600 mL) vorgelegt und auf 0 °C im Eisbad abgekühlt. Anschließend wird vorsichtig BBr₃ (6.0 mL, 63.2 mmol) zugetropft. Nach beendeter Zugabe wird der Ansatz auf Raumtemperatur erwärmen gelassen. Nach vollständiger Umsetzung wird der Ansatz erneut auf 0 °C abgekühlt und vorsichtig mit MeOH (150 mL) gequencht. Das Lösungsmittel wird am Rotationsverdampfer abgezogen. Anschließend wird der Ansatz dreimal mit je 300 mL MeOH versetzt und dieses dann jeweils am Rotationsverdampfer abgezogen. Es wird erneut 200 mL MeOH zugegeben und der Feststoff abgesaugt. Das Rohprodukt wird getrocknet und ohne weitere Aufreinigung in der nächsten Stufe verwendet. Ausbeute: 15.8 g (61 mmol, 61%).

Analog können folgende Verbindungen dargestellt werden. Zur Aufreinigung kann Säulenchromatographie oder zur Umkristallisation können andere gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4-Dioxan, Dimethylsulfoxid, N,N-Dimethylformamid, N,N-Dimethylacetamid, N-Methylpyrrolidon, etc. verwendet werden.

| Edukt 1 | Produkt | Ausbeute |
|---|---|---|
| 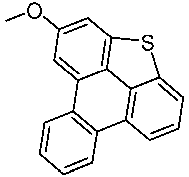 S2d | 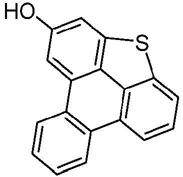 S2e | 64% |
| 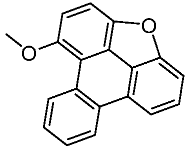 S5d | 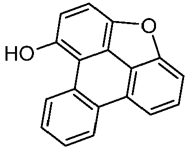 S3e | 71% |
| 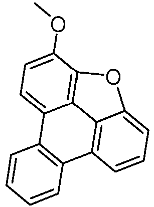 S4d | 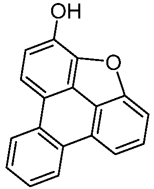 S4e | 63% |

### S1f:

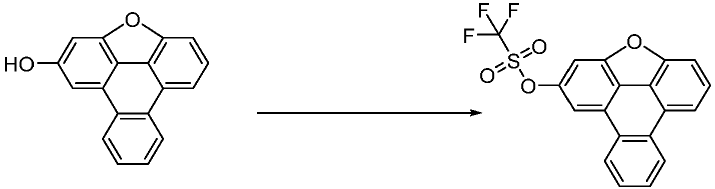

S1e (12.91 g, 50.0 mmol) und Triethylamin (20.8 mL, 150 mmol) werden in Dichlormethan (700 mL) vorgelegt und im Eisbad auf 0 °C gekühlt. Anschließend wird Trifluormethansulfonsäureanhydrid (10.9 mL, 65.0 mmol) langsam zugetropft. Nach beendeter Zugabe wird der Ansatz auf Raumtemperatur erwärmen gelassen. Nach vollständiger Umsetzung wird der Ansatz extraktiv mit Dichlormethan und Wasser aufgearbeitet, die vereinigten organischen Phasen über Na₂SO₄ getrocknet und das Lösungsmittel am Rotationsverdampfer entfernt. Der Rückstand wird in 300 mL Cyclohexan aufgenommen und 30 min. bei Raumtemperatur gerührt. Der Feststoff wird abgesaugt und im VTS getrocknet. Ausbeute 11.8 g (30.1 mmol, 60%).

Analog können folgende Verbindungen dargestellt werden. Zur Aufreinigung kann Säulenchromatographie oder zur Umkristallisation können andere gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4-Dioxan, Dimethylsulfoxid, N,N-Dimethylformamid, N,N-Dimethylacetamid, N-Methylpyrrolidon, etc. verwendet werden.

| Edukt 1 | Produkt | Ausbeute |
|---|---|---|
| 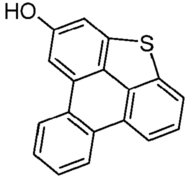 S2e | 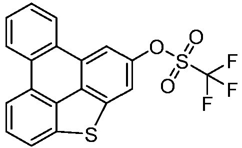 S2f | 73% |
| 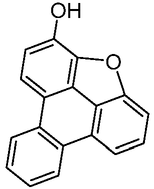 S4e | 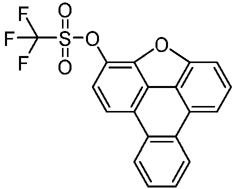 S4f | 64% |

### S1g:

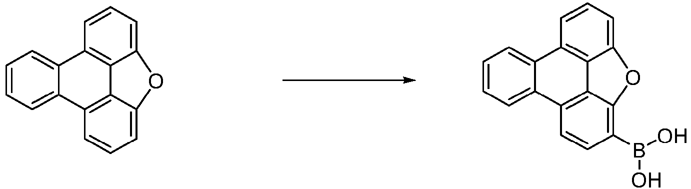

S4d (24.23 g, 100 mmol) wird in 300 mL THF vorgelegt und auf -75 °C gekühlt. Anschließend wird Hexyllithium (44.0 mL, c=2.5 mol/L, 110 mmol) so zugetropft, dass die Temperatur nicht über -65 °C steigt. Nach beendeter Zugabe wird 1 h bei -75 °C nachgerührt. Anschließend wird das Reaktionsgemisch langsam auf Raumtemperatur aufgewärmt, 1 h bei Raumtemperatur gerührt, wieder auf -75 °C abgekühlt und Trimethylborat (15.59 g, 150.0 mmol) so zugetropft, dass die Temperatur nicht über -65 °C steigt. Der Ansatz wird über Nacht auf Raumtemperatur erwärmt und am nächsten Tag vorsichtig mit HCl (c=5 mol/L, 50 mL) gequencht. Der Ansatz wird extraktiv mit Wasser aufgearbeitet und die organische Phase dreimal mit Wasser gewaschen. Das THF wird bis auf 50 mL abrotiert, dann werden 150 mL n-Heptan zugegeben und der ausgefallene Feststoff abgesaugt und mit n-Heptan gewaschen. Ausbeute: 24.03 g (84.2 mmol, 84%), Reinheit 96%ig nach ¹H-NMR.

### S1h:

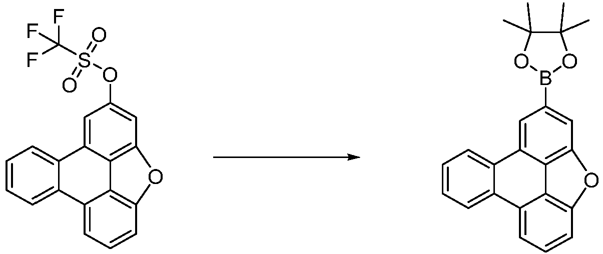

S1f (12.88 g, 33.0 mmol), Bis(pinacolato)diboron (10.34 g, 39.9 mmol) und KOAc (9.79 g, 99.75 mmol) werden in 1,4-Dioxan (250 mL) vorgelegt und 30 min. mit Argon inertisiert. Anschließend wird Pd(dppf)Cl₂ (740 mg, 0.91 mmol) zugegeben und der Ansatz 20 h unter Rückfluss gerührt. Nach dem Abkühlen wird das Lösungsmittel am Rotationsverdampfer abgezogen und der Rückstand extraktiv mit Dichlormethan und Wasser aufgearbeitet. Die vereinigten organischen Phasen werden über Na₂SO₄ getrocknet, mit Ethanol (180 ml) versetzt und das Dichlormethan am Rotationsverdampfer abgezogen. Der ausgefallene Feststoff wird abgesaugt und im Vakuumtrockenschrank getrocknet. Das Rohprodukt wird ohne weitere Aufreinigung in der nächsten Stufe eingesetzt. Ausbeute: 9.11 g (24.75 mmol, 75%), Reinheit 95%ig nach ¹H-NMR.

Analog können folgende Verbindungen dargestellt werden. Als Katalysatorsystem kann alternativ auch Pd(PCy₃)₂Cl₂ oder Pd₂(dba)₃ mit S-Phos (1:3) eingesetzt werden. Zur Aufreinigung kann neben Säulenchromatographie auch Heißextraktion verwendet werden, zur Umkristallisation oder Heißextraktion können andere gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4-Dioxan oder zur Umkristallisation Hochsieder wie Dimethylsulfoxid, N,N-Dimethylformamid, N,N-Dimethylacetamid, N-Methylpyrrolidon, etc. verwendet werden.

| Edukt 1 | Produkt | Ausbeute |
|---|---|---|
| 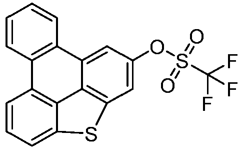 S2f | 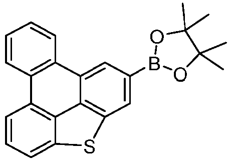 S2h | 85% |
| 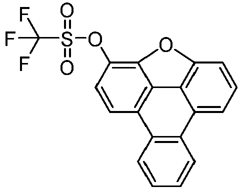 S4f | 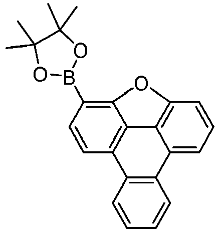 S3h | 70% |
| 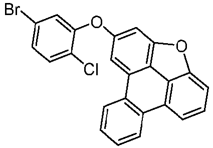 S2r | 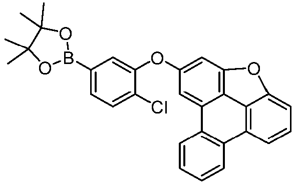 S4h | 68% |
| 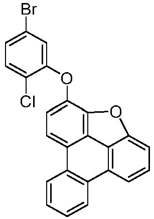 S3r | 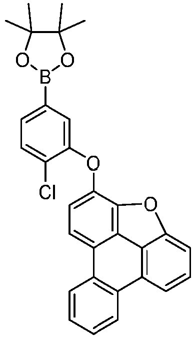 S5h | 73% |
| 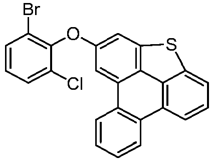 S4r | 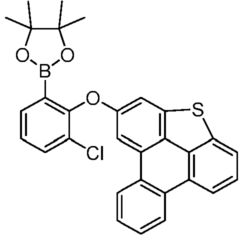 S6h | 42% |
| 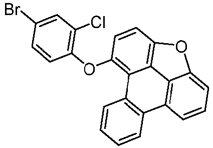 S5r | 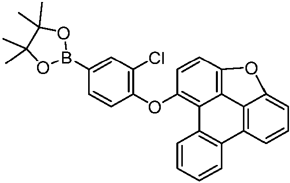 S7h | 80% |
| 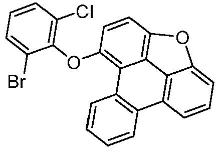 S6r | 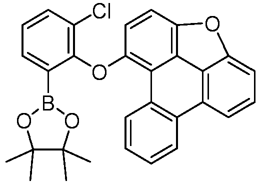 S8h | 36% |

### S1i:

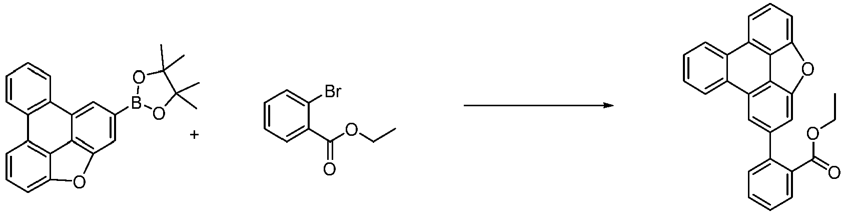

S1h (36.82 g, 100 mmol), Ethyl-2-brombenzoat (22.91 g, 100 mmol) [CAS-4688-76-0] und Natriumcarbonat (21.2 g, 200 mmoL) werden unter inerter Atmosphäre in Toluol (650 mL) und Wasser (150 mL) vorgelegt. Anschließend wird Tetrakis(triphenylphosphin)-palladium(0) (2.32 g, 2.00 mmol) zugegeben und das Reaktionsgemisch für 24 h unter Rückfluss gerührt. Nach dem Abkühlen wird das Reaktionsgemisch über eine Fritte gepackt, mit Toluol und Celite abgesaugt und anschließend extraktiv mit Toluol und Wasser aufgearbeitet. Die organische Phase wird mit Wasser (200 mL) und gesättigter NaCl-Lösung (100 mL) gewaschen, über Na₂SO₄ getrocknet und das Lösungsmittel am Rotationsverdampfer abgezogen. Das Rohprodukt wird über Säulenchromatographie weiter aufgereinigt. Ausbeute: 25.0 g (64 mmol, 64%), Reinheit 97%ig nach ¹H-NMR.

### S1l:

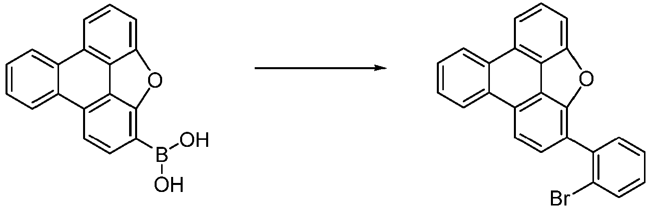

S1g (12.02 g, 42.0 mmol), 1-Brom-2-iod-benzol (9.27 g, 42.8 mmol), K₂CO₃, (11.61 g, 84.0 mmol) werden in Toluol:Ethanol:Wasser (200 mL:50 mL:100 mL) vorgelegt und 30 min. mit Argon inertisiert. Anschließend wird Pd(dppf)Cl₂ (118 mg, 168 µmol) zugegeben und der Ansatz 16 h unter Rückfluss gerührt. Der Ansatz wird extraktiv mit Toluol/Wasser aufgearbeitet. Die vereinigten organischen Phasen werden über Na₂SO₄ getrocknet und das Lösungsmittel am Rotationsverdampfer abgezogen. Das Rohprodukt wird aus einer Mischung aus 2-Propanol und Toluol umkristallisiert. Ausbeute: 15.18 g (38.2 mmol, 91 %), Reinheit 98%ig nach ¹H-NMR.

### S1m:

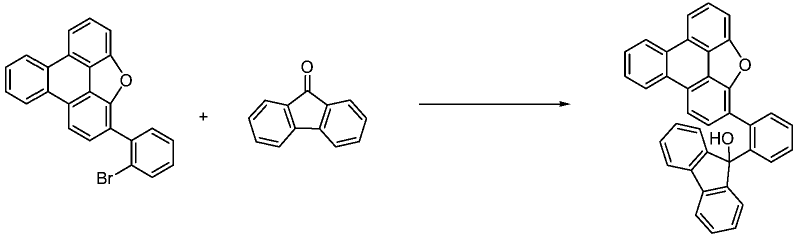

S1l (13.98 g, 35.2 mmol) wird in 250 mL THF vorgelegt und auf -75 °C gekühlt. Anschließend wird n-BuLi (16.0 mL, c=2.5 mol/L, 40.0 mmol) so zugetropft, dass die Temperatur nicht über -65 °C steigt. Nach beendeter Zugabe wird 1 h bei -75 °C nachgerührt. Eine Lösung aus Fluoren-9-on (6.48 g, 36.0 mmol) in THF (50 mL) wird so zugetropft, dass die Temperatur nicht über -65 °C steigt. Der Ansatz wird über Nacht auf Raumtemperatur aufgewärmt, dann wird Wasser (100 mL) zugetropft. Die Phasen werden getrennt, die organische Phase über Na₂SO₄ getrocknet und das Lösungsmittel am Rotationsverdampfer entfernt. Das Rohprodukt wird ohne weitere Aufreinigung im nächsten Schritt umgesetzt. Ausbeute: quantitativ.

### S1n:

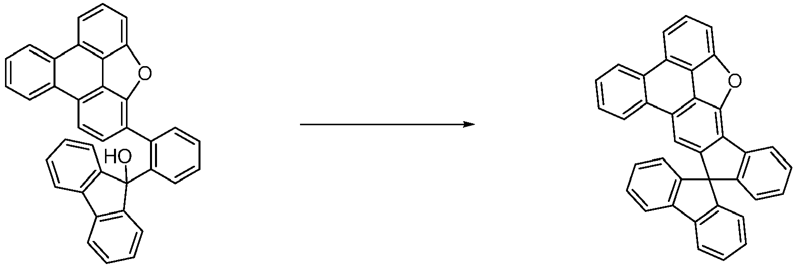

S1m (49.86 g, 100 mmol) wird in konz. HCl (37%ig, 41.4 mL, 500 mmol) und Eisessig (450 mL) vorgelegt und 20 h unter Rückfluss gerührt. Der ausgefallene Feststoff wird abgesaugt und fünfmal mit Wasser und fünfmal mit Ethanol gewaschen. Das Rohprodukt wird zweimal mit 2-Propanol unter Rückfluss ausgerührt. Ausbeute: 39.0 g (81.2 mmol, 81%), Reinheit 98%ig nach ¹H-NMR.

### S1o:

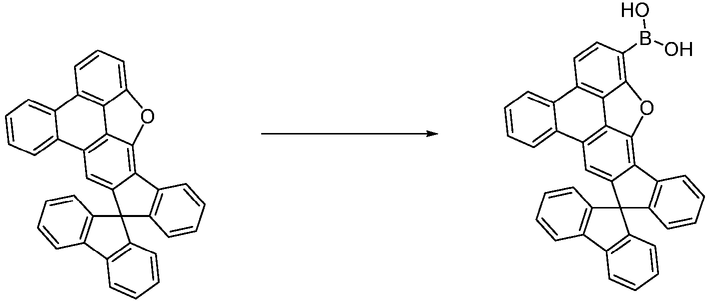

S1n (32.02 g, 66.7 mmol) wird in THF (500 mL) unter inerter Atmosphäre vorgelegt und auf -20 °C abgekühlt. Dann wird sec-BuLi (95.3 mL, 133.4 mmol, 1.4 mol/L in Cyclohexan) langsam zugetropft und der Ansatz 1 h nachgerührt. Anschließend wird Trimethylborat (9.30 g, 89.5 mmol) über 10 min zugetropft, der Ansatz auf Raumtemperatur erwärmen gelassen und 12 h bei Raumtemperatur gerührt. Es werden langsam 50 mL HCl zugetropft und der Ansatz wird extraktiv mit Wasser aufgearbeitet. Das Lösungsmittel wird am Rotationsverdampfer abgezogen und das Rohprodukt ohne weitere Aufreinigung in der nächsten Stufe eingesetzt. Ausbeute: 30.78 g (58.7 mmol, 88%), Reinheit 95%ig nach ¹H-NMR.

Analog können folgende Verbindungen dargestellt werden. Zur Aufreinigung kann neben Säulenchromatographie auch Heißextraktion verwendet werden. Zur Umkristallisation oder Heißextraktion können andere gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4-Dioxan oder zur Umkristallisation Hochsieder wie Dimethylsulfoxid, N,N-Dimethylformamid, N,N-Dimethylacetamid, N-Methylpyrrolidon, etc. verwendet werden.

| Edukt 1 | Produkt | Ausbeute |
|---|---|---|
| 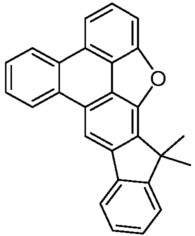 S1q | 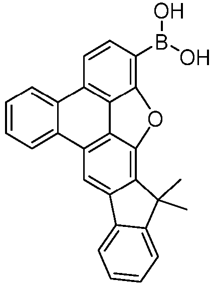 S2o | 41% |
| 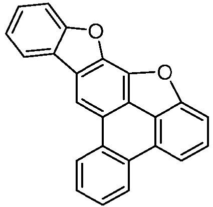 S6d | 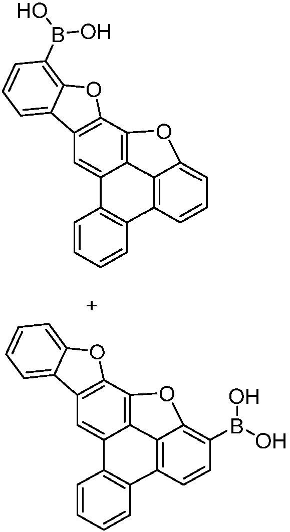 S3o + S4o | S3o 26% + S4o 28% |

### S1p:

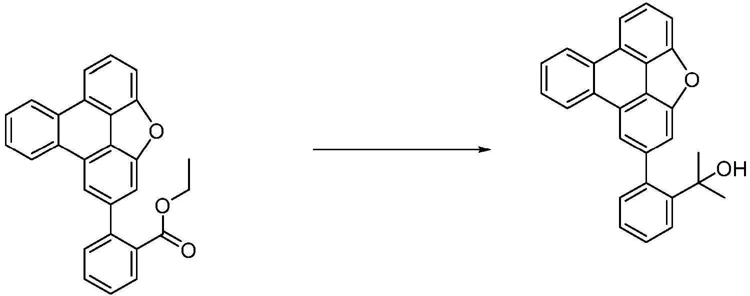

Trockenes CeCl₃ (50.53 g, 205 mmol) wird in THF (250 mL getrocknet) vorgelegt und 30 min. bei Raumtemperatur gerührt. Anschließend wird S6i (39.04 g, 100 mmol) in 500 mL THF gelöst und bei 0 °C zur CeCl₃-Suspension zugegeben. Anschließend wird das Gemisch 30 min. bei Raumtemperatur gerührt. Die Reaktionsmischung wird auf 0 °C gekühlt und MeMgCl (100 mL, 3 mol/L in THF, 300 mmol) langsam zugetropft. Nach beendeter Zugabe wird die Reaktionsmischung 2 h bei Raumtemperatur gerührt und anschließend vorsichtig mit NH₄Cl-Lösung (100 ml) gequencht. Das Reaktionsgemisch wird extraktiv mit Dichlormethan und Wasser aufgearbeitet, die vereinigten organischen Phasen mit Wasser und gesättigter NaCl-Lösung gewaschen und das Lösungsmittel am Rotationsverdampfer abgezogen. Das Rohprodukt wird ohne weitere Aufreinigung in der nächsten Stufe umgesetzt. Ausbeute: quantitativ, ca. 90 %ig nach 1H-NMR.

### S1q + S2q:

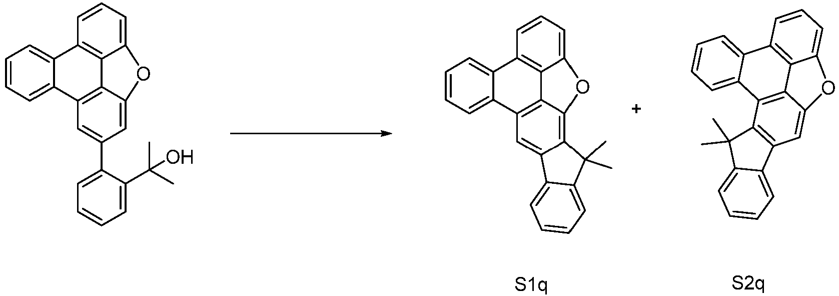

Methansulfonsäure (48.05 g, 500 mmol) und Polyphosphorsäure (75 g) werden in 250 mL vorgelegt und auf 0 °C gekühlt. Anschließend wird S1p (37.65 g, 100 mmol) in Dichlormethan (250 mL) gelöst und bei 0 °C zum Methansulfonsäure/Polyphosphorsäure Gemisch zugetropft. Das Reaktionsgemisch wird 1 h bei RT gerührt. Es werden 400 mL Wasser und 200 mL EtOH zugegeben und das Gemisch für 1 h bei Raumtemperatur gerührt. Die organische Phase wird abgetrennt, mit Wasser und gesättigter NaCl-Lösung gewaschen und über ein Kiesegelbett (vorgeschlämmt mit Dichlormethan) filtriert. Es wird n-Heptan (300 mL) zum Filtrat gegeben und das Dichlormethan am Rotationsverdampfer abrotiert. Der ausgefallene Feststoff wird abgesaugt und mit n-Heptan gewaschen. Das Isomerengemisch wird chromatographisch getrennt. Reinheit S1q und S2q ca. 95%ig nach 1H-NMR.

### S1r:

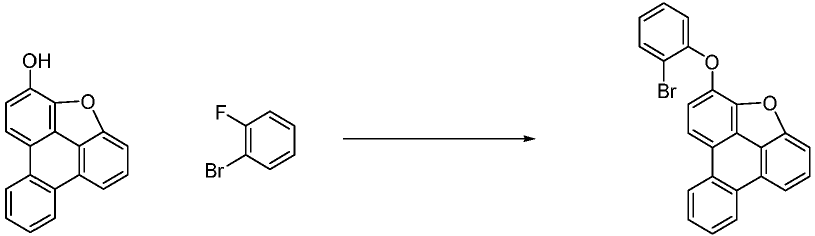

S4e (12.91 g, 50.0 mmol), 1-Brom-2-fluorbenzol (9.63 g, 55.0 mmol) und Kaliumcarbonat (17.27 g, 125.0 mmol) werden unter inerter Atmosphäre in DMAC (300 mL) vorgelegt und für 30 h bei 120 °C gerührt. Nach dem Abkühlen zieht man das DMAC am Rotationsverdampfer weitgehend ab und arbeitet das Rohprodukt extraktiv mit Dichlormethan und Wasser auf. Die vereinigten organischen Phasen werden mit Wasser (3x 150 mL) und gesättigter NaCl-Lösung (100 mL) gewaschen, über Na₂SO₄ getrocknet und anschließend das Lösungsmittel am Rotationsverdampfer abgezogen. Das Rohprodukt wird mit Ethanol am Rückfluss ausgekocht. Ausbeute: 14.1 g (34.2 mmol, 68%), 95%ig nach 1H-NMR.

Analog können folgende Verbindungen dargestellt werden: Zur Aufreinigung kann neben Säulenchromatographie auch Heißextraktion verwendet werden, zur Umkristallisation oder Heißextraktion können andere gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4-Dioxan oder zur Umkristallisation Hochsieder wie Dimethylsulfoxid, N,N-Dimethylformamid, N,N-Dimethylacetamid, N-Methylpyrrolidon, etc. verwendet werden.

| Edukt 1 | Edukt 2 | Produkt | Ausbeute |
|---|---|---|---|
| 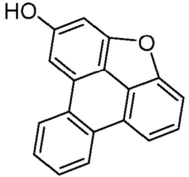 S1e | 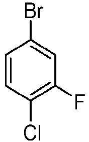 60811-18-9 | 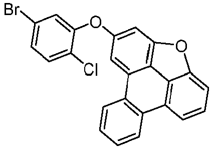 S2r | 58% |
| 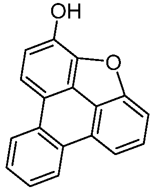 S4e | 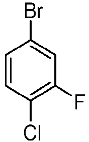 60811-18-9 | 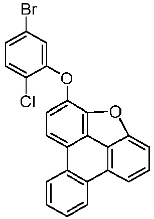 S3r | 60% |
| 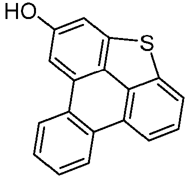 S2e | 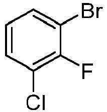 144584-65-6 | 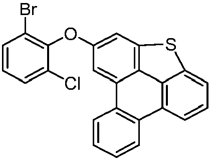 S4r | 41% |
| 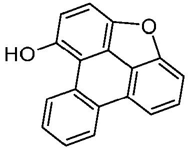 S3e | 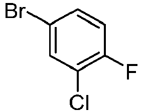 60811-21-4 | 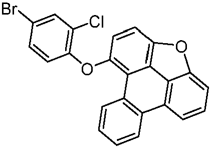 S5r | 68% |
| 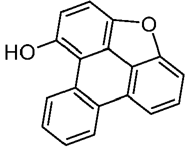 S3e | 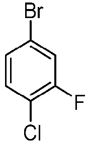 60811-18-9 | 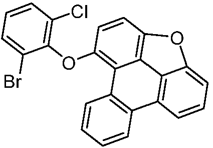 S6r | 37% |

### S1s:

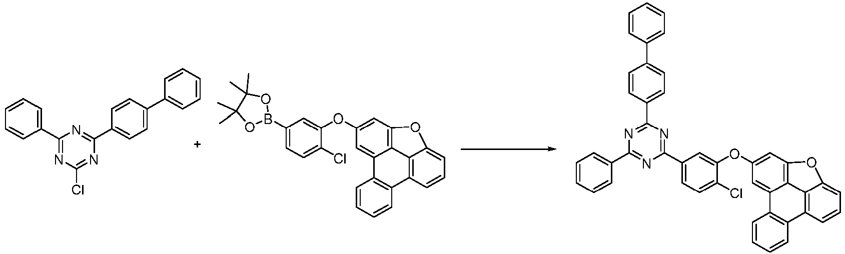

S5h (49.48 g, 100.0 mmol), 2-[1,1'-Biphenyl]-4-yl-4-chlor-6-phenyl-1,3,5-triazin [CAS-1472062-94-4] (37.82 g, 110.0 mmol) [5720-07-0] und K₂CO₃ (38.70 g, 280.0 mmol) werden in THF (700 mL) und Wasser (250 mL) vorgelegt und 30 min. inertisiert. Anschließend wird Tetrakis(triphenylphosphin)palladium [14221-01-3] (3.56 g, 3.08 mmol) zugegeben und das Reaktionsgemisch 20 h unter Rückfluss gerührt. Der Ansatz wird extraktiv mit Toluol und Wasser aufgearbeitet, die vereinigten organischen Phasen mit Wasser und gesättigter NaCl-Lösung gewaschen, über Na₂SO₄ getrocknet und das Lösungsmittel am Rotationsverdampfer abgezogen. Das Rohprodukt wird aus n-Butylacetat umkristallisiert. Ausbeute: 52.7 g (78 mmol, 78%), Reinheit 98%ig nach ¹H-NMR.

Analog können folgende Verbindungen dargestellt werden. Zur Aufreinigung kann Säulenchromatographie oder zur Umkristallisation können andere gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4-Dioxan, Dimethylsulfoxid, N,N-Dimethylformamid, N,N-Dimethylacetamid, N-Methylpyrrolidon, etc. verwendet werden.

| Edukt 1 | Edukt 2 | Produkt | Ausbeute |
|---|---|---|---|
| 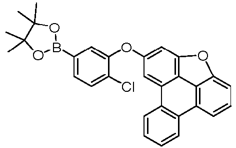 S4h | 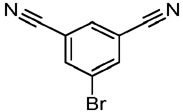 CAS-160892-07-9 | 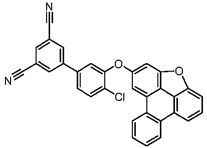 S2s | 79% |
| 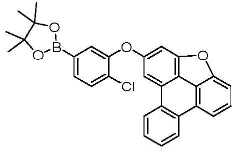 S4h | 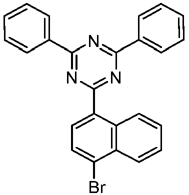 CAS-1800220-86-7 | 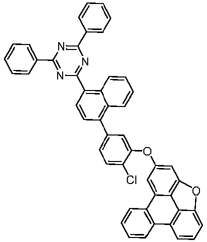 S3s | 61% |
| 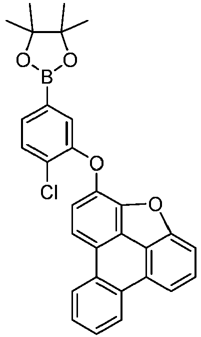 S5h | 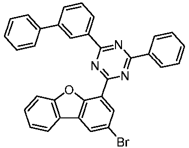 CAS-2375066-17-2 | 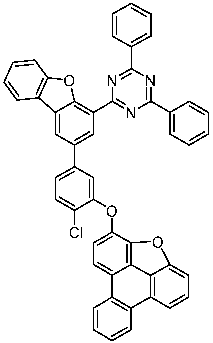 S4s | 60% |
| 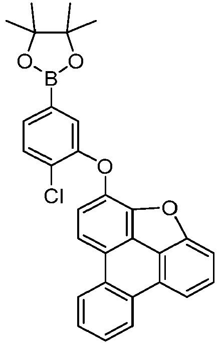 | 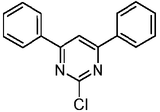 CAS-2915-16-4 | 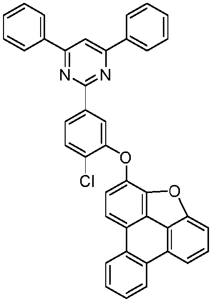 S5s | 84% |
| 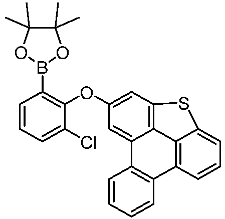 S6h | 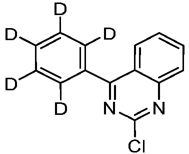 CAS-1614244-83-5 | 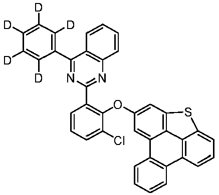 S6s | 65% |
| 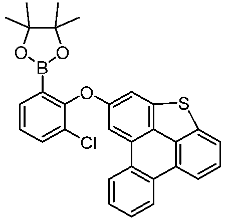 S6h | 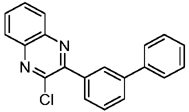 | 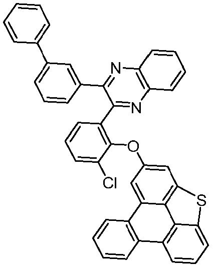 S7s | 28% |
| 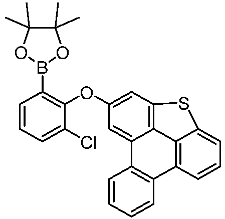 S6h | 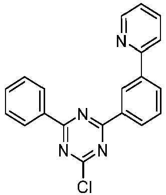 CAS-1943719-88-7 | 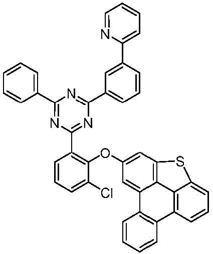 S8s | 48% |
| 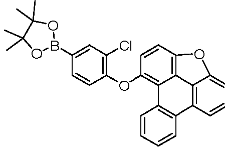 S7h | 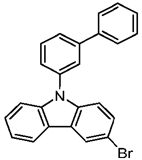 CAS-1428551-28-3 | 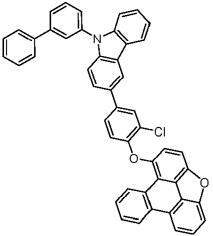 S9s | 65% |
| 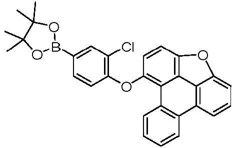 | 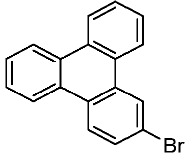 | 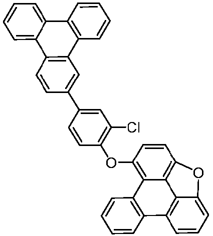 S10s | 75% |
| 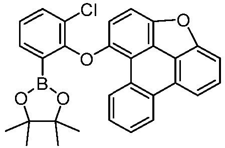 S8h | 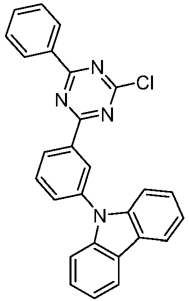 2260561 -80-4 | 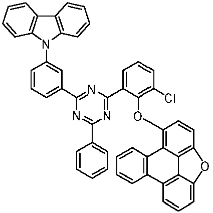 S11s | 45% |
| 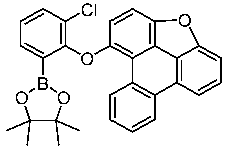 S8h | 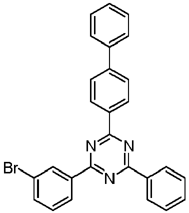 1955546-91-4 | 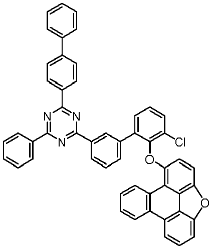 S12s | 56% |
| 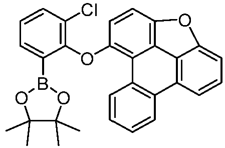 S8h | 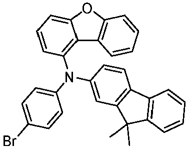 | 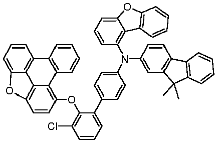 S13s | 40% |

### P1a + P2a nicht erfindungsgemäß

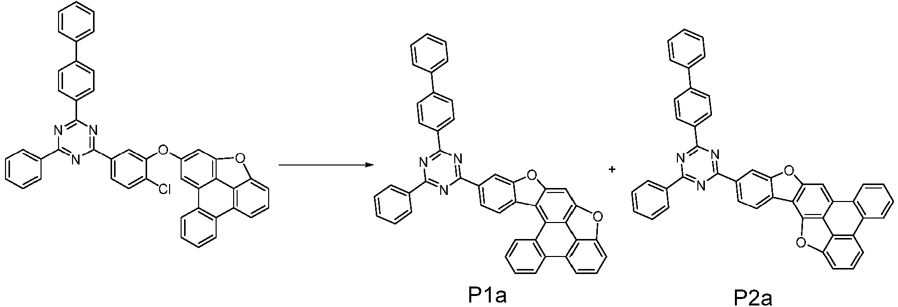

S1s (67.51 g, 100 mmol) und K₂CO₃ (41.46 g, 300 mmol) werden unter inerter Atmosphäre vorgelegt, mit DMAc (700 mL) versetzt und 30 min. inertisiert. Anschließend werden Pd(OAc)₂ (447 mg, 1.99 mmol) und 1,3-Bis-(2,6-diisopropyl-phenly)3-*H*-imidazol-1-iumchlorid (1.69 g, 3.98 mmol) zugegeben und das Reaktionsgemisch 22 h bei 150 °C gerührt. Nach dem Abkühlen wird der Ansatz in Ethanol/Wasser (1:1, 600 mL) gegossen und 30 min. nachgerührt. Der ausgefallene Feststoff wird abgesaugt und fünfmal mit Wasser und dreimal mit Ethanol gewaschen. Das Rohproduktgemisch wird chromatographisch getrennt und anschließend weiter über Heißextraktion aufgereinigt. P1a wird mit o-Xylol dreimal über Alox basisch heißextrahiert, P2a wird viermal mit Toluol über Alox basisch heißextrahiert. Beide Materialien werden abschließend im Hochvakuum sublimiert. Ausbeute: P1a: 11.5 g (18.0 mmol, 18%); Reinheit >99.9% nach HPLC; P2a: 14.5 g (22.7 mmol, 23%); Reinheit >99.9% nach HPLC.

Analog können folgende Verbindungen dargestellt werden. Dabei kann neben 1,3-Bis-(2,6-diisopropyl-phenly)3-*H*-imidazol-1-iumchlorid auch Tritert-butylphosphin oder Tricyclohexylphosphin als Ligand verwendet werden oder als Pd-Quelle neben Pd(OAc)₂ auch Pd₂(dba)₃. Zur Aufreinigung kann Säulenchromatographie, Heißextraktion oder Umkristallisation verwendet werden. Zur Umkristallisation oder Heißextraktion können gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4-Dioxan oder zur Umkristallisation Hochsieder wie Dimethylsulfoxid, N,N-Dimethylformamid, N,N-Dimethylacetamid, N-Methylpyrrolidon, etc. verwendet werden.

| Edukt 1 | Produkt | Ausbeute |
|---|---|---|
| 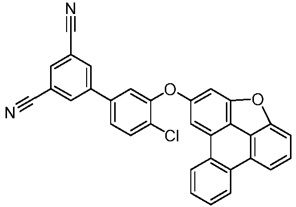 S2s | 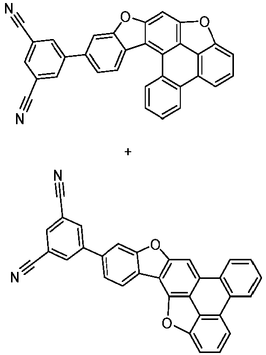 P3a* + P4a* | P3a* 14% + P4a* 11% |
| 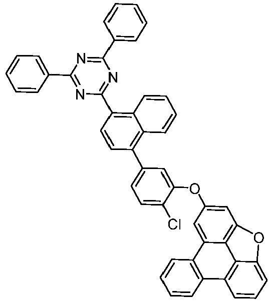 S3s | 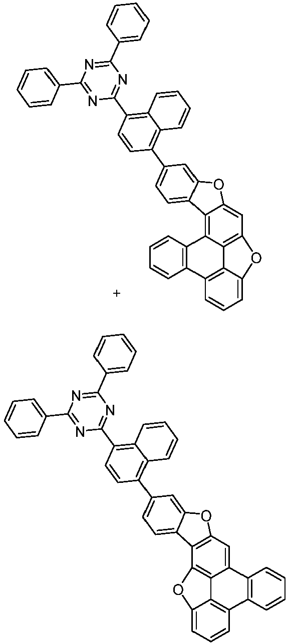 P5a* + P6a* | P5a* 20% + P6a* 13% |
| 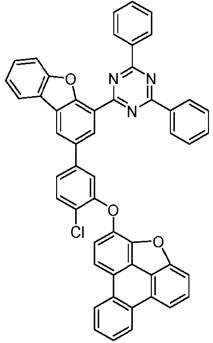 S4s | 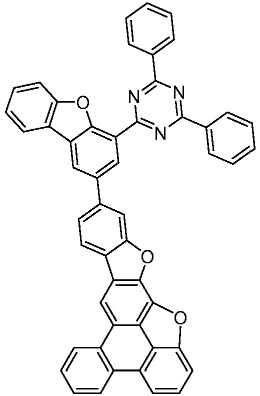 P7a* | 43% |
| 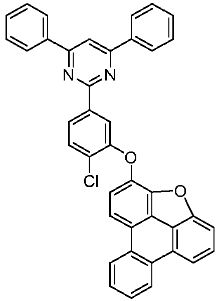 S5s | 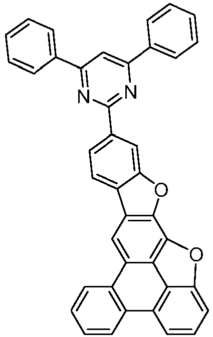 P8a* | 38% |
| 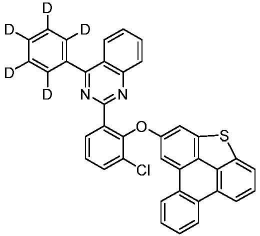 S6s | 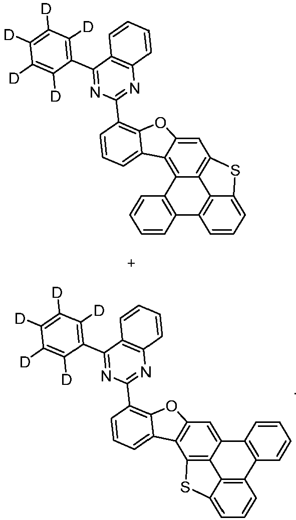 P9a* + P10a* | P9a* 12% + P10a* 17% |
| 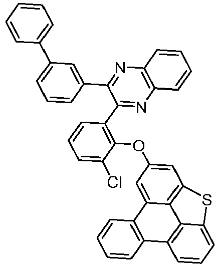 S7s | 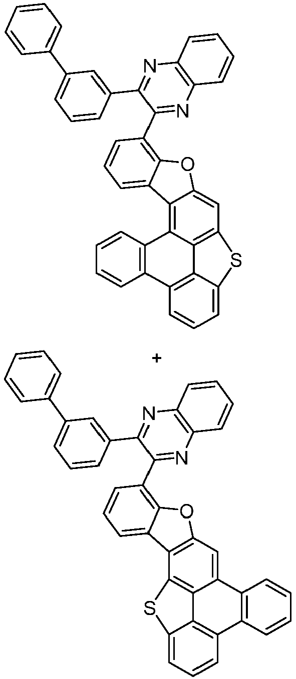 P11a* + P12a* | P11a* 15% |
| | | P12a* 16% |
| 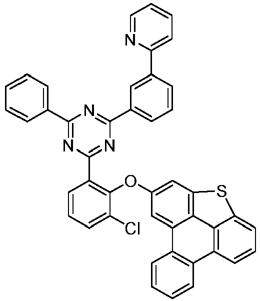 S8s | 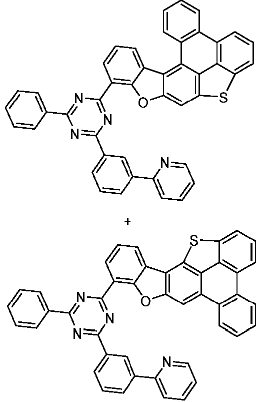 P13a* + P14a* | P13a* 9% + P14a* 14% |
| 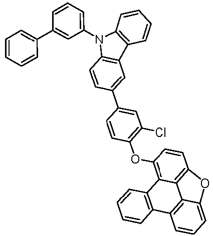 S9s | 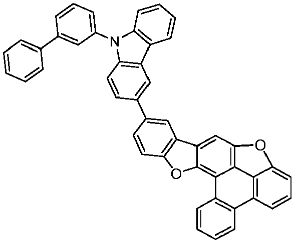 P15a* | 55% |
| 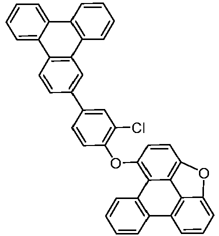 S10s | 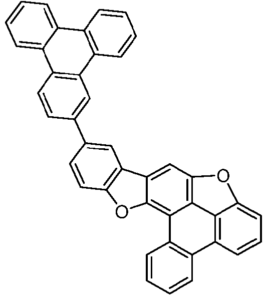 P16a* | 61% |
| 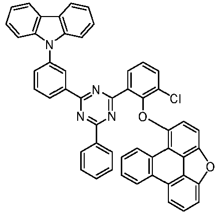 S11s | 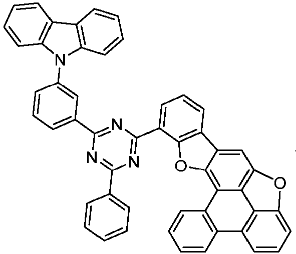 P17a* | 49% |
| 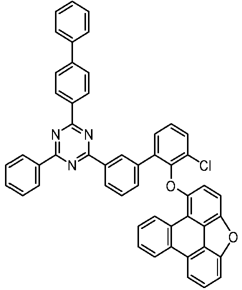 S12s | 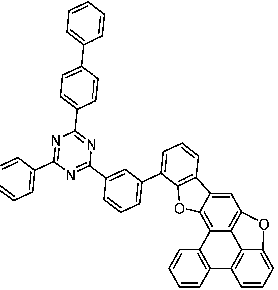 P18a* | 40% |
| 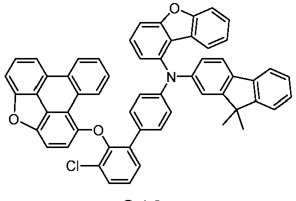 S13s | 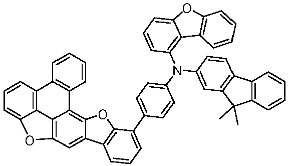 P19a* | 34% |

| | | |
|---|---|---|
| ***nicht erfindungsgemäß** | | |

### P1b:

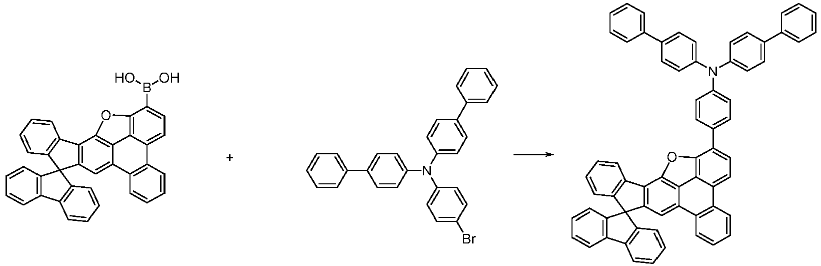

S1o (52.44 g, 100 mmol), Bis-biphenyl-4-yl-(4-bromphenyl)amin (47.64 g, 100 mmol) und K₃PO₄ (63.79 g, 300 mmol) werden in THF (1200 mL) und Wasser (300 mL) vorgelegt und 30 min. mit Argon inertisiert. Anschließend werden nacheinander Pd(OAc)₂ (448 mg, 2.00 mmol) und X-Phos (1.99 g, 4.00 mmol) zugegeben und der Ansatz 16 h unter Rückfluss gerührt. Nach dem Abkühlen wird der ausgefallene Feststoff abgesaugt und mit Wasser und Ethanol gewaschen. Das Rohprodukt wird viermal mit o-Xylol über Alox basisch heißextrahiert und abschließend im Hochvakuum sublimiert. Ausbeute: 54.58 g, (62.3 mmol, 62%); Reinheit >99.9% nach HPLC.

Analog können folgende Verbindungen dargestellt werden. Als Katalysatorsystem kann auch S-Phos oder P(o-tol)₃ oder P(tBu₃) mit Pd₂(dba)₃ oder Pd(OAc)₂ verwendet werden oder die Katalysatorsysteme Pd(PPh₃)₄ oder Pd(PPh₃)₂Cl₂. Zur Aufreinigung kann Säulenchromatographie, Heißextraktion oder Umkristallisation verwendet werden. Zur Umkristallisation oder Heißextraktion können gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4-Dioxan oder zur Umkristallisation Hochsieder wie Dimethylsulfoxid, N,N-Dimethylformamid, N,N-Dimethylacetamid, N-Methylpyrrolidon, etc. verwendet werden.

| Edukt 1 | Edukt 2 | Produkt | Ausbeute |
|---|---|---|---|
| 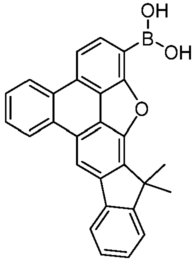 S2o | 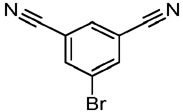 CAS-160892-07-9 | 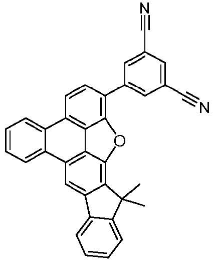 P2b | 57% |
| 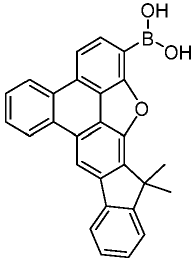 S2o | 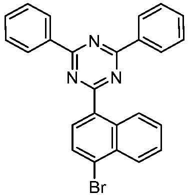 CAS-1800220-86-7 | 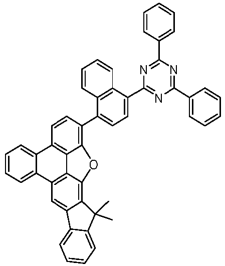 P3b | 42% |
| 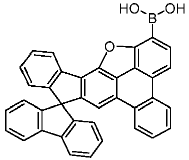 S1o | 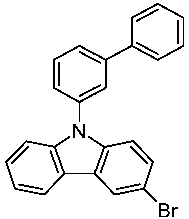 CAS-1428551-28-3 | 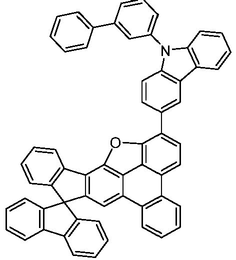 P4b | 51% |
| 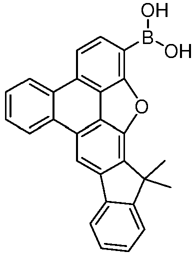 S2o | 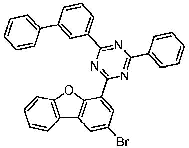 CAS-2375066-17-2 | 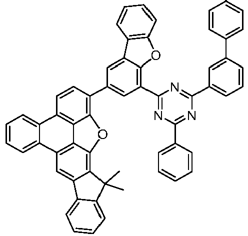 P5b | 36% |
| 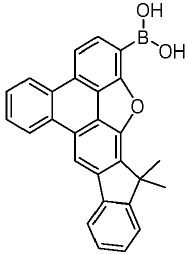 S2o | 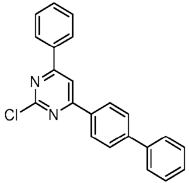 | 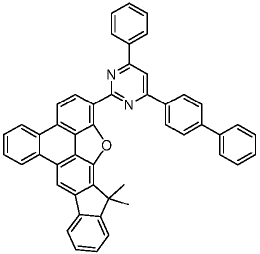 P6b | 58% |
| 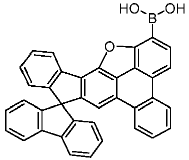 S1o | 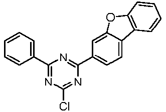 CAS-2142681-84-1 | 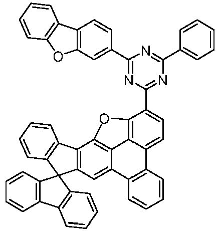 P7b | 49% |
| 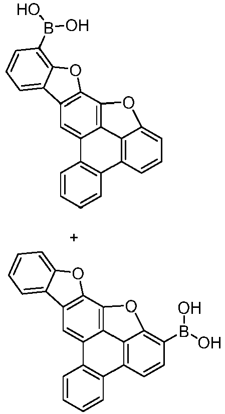 | 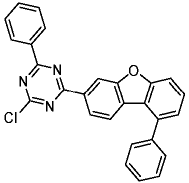 | 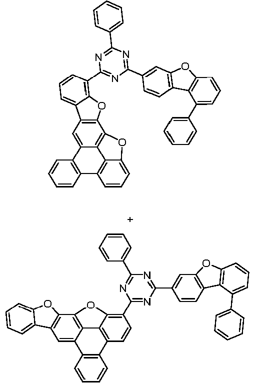 P8b* + P9b* Chromatograph. Trennung | 13% P8b* + 17% P9b* |

| | | | |
|---|---|---|---|
| *nicht erfindungsgemäß | | | |

### Herstellung der OLEDs

In den folgenden Beispielen (siehe Tabellen 1 bis 5) wird der Einsatz der erfindungsgemäßen Verbindungen in OLEDs im Vergleich zu Materialien aus dem Stand der Technik vorgestellt.

### Vorbehandlung für die Beispiele V1 bis V6 und E1a bis E6h:

Glasplättchen, die mit strukturiertem ITO (Indium Zinn Oxid) der Dicke 50 nm beschichtet sind, werden vor der Beschichtung zunächst mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 und 3 zu entnehmen. Die zur Herstellung der OLEDs benötigten Materialien, sofern sie nicht schon zuvor beschrieben wurden, sind in Tabelle 7 gezeigt. Die Device-Daten der OLEDs sind in Tabelle 2 und 4 aufgelistet.

Alle Materialien werden in einer Vakuumkammer thermisch aufgedampft. Dabei besteht die Emissionsschicht immer aus mindestens zwei Matrixmaterialien und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie P1a:H1:TE2 (32%:60%:8%) bedeutet hierbei, dass das Material P1a in einem Volumenanteil von 32%, H1 in einem Volumenanteil von 60% und TE2 in einem Volumenanteil von 8% in der Schicht vorliegt. Analog kann auch die Elektronentransportschicht aus einer Mischung von zwei Materialien bestehen.

Die Elektrolumineszenzspektren werden bei einer Leuchtdichte von 1000 cd/m² bestimmt und daraus die CIE 1931 x und y Farbkoordinaten berechnet. Die Angabe U10 in Tabelle 2 und 6 bezeichnet die Spannung, die für eine Stromdichte von 10 mA/cm² benötigt wird. EQE10 bezeichnet die externe Quanteneffizienz, die bei 10 mA/cm² erreicht wird. Als Lebensdauer LD wird die Zeit definiert, nach der die Leuchtdichte gemessen in cd/m² in Vorwärtsrichtung, bei Betrieb mit konstanter Stromdichte j₀ von der Startleuchtdichte auf einen gewissen Anteil L1 absinkt. Eine Angabe L1=80% in Tabelle 2 bedeutet, dass die in Spalte LD angegebene Lebensdauer der Zeit entspricht, nach der die Leuchtdichte in cd/m² auf 80% ihres Anfangswertes absinkt.

Die Angabe U1000 in Tabelle 4 bezeichnet die Spannung, die für eine Leuchtdichte von 1000 cd/m² benötigt wird. EQE1 000 bezeichnet die externe Quanteneffizienz, die bei 1000 cd/m² erreicht wird. Als Lebensdauer LD wird die Zeit definiert, nach der die Leuchtdichte bei Betrieb mit konstanter Stromdichte j₀ von der Startleuchtdichte auf einen gewissen Anteil L1 absinkt. Eine Angabe L1=95% in Tabelle 4 bedeutet, dass die in Spalte LD angegebene Lebensdauer der Zeit entspricht, nach der die Leuchtdichte auf 95% ihres Anfangswertes absinkt.

### Verwendung von erfindungsgemäßen Verbindungen in OLEDs

Die erfindungsgemäßen Materialien werden in den Beispielen E1c, E2f, E6g und E6i als Matrixmaterialien in der Emissionsschicht grün bzw. rot phosphoreszierender OLEDs, eingesetzt. Als Vergleich aus dem Stand der Technik kommen die Materialien SdT1, SdT2 und SdT3 in Kombination mit den Hostmaterialien H1, H2 und H3 bzw. mit den Hostmaterialien E1 und E2 in den Vergleichsbeispielen V1 bis V6 zum Einsatz. Beim Vergleich der erfindungsgemäßen Beispiele mit den entsprechenden Vergleichsbeispielen ist deutlich ersichtlich, dass die erfindungsgemäßen Beispiele jeweils einen deutlichen Vorteil in der Lebensdauer der OLED aufzeigen.

**Tabelle 1: Aufbau der OLEDs für grün**

| Bsp | HIL Dicke | HTL Dicke | EBL Dicke | EML Dicke | HBL Dicke | ETL Dicke | EIL Dicke |
|---|---|---|---|---|---|---|---|
| V1 | SpMA1:PD1 (95%:5%) 20nm | SpMA1 200nm | SpMA2 20nm | E2:SdT1:TE1 (22%:70%:8%) 40nm | ST2 5nm | ST2:LiQ (50%:50%) 30nm | LiQ 1nm |
| E1a* | SpMA1:PD1 (95%:5%) 20nm | SpMA1 200nm | SpMA2 20nm | E2:P15a:TE1 (22%:70%:8%) 40nm | ST2 5nm | ST2:LiQ (50%:50%) 30nm | LiQ 1nm |
| E1b* | SpMA1:PD1 (95%:5%) 20nm | SpMA1 200nm | SpMA2 20nm | E2:P19a:TE1 (22%:70%:8%) 40nm | ST2 5nm | ST2:LiQ (50%:50%) 30nm | LiQ 1nm |
| E1c | SpMA1:PD1 (95%:5%) 20nm | SpMA1 200nm | SpMA2 20nm | E2:P4b:TE1 (22%:70%:8%) 40nm | ST2 5nm | ST2:LiQ (50%:50%) 30nm | LiQ 1nm |
| | | | | | | | |
| V2 | SpMA1:PD1 (95%:5%) 20nm | SpMA1 200nm | SpMA2 20nm | SdT2:H1:TE2 (32%:60%:8%) 40nm | ST2 5nm | ST2:LiQ (50%:50%) 30nm | LiQ 1nm |
| E2a* | SpMA1:PD1 (95%:5%) 20nm | SpMA1 200nm | SpMA2 20nm | P1a:H1:TE2 (32%:60%:8%) 40nm | ST2 5nm | ST2:LiQ (50%:50%) 30nm | LiQ 1nm |
| E2b* | SpMA1:PD1 (95%:5%) 20nm | SpMA1 200nm | SpMA2 20nm | P2a:H1:TE2 (32%:60%:8%) 40nm | ST2 5nm | ST2:LiQ (50%:50%) 30nm | LiQ 1nm |
| E2c* | SpMA1:PD1 (95%:5%) 20nm | SpMA1 200nm | SpMA2 20nm | P3a:H1:TE2 (32%:60%:8%) 40nm | ST2 5nm | ST2:LiQ (50%:50%) 30nm | LiQ 1nm |
| E2d* | SpMA1:PD1 (95%:5%) 20nm | SpMA1 200nm | SpMA2 20nm | P7a:H1:TE2 (32%:60%:8%) 40nm | ST2 5nm | ST2:LiQ (50%:50%) 30nm | LiQ 1nm |
| E2e* | SpMA1:PD1 (95%:5%) 20nm | SpMA1 200nm | SpMA2 20nm | P8a:H1:TE2 (32%:60%:8%) 40nm | ST2 5nm | ST2:LiQ (50%:50%) 30nm | LiQ 1nm |
| E2f | SpMA1:PD1 (95%:5%) 20nm | SpMA1 200nm | SpMA2 20nm | P7b:H1:TE2 (32%:60%:8%) 40nm | ST2 5nm | ST2:LiQ (50%:50%) 30nm | LiQ 1nm |
| E2g* | SpMA1:PD1 (95%:5%) 20nm | SpMA1 200nm | SpMA2 20nm | P8b:H1:TE2 (32%:60%:8%) 40nm | ST2 5nm | ST2:LiQ (50%:50%) 30nm | LiQ 1nm |
| | | | | | | | |
| V3 | SpMA1:PD1 (95%:5%) 20nm | SpMA1 200nm | SpMA2 20nm | SdT3:H2:TE1 (38%:50%:12%) 40nm | ST2 5nm | ST2:LiQ (50%:50%) 30nm | LiQ 1nm |
| E3a* | SpMA1:PD1 (95%:5%) 20nm | SpMA1 200nm | SpMA2 20nm | P17a:H2:TE1 (38%:50%:12%) 40nm | ST2 5nm | ST2:LiQ (50%:50%) 30nm | LiQ 1nm |
| E3b* | SpMA1:PD1 (95%:5%) 20nm | SpMA1 200nm | SpMA2 20nm | P9b:H2:TE1 (38%:50%:12%) 40nm | ST2 5nm | ST2:LiQ (50%:50%) 30nm | LiQ 1nm |
| E3c* | SpMA1:PD1 (95%:5%) 20nm | SpMA1 200nm | SpMA2 20nm | P18a:H2:TE1 (38%:50%:12%) 40nm | ST2 5nm | ST2:LiQ (50%:50%) 30nm | LiQ 1nm |
| | | | | | | | |
| V4 | SpMA1:PD1 (95%:5%) 20nm | SpMA1 200nm | SpMA2 20nm | E1:SdT1:TE2 (32%:60%:8%) 40nm | ST2 5nm | ST2:LiQ (50%:50%) 30nm | LiQ 1nm |
| E4a* | SpMA1:PD1 (95%:5%) 20nm | SpMA1 200nm | SpMA2 20nm | E2:P16a:TE1 (22%:70%:8%) 40nm | ST2 5nm | ST2:LiQ (50%:50%) 30nm | LiQ 1nm |
| | | | | | | | |
| V5 | SpMA1:PD1 (95%:5%) 20nm | SpMA1 200nm | SpMA2 20nm | E1:H1:TE2 (32%:60%:8%) 40nm | ST2 5nm | ST2:LiQ (50%:50%) 30nm | LiQ 1nm |
| E5a* | SpMA1:PD1 (95%:5%) 20nm | SpMA1 200nm | P19a 20nm | E1:H1:TE2 (32%:60%:8%) 40nm | ST2 5nm | ST2:LiQ (50%:50%) 30nm | LiQ 1nm |

| | | | | | | | |
|---|---|---|---|---|---|---|---|
| *nicht erfindungsgemäß | | | | | | | |

**Tabelle 2: Daten der OLEDs für grün**

| Bsp. | U10 (V) | EQE10 (%) | CIE x/y bei 1000 cd/m² | j₀ (mA/cm²) | L1 (%) | LD (h) |
|---|---|---|---|---|---|---|
| V1 | 5.2 | 17.8 | 0.34/0.62 | 40 | 80 | 190 |
| E1a* | 5.4 | 18.7 | 0.34/0.62 | 40 | 80 | 590 |
| E1b* | 5.2 | 18.2 | 0.34/0.63 | 40 | 80 | 440 |
| E1c | 5.4 | 18.5 | 0.34/0.63 | 40 | 80 | 665 |
| | | | | | | |
| V2 | 4.4 | 20.5 | 0.34/0.63 | 40 | 80 | 200 |
| E2a* | 4.1 | 20.4 | 0.35/0.63 | 40 | 80 | 370 |
| E2b* | 4.2 | 20.0 | 0.35/0.63 | 40 | 80 | 440 |
| E2c* | 4.3 | 21.9 | 0.34/0.63 | 40 | 80 | 315 |
| E2d* | 4.4 | 22.2 | 0.35/0.63 | 40 | 80 | 690 |
| E2e* | 4.2 | 22.9 | 0.34/0.63 | 40 | 80 | 570 |
| E2f | 4.1 | 21.8 | 0.35/0.63 | 40 | 80 | 555 |
| E2g* | 4.2 | 22.3 | 0.34/0.63 | 40 | 80 | 710 |
| | | | | | | |
| V3 | 5.3 | 16.6 | 0.34/0.62 | 40 | 80 | 450 |
| E3a* | 5.2 | 17.0 | 0.33/0.63 | 40 | 80 | 990 |
| E3b* | 4.9 | 17.5 | 0.34/0.62 | 40 | 80 | 590 |
| E3c* | 5.0 | 17.4 | 0.33/0.62 | 40 | 80 | 915 |
| | | | | | | |
| V4 | 4.5 | 21.2 | 0.34/0.63 | 40 | 80 | 220 |
| E4a* | 4.9 | 21.2 | 0.34/0.63 | 40 | 80 | 550 |
| | | | | | | |
| V5 | 4.4 | 22.2 | 0.34/0.63 | 40 | 80 | 760 |
| E5a* | 4.2 | 22.7 | 0.33/0.63 | 40 | 80 | 1120 |

| | | | | | | |
|---|---|---|---|---|---|---|
| *nicht erfindungsgemäß | | | | | | |

**Tabelle 3: Aufbau der OLEDs für rot**

| Bsp | HIL Dicke | HTL Dicke | EBL Dicke | EML Dicke | HBL Dicke | ETL Dicke | EIL Dicke |
|---|---|---|---|---|---|---|---|
| V6 | SpMA1:PD1 (95%:5%) 20nm | SpMA1 110nm | SpMA2 10nm | SdT2:H3:TER1 (57%:40%:3%) 35nm | ST2 10nm | ST2:LiQ (50%:50%) 30nm | LiQ 1nm |
| E6a* | SpMA1:PD1 (95%:5%) 20nm | SpMA1 110nm | SpMA2 10nm | P5a:H3:TER1 (57%:40%:3%) 35nm | ST2 10nm | ST2:LiQ (50%:50%) 30nm | LiQ 1nm |
| E6b* | SpMA1:PD1 (95%:5%) 20nm | SpMA1 110nm | SpMA2 10nm | P6a:H3:TER1 (57%:40%:3%) 35nm | ST2 10nm | ST2:LiQ (50%:50%) 30nm | LiQ 1nm |
| E6c* | SpMA1:PD1 (95%:5%) 20nm | SpMA1 110nm | SpMA2 10nm | P9a:H3:TER1 (57%:40%:3%) 35nm | ST2 10nm | ST2:LiQ (50%:50%) 30nm | LiQ 1nm |
| E6d* | SpMA1:PD1 (95%:5%) 20nm | SpMA1 110nm | SpMA2 10nm | P10a:H3:TER1 (57%:40%:3%) 35nm | ST2 10nm | ST2:LiQ (50%:50%) 30nm | LiQ 1nm |
| E6e* | SpMA1:PD1 (95%:5%) 20nm | SpMA1 110nm | SpMA2 10nm | P11a:H3:TER1 (57%:40%:3%) 35nm | ST2 10nm | ST2:LiQ (50%:50%) 30nm | LiQ 1nm |
| E6f* | SpMA1:PD1 (95%:5%) 20nm | SpMA1 110nm | SpMA2 10nm | P12a:H3:TER1 (57%:40%:3%) 35nm | ST2 10nm | ST2:LiQ (50%:50%) 30nm | LiQ 1nm |
| E6g | SpMA1:PD1 (95%:5%) 20nm | SpMA1 110nm | SpMA2 10nm | P6b:H3:TER1 (57%:40%:3%) 35nm | ST2 10nm | ST2:LiQ (50%:50%) 30nm | LiQ 1nm |
| E6h* | SpMA1:PD1 (95%:5%) 20nm | SpMA1 110nm | SpMA2 10nm | P13a:H3:TER1 (57%:40%:3%) 35nm | ST2 10nm | ST2:LiQ (50%:50%) 30nm | LiQ 1nm |
| E6i | SpMA1:PD1 (95%:5%) 20nm | SpMA1 110nm | SpMA2 10nm | SdT1:P1b:TER1 (57%:40%:3%) 35nm | ST2 10nm | ST2:LiQ (50%:50%) 30nm | LiQ 1nm |

| | | | | | | | |
|---|---|---|---|---|---|---|---|
| *nicht erfindungsgemäß | | | | | | | |

**Tabelle 4: Daten der OLEDs für rot**

| Bsp. | U100 0 (V) | EQE 1000 (%) | CIE x/y bei 1000 cd/m² | j₀ (mA/cm²) | L1 (%) | LD (h) |
|---|---|---|---|---|---|---|
| V6 | 3.7 | 24.1 | 0.66/0.33 | 60 | 95 | 12 |
| E6a* | 3.5 | 25.4 | 0.67/0.33 | 60 | 95 | 66 |
| E6b* | 3.4 | 26.5 | 0.67/0.33 | 60 | 95 | 74 |
| E6c* | 3.8 | 25.8 | 0.67/0.33 | 60 | 95 | 105 |
| E6d* | 3.8 | 25.7 | 0.67/0.33 | 60 | 95 | 112 |
| E6e* | 3.3 | 26.3 | 0.66/0.33 | 60 | 95 | 84 |
| E6f* | 3.4 | 26.6 | 0.66/0.33 | 60 | 95 | 80 |
| E6g | 3.5 | 26.8 | 0.66/0.33 | 60 | 95 | 35 |
| E6h* | 3.3 | 25.5 | 0.66/0.33 | 60 | 95 | 21 |
| E6i | 3.4 | 24.7 | 0.66/0.33 | 60 | 95 | 17 |

| | | | | | | |
|---|---|---|---|---|---|---|
| *nicht erfindungsgemäß | | | | | | |

**Tabelle 5: Strukturformeln der verwendeten Materialien der OLEDs, sofern nicht schon zuvor beschrieben:**

| | |
|---|---|
| 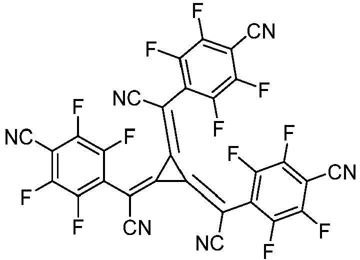 | 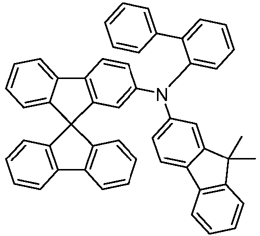 |
| PD1 (CAS Reg. No. 1224447-88-4) | SpMA1 |
| 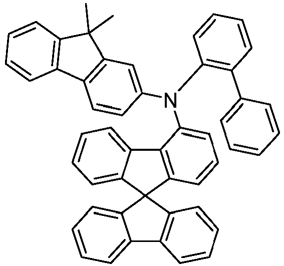 | 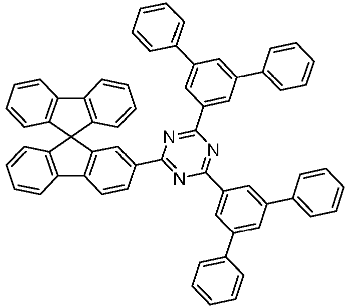 |
| SpMA2 | ST2 |
| 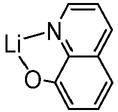 | 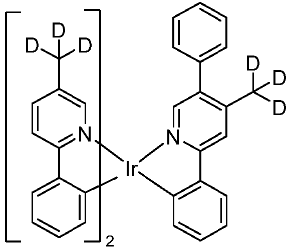 |
| LiQ | TE1 |
| 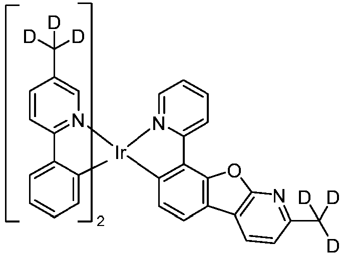 | 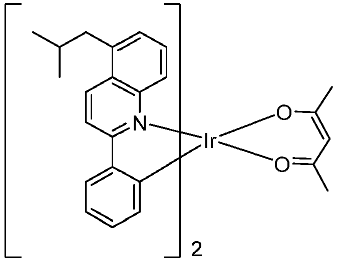 |
| TE2 | TER1 |
| 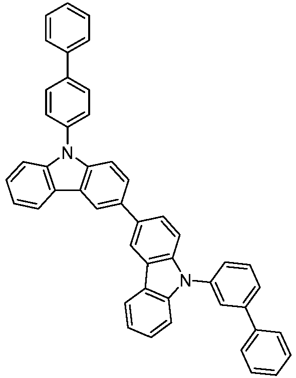 | 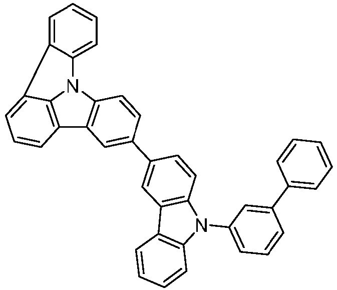 |
| H1 | H2 |
| 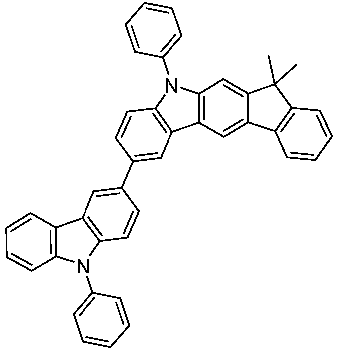 | |
| H3 | |
| 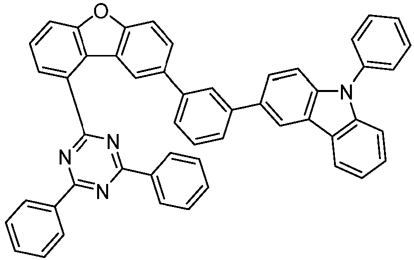 | 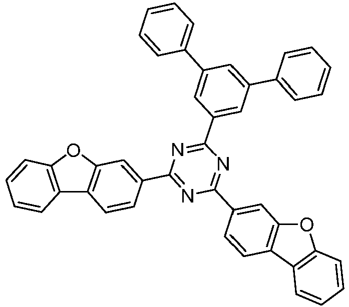 |
| E1 | E2 |
| 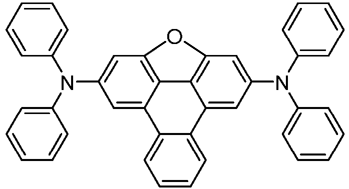 | 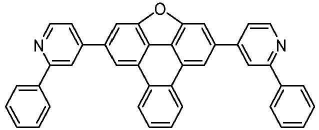 |
| SdT1 | SdT2 (WO 2012/048781) |
| 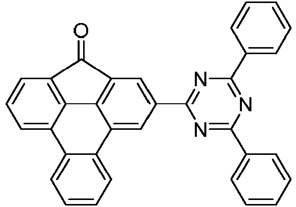 | |
| SdT3 (WO 2012/048781) | |

## Patentansprüche

1. Verbindung gemäß Formel (1), 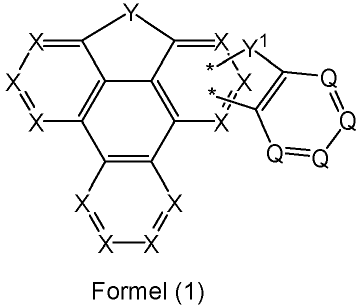 wobei für die verwendeten Symbole gilt:
X ist gleich oder verschieden bei jedem Auftreten CR oder N mit der Maßgabe, dass maximal zwei Gruppen X pro Zyklus für N stehen und dass zwei benachbarte Gruppen X, die Teil des Zyklus mit drei Gruppen X sind, für C stehen, welche über die mit * bezeichneten Bindungen ein an den Zyklus ankondensiertes aromatisches oder heteroaromatisches Ringsystem bilden;
Y ist C(R)₂, O oder S;
Y¹ ist C(R)₂;
Q ist gleich oder verschieden bei jedem Auftreten CR oder N mit der Maßgabe, dass maximal zwei Gruppen Q pro Zyklus für N stehen.
R ist bei jedem Auftreten gleich oder verschieden H, D, F, Cl, Br, I, N(Ar')₂, N(R¹)₂, OAr', SAr', B(OR¹)₂, CHO, C(=O)R¹, CR¹=C(R¹)₂, CN, C(=O)OR¹, C(=O)NR¹, Si(R¹)₃, NO₂, P(=O)(R¹)₂, OSO₂R¹, OR¹, S(=O)R¹, S(=O)₂R¹, SR¹, eine geradkettige Alkylgruppe mit 1 bis 20 C-Atomen oder eine Alkenyl- oder Alkinylgruppe mit 2 bis 20 C-Atomen oder eine verzweigte oder zyklische Alkyl- gruppe mit 3 bis 20 C-Atomen, wobei die Alkyl-, Alkenyl- oder Alkinylgruppe jeweils mit einem oder mehreren Resten R¹ substituiert sein kann, wobei eine oder mehrere nicht benachbarte CH₂-Gruppen durch -R¹C=CR¹-, -C≡C-, Si(R¹)₂, NR¹, CONR¹, C=O, C=S, -C(=O)O-, P(=O)(R¹), -O-, -S-, SO oder SO₂ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann, wobei zwei oder mehr an den gleichen Zyklus gebundene Reste R miteinander ein aliphatisches, heteroaliphatisches, aromatisches oder heteroaromatisches Ringsystem bilden können, das mit einem oder mehreren Resten R¹ substituiert sein kann, und wobei zwei an dasselbe Kohlenstoff-, Silicium-, Germanium- oder Zinnatom gebundene Reste R ein monozyklisches oder polyzyklisches, aliphatisches, aromatisches oder heteroaromatisches Ringsystem miteinander bilden können, das mit einem oder mehreren Resten R¹ substituiert sein kann;
Ar' ist bei jedem Auftreten gleich oder verschieden ein aromatisches oder heteroaromatisches Ringsystem mit 5 bis 40 aromatischen Ringatomen, das durch einen oder mehrere Reste R¹ substituiert sein kann;
R¹ ist bei jedem Auftreten gleich oder verschieden H, D, F, I, B(OR²)₂, N(R²)₂, CHO, C(=O)R², CR²=C(R²)₂, CN, C(=O)OR², Si(R²)₃, NO₂, P(=O)(R²)₂, OSO₂R², SR², OR², S(=O)R², S(=O)₂R², eine geradkettige Alkylgruppe mit 1 bis 20 C-Atomen oder eine Alkenyl- oder Alkinylgruppe mit 2 bis 20 C-Atomen oder eine verzweigte oder zyklische Alkylgruppe mit 3 bis 20 C-Atomen, wobei die Alkyl-, Alkenyl- oder Alkinylgruppe jeweils mit einem oder mehreren Resten R² substituiert sein kann und wobei eine oder mehrere CH₂-Gruppen in den oben genannten Gruppen durch -R²C=CR²-, -C=C-, Si(R²)₂, C=O, C=S, -C(=O)O-, NR², CONR², P(=O)(R²), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R² substituiert sein kann, wobei zwei oder mehr Reste R¹ miteinander ein aliphatisches, heteroaliphatisches, aromatisches oder heteroaromatisches Ringsystem bilden können;
R² ist bei jedem Auftreten gleich oder verschieden H, D, F, CN oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R² miteinander verknüpft sein und einen Ring bilden.

2. Verbindung nach Anspruch 1, ausgewählt aus den Verbindungen der Formeln (2-1) bis (2-4), 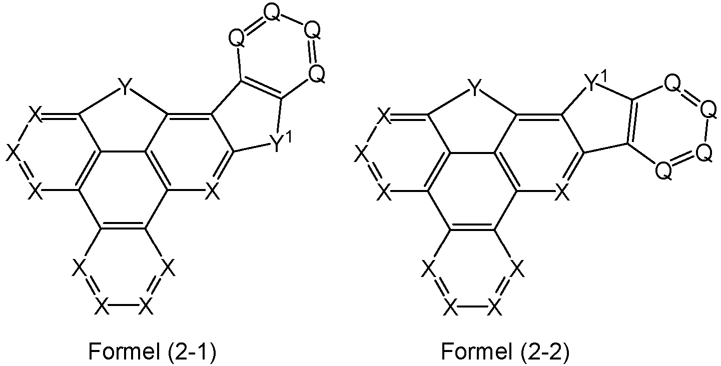 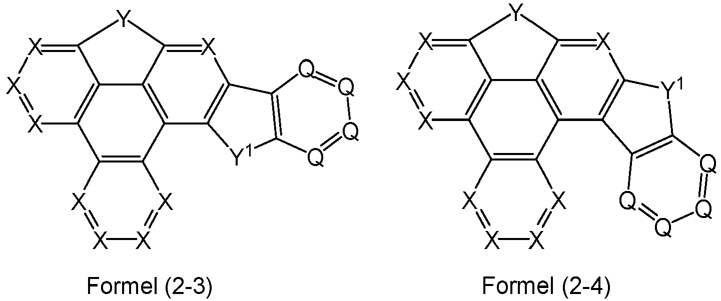 wobei die verwendeten Symbole die in Anspruch 1 genannten Bedeutungen aufweisen.

3. Verbindung nach Anspruch 1 oder 2, ausgewählt aus den Verbindungen der Formeln (3-1) bis (3-4), 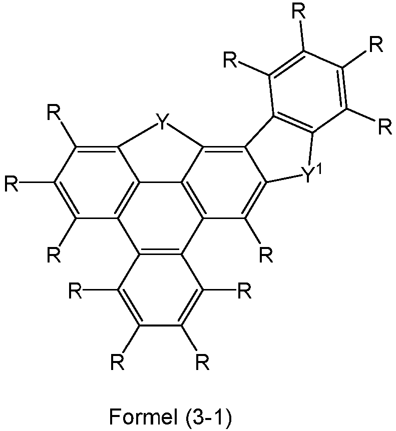 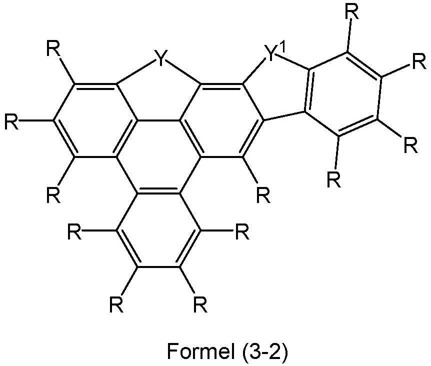 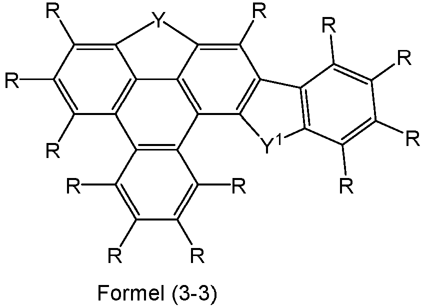 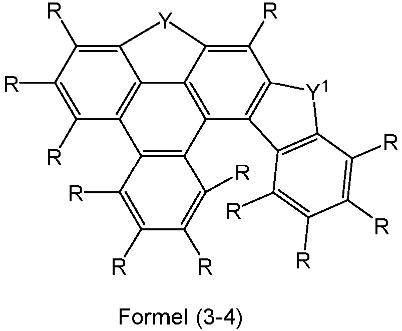 wobei die verwendeten Symbole die in Anspruch 1 genannten Bedeutungen aufweisen.

4. Verbindung nach einem oder mehreren der Ansprüche 1 bis 3, ausgewählt aus den Verbindungen der Formeln (3-1a) bis (3-4a), 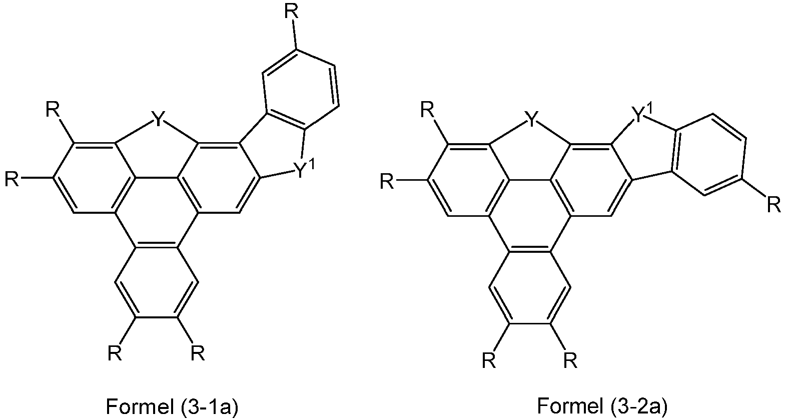 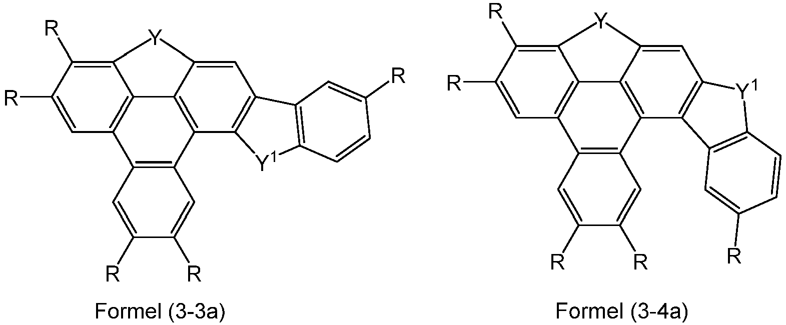 wobei die verwendeten Symbole die in Anspruch 1 genannten Bedeutungen aufweisen.

5. Verbindungen nach einem oder mehreren der Ansprüche 1 bis 4, ausgewählt aus den folgenden Verbindungen
| | | |
|---|---|---|
| | 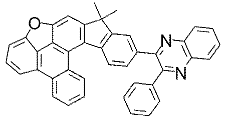 | 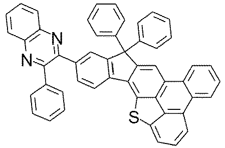 |
| 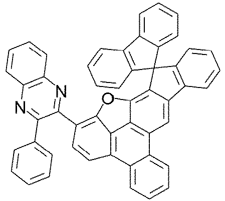 | 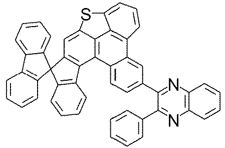 | 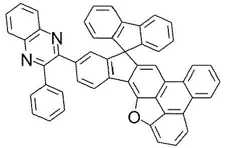 |
| | | 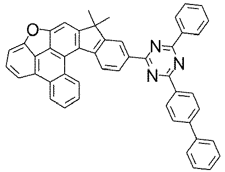 |
| 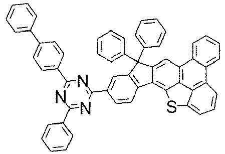 | 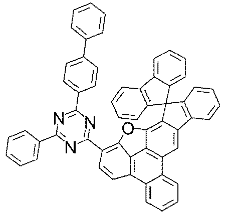 | 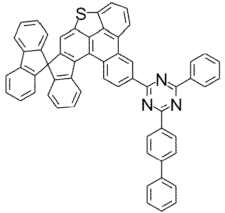 |
| 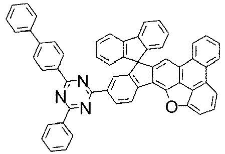 | 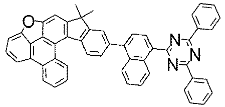 | |
| | 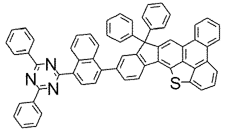 | 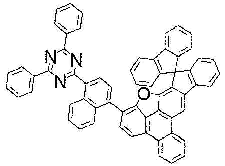 |
| 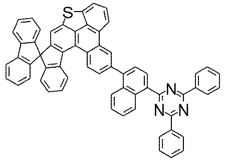 | 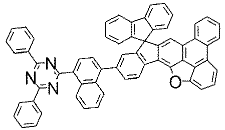 | |
| | 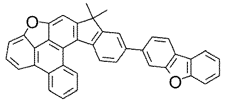 | 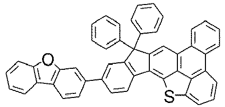 |
| 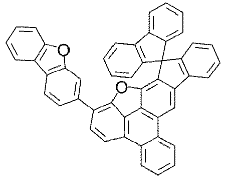 | 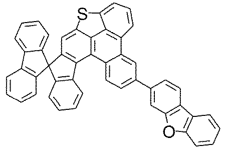 | 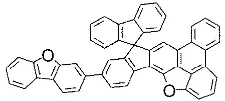 |
| | | 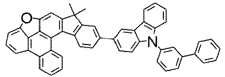 |
| 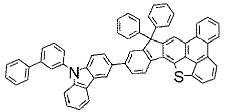 | 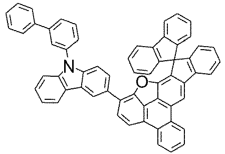 | 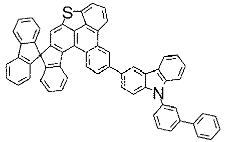 |
| 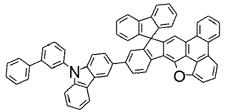 | 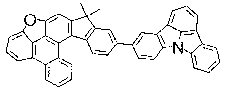 | |
| | 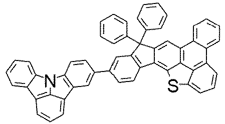 | 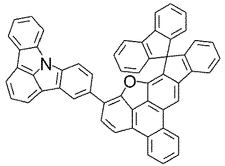 |
| 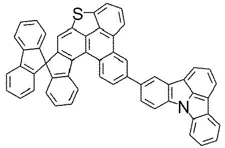 | 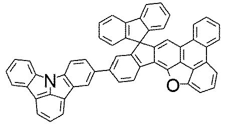 | 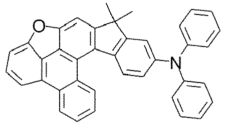 |
| | | 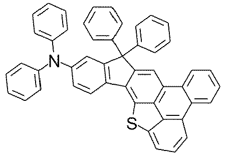 |
| 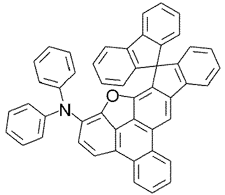 | 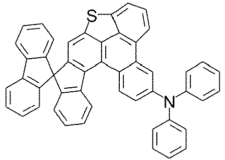 | 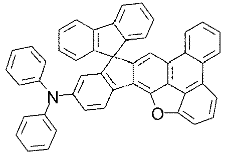 |
| | | 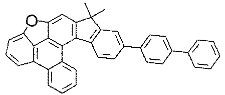 |
| 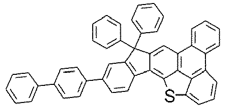 | 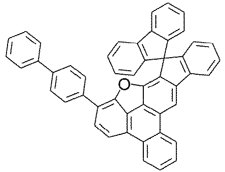 | 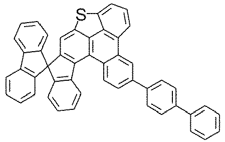 |
| 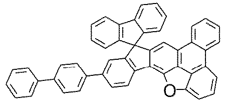 | 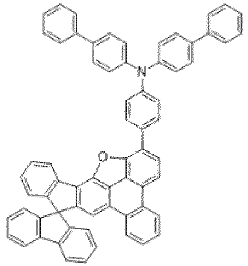 P1b | 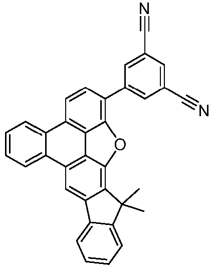 P2b |
| 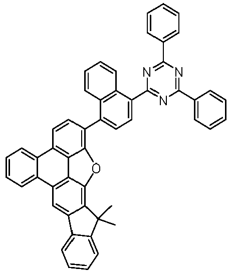 P3b | 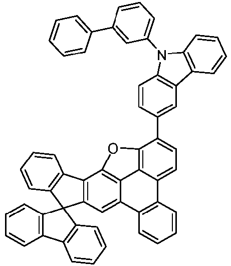 P4b | 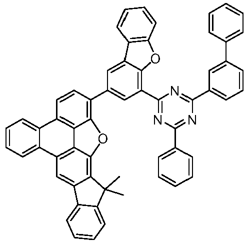 P5b |
| 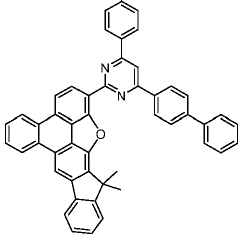 P6b | 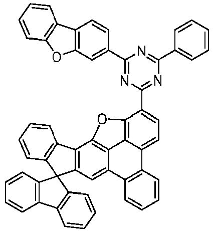 P7b. | |

6. Verfahren zur Herstellung einer Verbindung nach Anspruch 1, **gekennzeichnet durch** die folgenden Schritte:
(A) Synthese des Grundgerüsts nach Formel (1);
(B) Ankondensieren des aromatischen oder heteroaromatischen Ringsystems unter Bildung der mit * bezeichneten Bindungen nach Formel (1) durch Kupplungs- und Ringschlussreaktion.

7. Formulierung, enthaltend mindestens eine Verbindung nach einem oder mehreren der Ansprüche 1 bis 5 und mindestens eine weitere Verbindung und/oder mindestens ein Lösemittel.

8. Verwendung einer Verbindung nach einem oder mehreren der Ansprüche 1 bis 5 und oder einer Formulierung nach Anspruch 7 in einer elektronischen Vorrichtung.

9. Elektronische Vorrichtung enthaltend mindestens eine Verbindung nach einem oder mehreren der Ansprüche 1 bis 5 und/oder eine Formulierung nach Anspruch 7.

10. Elektronische Vorrichtung nach Anspruch 9, wobei es sich um eine organische Elektrolumineszenzvorrichtung handelt, **dadurch gekennzeichnet, dass** die Verbindung nach einem oder mehreren der Ansprüche 1 bis 5 in einer emittierenden Schicht als Matrixmaterial für phosphoreszierende oder fluoreszierende Emitter oder für Emitter, die TADF (thermally activated delayed fluorescence) zeigen, und/oder in einer Elektronentransportschicht und/oder in einer Lochblockierschicht und/oder in einer Lochtransportschicht und/oder in einer Elektronenblockierschicht eingesetzt wird.

11. Elektronische Vorrichtung nach Anspruch 10, wobei die emittierende Schicht die Verbindung nach einem oder mehreren der Ansprüche 1 bis 5 in Kombination mit einem oder mehreren phosphoreszierenden Materialien enthält.

12. Elektronische Vorrichtung nach Anspruch 10, wobei die emittierende Schicht die Verbindung nach einem oder mehreren der Ansprüche 1 bis 5 in Kombination mit einem weiteren Matrixmaterial enthält.

13. Elektronische Vorrichtung nach Anspruch 12, wobei das weitere Matrixmaterial aus der Gruppe der Biscarbazole, der verbrückten Carbazole, der Triarylamine, der Dibenzofuran-Carbazole, der Dibenzofuran-Amine und der Carbazolamine ausgewählt wird.

14. Elektronische Vorrichtung nach Anspruch 12, wobei das weitere Matrixmaterial aus der Gruppe der Triazine, Pyrimidine, Chinazoline oder Chinoxaline ausgewählt wird.

## Claims

1. Compound of the formula (1), 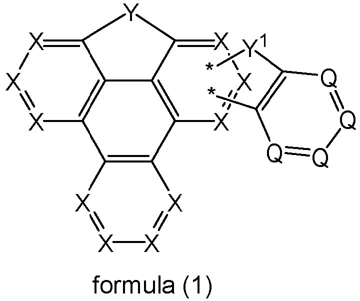 where the following applies to the symbols used:
X is, identically or differently on each occurrence, CR or N, with the proviso that a maximum of two groups X per ring stand for N and that two adjacent groups X that are part of the ring having three groups X stand for C and form, via the bonds denoted by *, an aromatic or heteroaromatic ring system which is condensed onto the ring;
Y is C(R)₂, O or S;
Y¹ is C(R)₂;
Q is, identically or differently on each occurrence, CR or N, with the proviso that a maximum of two groups Q per ring stand for N;
R is on each occurrence, identically or differently, H, D, F, Cl, Br, I, N(Ar')₂, N(R¹)₂, OAr', SAr', B(OR¹)₂, CHO, C(=O)R¹, CR¹=C(R¹)₂, CN, C(=O)OR¹, C(=O)NR¹, Si(R¹)₃, NO₂, P(=O)(R¹)₂, OSO₂R¹, OR¹, S(=O)R¹, S(=O)₂R¹, SR¹, a straight-chain alkyl group having 1 to 20 C atoms or an alkenyl or alkynyl group having 2 to 20 C atoms or a branched or cyclic alkyl group having 3 to 20 C atoms, where the alkyl, alkenyl or alkynyl group may in each case be substituted by one or more radicals R¹, where one or more non-adjacent CH₂ groups may be replaced by -R¹C=CR¹-, -C≡C-, Si(R¹)₂, NR¹, CONR¹, C=O, C=S, -C(=O)O-, P(=O)(R¹), -O-, -S-, SO or SO₂, or an aromatic or heteroaromatic ring system having 5 to 60 aromatic ring atoms, preferably having 5 to 40 aromatic ring atoms, which may in each case be substituted by one or more radicals R¹, where two or more radicals R bonded to the same ring may form with one another an aliphatic, heteroaliphatic, aromatic or heteroaromatic ring system, which may be substituted by one or more radicals R¹, and where two radicals R bonded to the same carbon, silicon, germanium or tin atom may form with one another a monocyclic or polycyclic, aliphatic, aromatic or heteroaromatic ring system, which may be substituted by one or more radicals R¹;
Ar' is on each occurrence, identically or differently, an aromatic or heteroaromatic ring system having 5 to 40 aromatic ring atoms, which may be substituted by one or more radicals R¹;
R¹ is on each occurrence, identically or differently, H, D, F, I, B(OR²)₂, N(R²)₂, CHO, C(=O)R², CR²=C(R²)₂, CN, C(=O)OR², Si(R²)₃, NO₂, P(=O)(R²)₂, OSO₂R², SR², OR², S(=O)R², S(=O)₂R², a straight-chain alkyl group having 1 to 20 C atoms or an alkenyl or alkynyl group having 2 to 20 C atoms or a branched or cyclic alkyl group having 3 to 20 C atoms, where the alkyl, alkenyl or alkynyl group may in each case be substituted by one or more radicals R² and where one or more CH₂ groups in the above-mentioned groups may be replaced by -R²C=CR²-, -C=C-, Si(R²)₂, C=O, C=S, -C(=O)O-, NR², CONR², P(=O)(R²), -O-, -S-, SO or SO₂ and where one or more H atoms in the above-mentioned groups may be replaced by D, F, Cl, Br, I, CN or NO₂, or an aromatic or heteroaromatic ring system having 5 to 30 aromatic ring atoms, which may in each case be substituted by one or more radicals R², where two or more radicals R¹ may form an aliphatic, heteroaliphatic, aromatic or heteroaromatic ring system with one another;
R² is on each occurrence, identically or differently, H, D, F, CN or an aliphatic, aromatic or heteroaromatic organic radical having 1 to 20 C atoms, in which, in addition, one or more H atoms may be replaced by D or F; two or more substituents R² may be linked to one another and form a ring.

2. Compound according to Claim 1, selected from the compounds of the formulae (2-1) to (2-4), 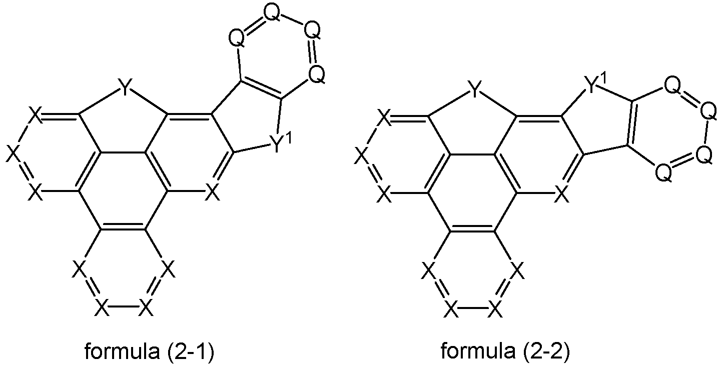 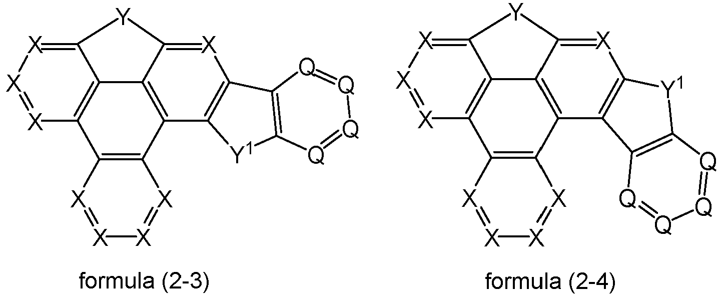 where the symbols used have the meanings given in Claim 1.

3. Compound according to Claim 1 or 2, selected from the compounds of the formulae (3-1) to (3-4), 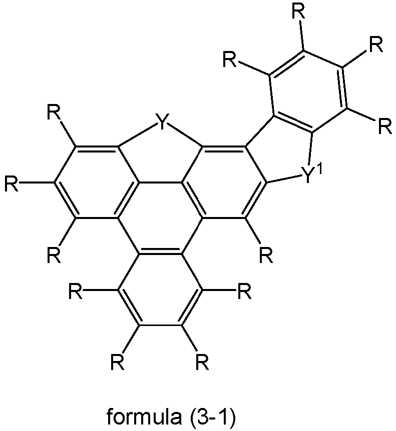 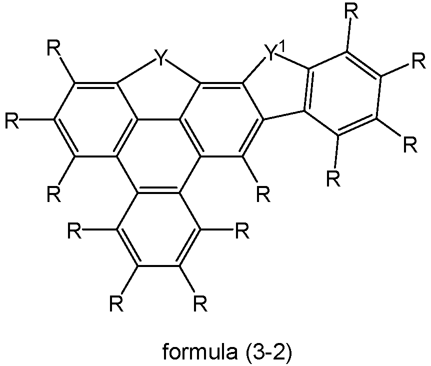 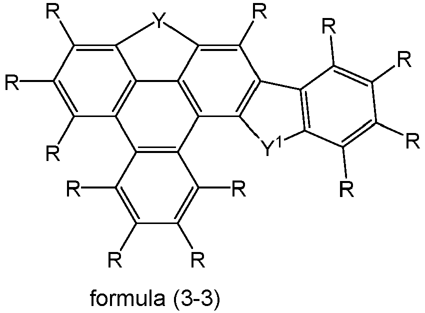 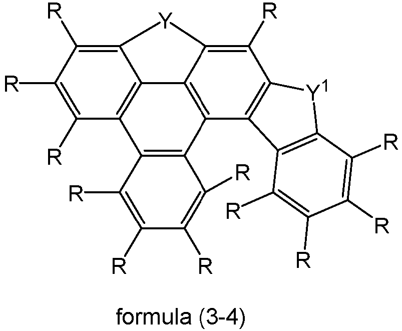 where the symbols used have the meanings given in Claim 1.

4. Compound according to one or more of Claims 1 to 3, selected from the compounds of the formulae (3-1a) to (3-4a), 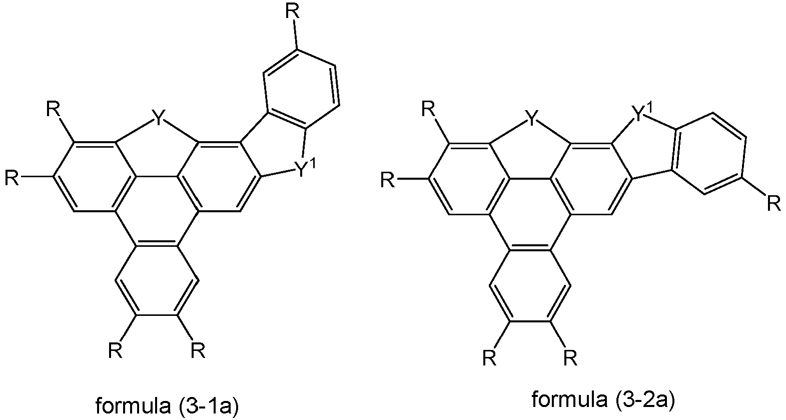 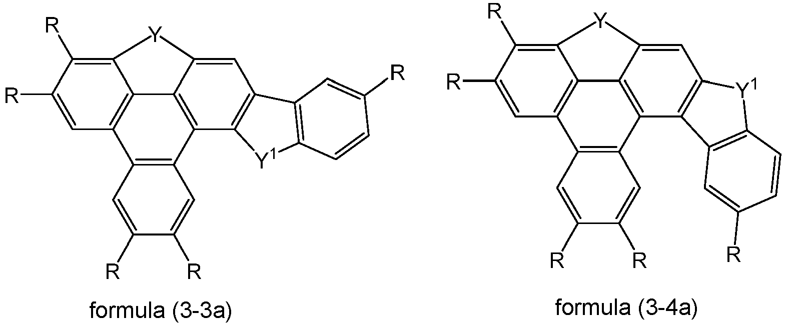 where the symbols used have the meanings given in Claim 1.

5. Compound according to one or more of Claims 1 to 4, selected from the following compounds
| | | |
|---|---|---|
| | 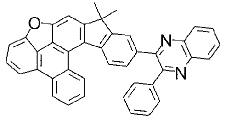 | 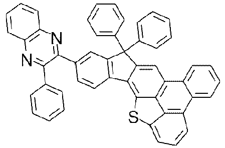 |
| 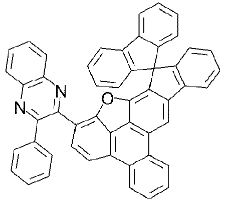 | 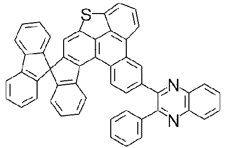 | 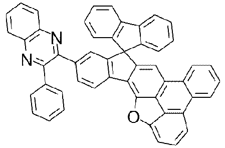 |
| | | 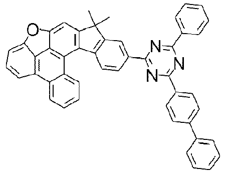 |
| 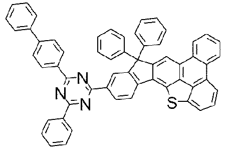 | 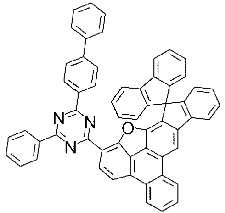 | 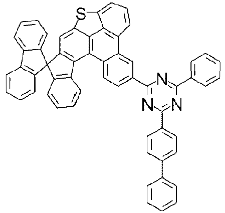 |
| 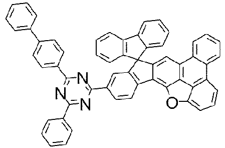 | 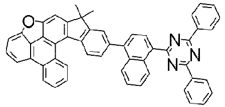 | |
| | 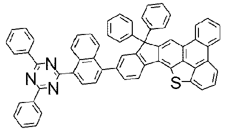 | 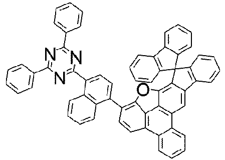 |
| 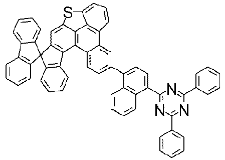 | 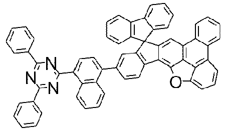 | |
| | 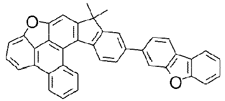 | 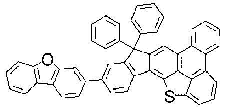 |
| 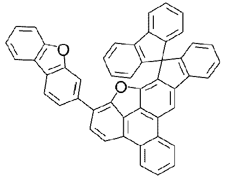 | 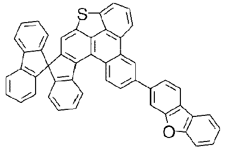 | 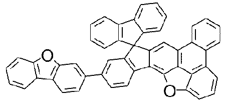 |
| | | 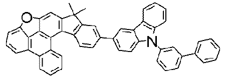 |
| 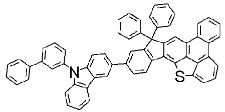 | 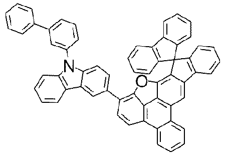 | 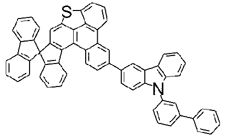 |
| 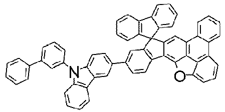 | 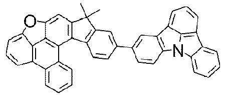 | |
| | 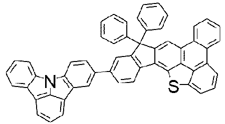 | 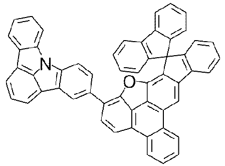 |
| 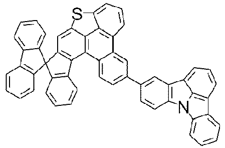 | 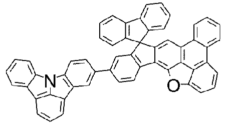 | 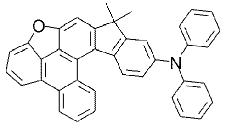 |
| | | 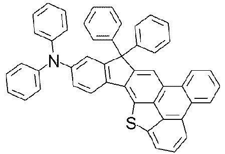 |
| 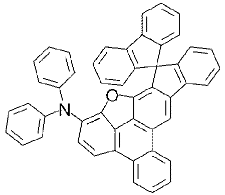 | 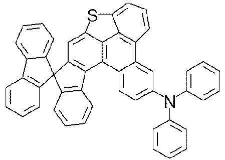 | 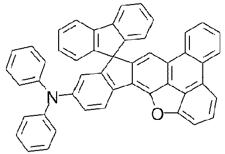 |
| | | 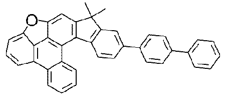 |
| 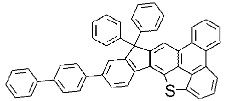 | 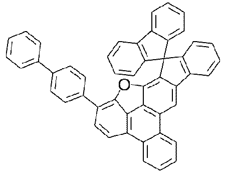 | 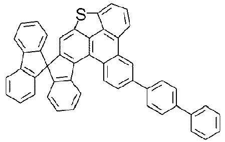 |
| 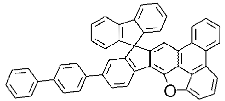 | 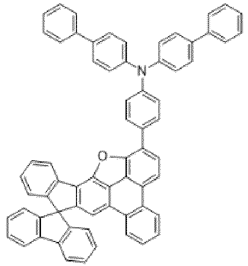 P1b | 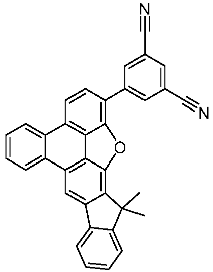 P2b |
| 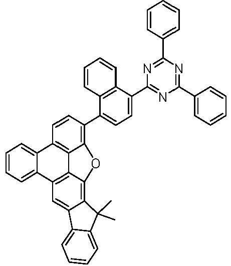 P3b | 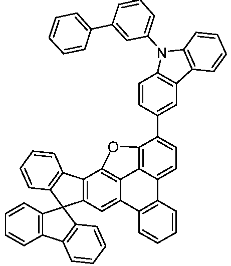 P4b | 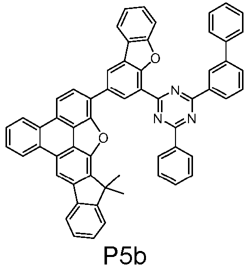 |
| 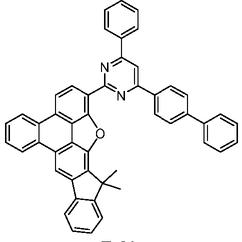 P6b | 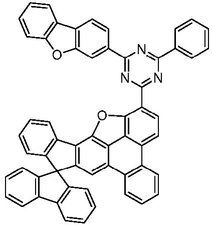 P7b. | |

6. Process for the preparation of a compound according to Claim 1, **characterised by** the following steps:
(A) synthesis of the basic structure of the formula (1);
(B) condensation-on of the aromatic or heteroaromatic ring system with formation of the bonds denoted by * in formula (1) by a coupling and ring-closure reaction.

7. Formulation comprising at least one compound according to one or more of Claims 1 to 5 and at least one further compound and/or at least one solvent.

8. Use of a compound according to one or more of Claims 1 to 5 and/or a formulation according to Claim 7 in an electronic device.

9. Electronic device containing at least one compound according to one or more of Claims 1 to 5 and/or a formulation according to Claim 7.

10. Electronic device according to Claim 9, which is an organic electroluminescent device, **characterised in that** the compound according to one or more of Claims 1 to 5 is employed in an emitting layer as matrix material for phosphorescent or fluorescent emitters or for emitters which exhibit TADF (thermally activated delayed fluorescence), and/or in an electron-transport layer and/or in a hole-blocking layer and/or in a hole-transport layer and/or in an electron-blocking layer.

11. Electronic device according to Claim 10, where the emitting layer comprises the compound according to one or more of Claims 1 to 5 in combination with one or more phosphorescent materials.

12. Electronic device according to Claim 10, where the emitting layer comprises the compound according to one or more of Claims 1 to 5 in combination with a further matrix material.

13. Electronic device according to Claim 12, where the further matrix material is selected from the group of the biscarbazoles, the bridged carbazoles, the triarylamines, the dibenzofurancarbazoles, the di-benzofuranamines and the carbazolamines.

14. Electronic device according to Claim 12, where the further matrix material is selected from the group of the triazines, pyrimidines, quinazolines or quinoxalines.

## Revendications

1. Composé de formule (1), 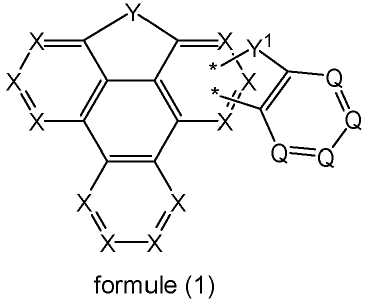 dans laquelle ce qui suit s'applique aux symboles utilisés :
X est, de manière identique ou différente à chaque occurrence, CR ou N, à condition qu'un maximum de deux groupements X par cycle représentent N et que deux groupements adjacents X qui font partie du cycle ayant trois groupements X représentent C et forment, via les liaisons désignées par *, un noyau aromatique ou hétéroaromatique qui est condensé sur le cycle ;
Y est C(R)₂, O ou S ;
Y¹ est C(R)₂ ;
Q est, de manière identique ou différente à chaque occurrence, CR ou N, à condition qu'un maximum de deux groupements Q par cycle représentent N ;
R est à chaque occurrence, de manière identique ou différente, H, D, F, Cl, Br, I, N(Ar')₂, N(R')₂, OAr', SAr', B(OR¹)₂, CHO, C(=O)R¹, CR¹=C(R¹)₂, CN, C(=O)OR¹, C(=O)NR¹, Si(R¹)₃, NO₂, P(=O)(R¹)₂, OSO₂R¹, OR¹, S(=O)R¹, S(=O)₂R¹, SR¹, un groupement alkyle à chaine linéaire ayant de 1 à 20 atomes de C ou un groupement alcényle ou alcynyle ayant de 2 à 20 atomes de C ou un groupement alkyle ramifié ou cyclique ayant de 3 à 20 atomes de C, où le groupement alkyle, alcényle ou alcynyle peut dans chaque cas être substitué par un ou plusieurs radicaux R¹, où un ou plusieurs groupements CH₂ non adjacents peuvent être remplacés par -R¹C=CR¹-, -C≡C-, Si(R¹)₂, NR¹, CONR¹, C=O, C=S, -C(=O)O-, P(=O)(R¹), -O-, -S-, SO ou SO₂, ou un noyau aromatique ou hétéroaromatique ayant de 5 à 60 atomes de cycle aromatique, préférablement ayant de 5 à 40 atomes de cycle aromatique, qui peut dans chaque cas être substitué par un ou plusieurs radicaux R¹, où deux, ou plus, radicaux R liés au même cycle peuvent former, les uns avec les autres, un noyau aliphatique, hétéroaliphatique, aromatique ou hétéroaromatique, qui peut être substitué par un ou plusieurs radicaux R¹, et où deux radicaux R liés au même atome de carbone, silicium, germanium ou étain peuvent former, les uns avec les autres, un noyau aliphatique, aromatique ou hétéroaromatique, monocyclique ou polycyclique, qui peut être substitué par un ou plusieurs radicaux R¹ ;
Ar' est à chaque occurrence, de manière identique ou différente, un noyau aromatique ou hétéroaromatique ayant de 5 à 40 atomes de cycle aromatique, qui peut être substitué par un ou plusieurs radicaux R¹ ;
R¹ est à chaque occurrence, de manière identique ou différente, H, D, F, I, B(OR²)₂, N(R²)₂, CHO, C(=O)R², CR²=C(R²)₂, CN, C(=O)OR², Si(R²)₃, NO₂, P(=O)(R²)₂, OSO₂R², SR², OR², S(=O)R², S(=O)₂R², un groupement alkyle à chaine linéaire ayant de 1 à 20 atomes de C ou un groupement alcényle ou alcynyle ayant de 2 à 20 atomes de C ou un groupement alkyle ramifié ou cyclique ayant de 3 à 20 atomes de C, où le groupement alkyle, alcényle ou alcynyle peut dans chaque cas être substitué par un ou plusieurs radicaux R² et où un ou plusieurs groupements CH₂ dans les groupements susmentionnés peuvent être remplacés par -R²C=CR²-, -C≡C-, Si(R²)₂, C=O, C=S, -C(=O)O-, NR², CONR², P(=O)(R²), -O-, -S-, SO ou SO₂ et où un ou plusieurs atomes de H dans les groupements susmentionnés peuvent être remplacés par D, F, Cl, Br, I, CN ou NO₂, ou un noyau aromatique ou hétéroaromatique ayant de 5 à 30 atomes de cycle aromatique, qui peut dans chaque cas être substitué par un ou plusieurs radicaux R², où deux, ou plus, radicaux R¹ peuvent former un noyau aliphatique, hétéroaliphatique, aromatique ou hétéroaromatique les uns avec les autres ;
R² est à chaque occurrence, de manière identique ou différente, H, D, F, CN ou un radical organique aliphatique, aromatique ou hétéroaromatique ayant de 1 à 20 atomes de C, où, de plus, un ou plusieurs atomes de H peuvent être remplacés par D ou F ; deux, ou plus, substituants R² peuvent être liés les uns aux autres et former un cycle.

2. Composé selon la revendication 1, choisi parmi les composés de formules (2-1) à (2-4), 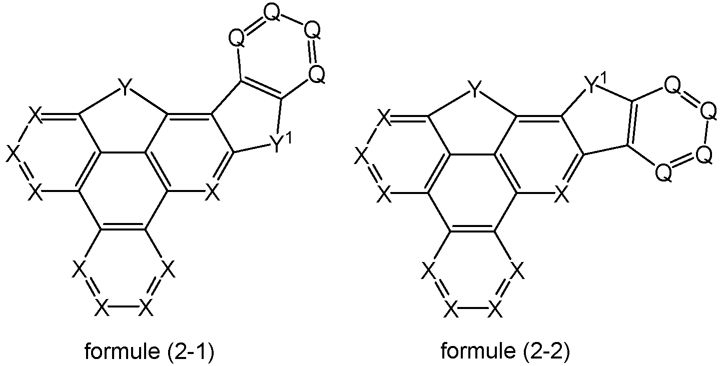 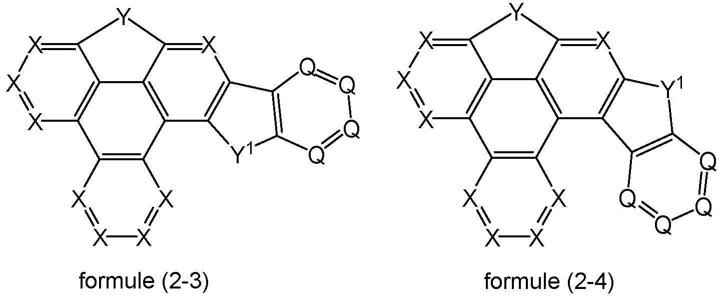 dans lesquelles les symboles utilisés revêtent les significations données selon la revendication 1.

3. Composé selon la revendication 1 ou 2, choisi parmi les composés de formules (3-1) à (3-4), 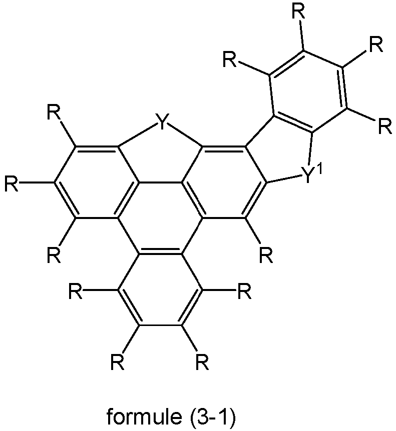 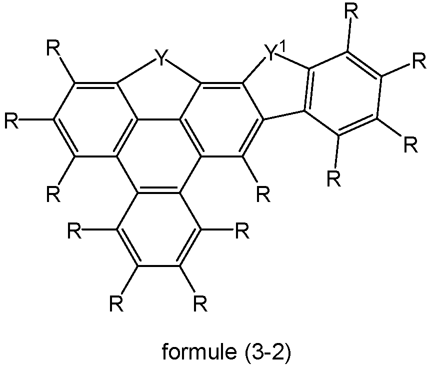 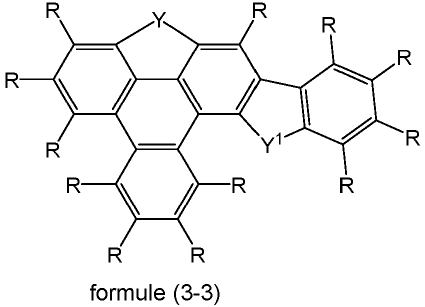 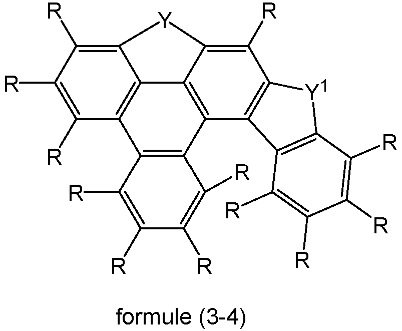 dans lesquelles les symboles utilisés revêtent les significations données selon la revendication 1.

4. Composé selon l'une ou plusieurs parmi les revendications 1 à 3, choisi parmi les composés de formules (3-1a) à (3-4a), 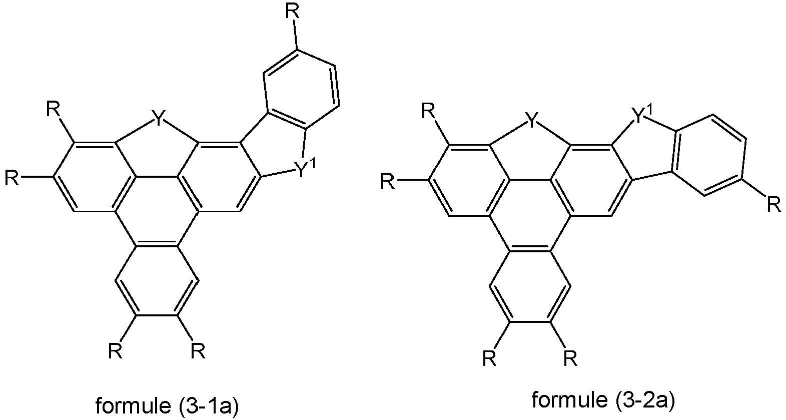 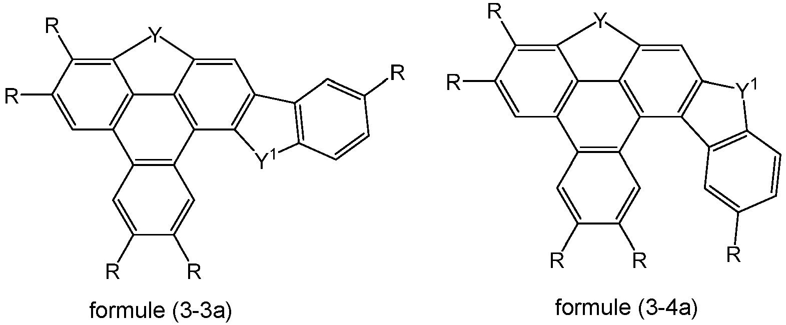 dans lesquelles les symboles utilisés revêtent les significations données selon la revendication 1.

5. Composé selon l'une ou plusieurs parmi les revendications 1 à 4, choisi parmi les composés suivants
| | | |
|---|---|---|
| | 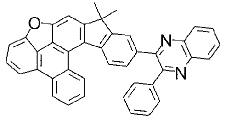 | 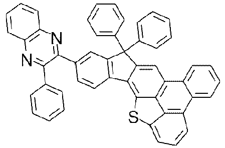 |
| 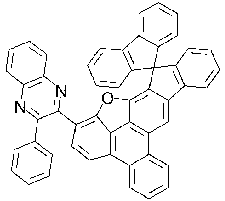 | 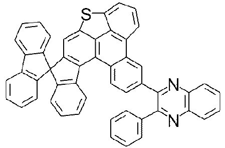 | 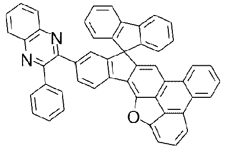 |
| | | 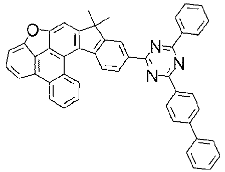 |
| 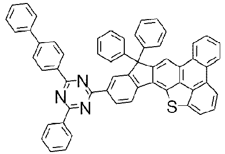 | 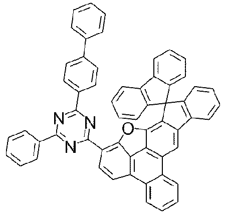 | 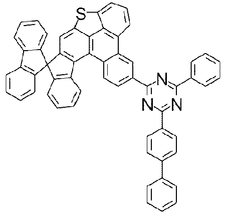 |
| 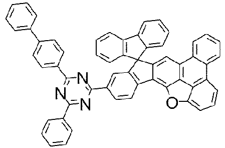 | 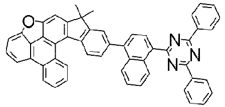 | |
| | 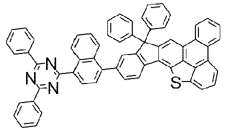 | 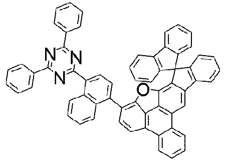 |
| 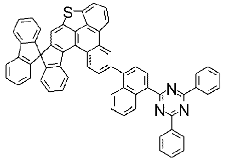 | 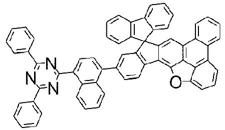 | |
| | 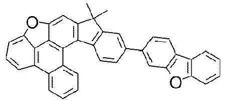 | 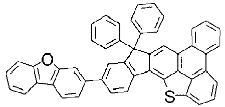 |
| 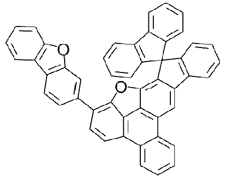 | 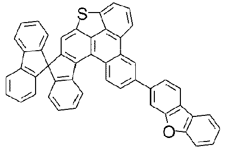 | 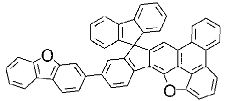 |
| | | 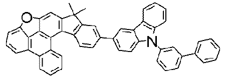 |
| 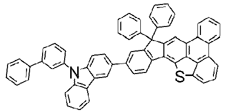 | 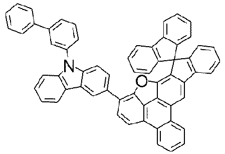 | 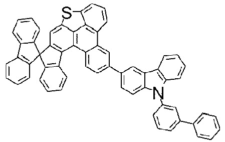 |
| 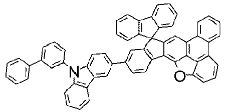 | 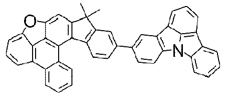 | |
| | 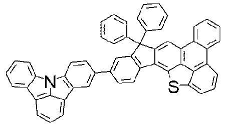 | 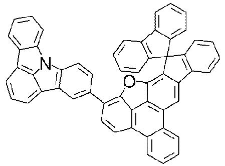 |
| 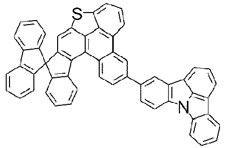 | 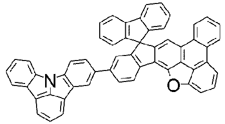 | 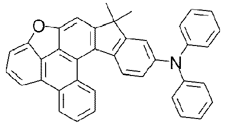 |
| | | 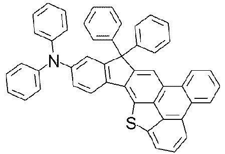 |
| 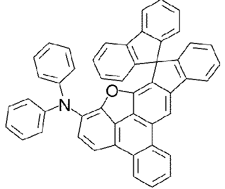 | 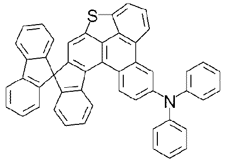 | 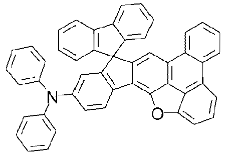 |
| | | 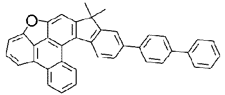 |
| 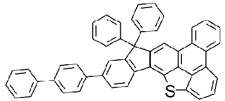 | 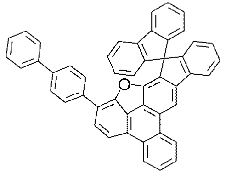 | 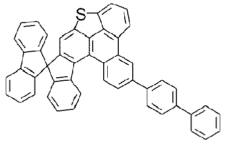 |
| 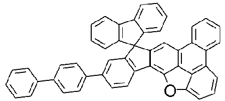 | 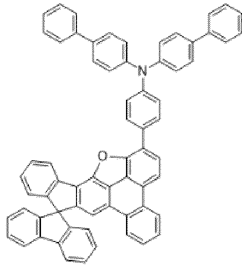 P1b | 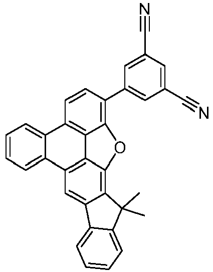 P2b |
| 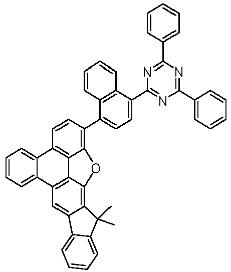 P3b | 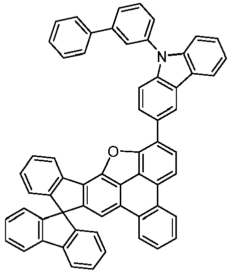 P4b | 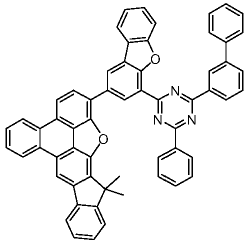 P5b |
| 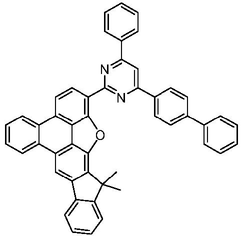 P6b | 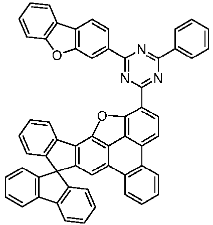 P7b. | |

6. Procédé de préparation d'un composé selon la revendication 1, **caractérisé par** les étapes suivantes :
(A) synthèse de la structure basique de formule (1) ;
(B) condensation du noyau aromatique ou hétéroaromatique avec formation des liaisons désignées par * dans la formule (1) par une réaction de couplage et de fermeture de cycle.

7. Formulation comprenant au moins un composé selon l'une ou plusieurs parmi les revendications 1 à 5 et au moins un autre composé et/ou au moins un solvant.

8. Utilisation d'un composé selon l'une ou plusieurs parmi les revendications 1 à 5 et/ou d'une formulation selon la revendication 7 dans un dispositif électronique.

9. Dispositif électronique contenant au moins un composé selon l'une ou plusieurs parmi les revendications 1 à 5 et/ou une formulation selon la revendication 7.

10. Dispositif électronique selon la revendication 9, qui est un dispositif électroluminescent organique, **caractérisé en ce que** le composé selon l'une ou plusieurs parmi les revendications 1 à 5 est employé dans une couche émettrice comme matériau de matrice pour les émetteurs phosphorescents ou fluorescents ou pour les émetteurs présentant une TADF (fluorescence retardée activée thermiquement), et/ou dans une couche de transport d'électrons et/ou dans une couche de blocage de trous et/ou dans une couche de transport de trous et/ou dans une couche de blocage d'électrons.

11. Dispositif électronique selon la revendication 10, dans lequel la couche émettrice comprend le composé selon l'une ou plusieurs parmi les revendications 1 à 5 en combinaison avec un ou plusieurs matériaux phosphorescents.

12. Dispositif électronique selon la revendication 10, dans lequel la couche émettrice comprend le composé selon l'une ou plusieurs parmi les revendications 1 à 5 en combinaison avec un matériau de matrice supplémentaire.

13. Dispositif électronique selon la revendication 12, dans lequel le matériau de matrice supplémentaire est choisi dans le groupe constitué par les biscarbazoles, les carbazoles pontés, les triarylamines, les di-benzofurane carbazoles, les dibenzofuranamines et les carbazolamines.

14. Dispositif électronique selon la revendication 12, dans lequel le matériau de matrice supplémentaire est choisi dans le groupe constitué par les triazines, les pyrimidines, les quinazolines ou les quinoxalines.
